# EUROPEAN PATENT APPLICATION

(11) **EP 4 494 659 A1**
(43) Date of publication of application: **22.01.2025**
(21) Application number: 23769903.8
(22) Date of filing: 17.03.2023
(51) Int. Cl.: A61K 47/68, A61K 39/395, A61K 31/4745, C07K 16/30, A61P 35/00

(54) **ANTI-GPC3 ANTIBODY DRUG CONJUGATE AND USE THEREOF**

(30) Priority: 18.03.2022 CN 202210273494; 13.03.2023 CN 202310238636
(71) Applicant: Duality Biologics (Suzhou) Co., Ltd., Suzhou, Jiangsu 215000 (CN)
(72) Inventor: LIN, Shengchao, Shanghai 201204 (CN); ZHANG, Yu, Shanghai 201204 (CN); HUA, Haiqing, Shanghai 201204 (CN); ZHU, Zhongyuan, Shanghai 201204 (CN)
(74) Representative: Greaves Brewster LLP
(86) International application number: PCT/CN2023/082141
(87) International publication number: WO 2023/174401

(57) **Abstract**

An anti-GPC3 antibody drug conjugate that specifically binds to GPC3 and a use thereof. The anti-GPC3 antibody drug conjugate has a structure as shown in formula (1-1). The anti-GPC3 antibody drug conjugate has better inhibitory activity on in-vitro proliferation of tumor cells and a better in-vivo tumor suppression effect.

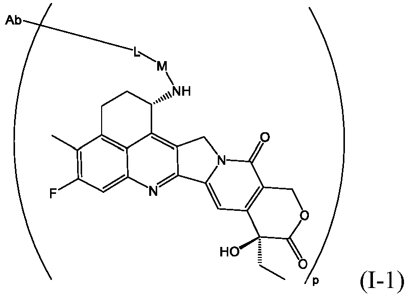

## Description

The present application claims the right of the priorities of Chinese patent application 2022102734945 filed on March 18, 2022 and Chinese patent application 2023102386369 filed on March 13, 2023. The contents of the Chinese patent applications are incorporated herein by reference in their entireties.

### TECHNICAL FIELD

The present disclosure provides an antibody-drug conjugate that specifically binds to GPC3, and a composition containing the same. The present disclosure further provides a method for the use of the antibody-drug conjugate, and a use of the antibody-drug conjugate.

### BACKGROUND

Glypican-3 (GPC3) is a 70 kDa membrane protein composed of 580 amino acids, anchored to the cell surface through glycosylphosphatidylinositol (GPI). GPC3 plays a significant role in cell growth, differentiation, and migration. GPC3 protein is expressed in embryonic tissues (such as liver, kidney, and placenta), but it is almost absent in adult tissues due to epigenetic silencing caused by DNA methylation. However, immunohistochemical studies have found that GPC3 is specifically and highly expressed in more than 70% of liver cancer tissues, and clinical studies have also shown that GPC3 expression is associated with poor clinical prognosis in hepatocellular carcinoma (HCC). Therefore, GPC3 has the potential to be a therapeutic target for liver cancer.

According to the latest statistics from the National Cancer Center, liver cancer ranks fifth in incidence and second in mortality among all malignant tumors in China. The number of new liver cancer cases in China accounts for about half of the global total, with approximately 460,000 new cases annually, but the five-year survival rate is only 10%, making it one of the malignancies with the poorest prognosis. Previously, first-line treatment drugs/schemes for advanced liver cancer patients mainly included sorafenib and systemic chemotherapy, which had low objective response rates, short progression-free survival (PFS), and significant adverse effects. In recent years, the treatment schemes of lenvatinib, atezolizumab combined with bevacizumab have extended PFS by nearly three months, but treatment options for liver cancer remain limited after recurrence or progression. There are various types of clinical drugs under development targeting GPC3, including monoclonal antibodies, bispecific antibodies, antibody-drug conjugates (ADCs), and CAR-T cell therapies, etc.

Anti-GPC3 monoclonal antibody Codrituzumab (development code GC33) developed by Roche is currently the fastest progressing GPC3-targeted molecule in clinical development. In a Phase Ib clinical study (NCT01507168) for the treatment of advanced liver cancer, Codrituzumab showed a trend of delayed disease progression in patients with high GPC3 expression. However, in a Phase II study (NCT01507168), Codrituzumab did not demonstrate a statistically significant improvement in survival, leading Roche to suspend the development of Codrituzumab. At the ASCO meeting in 2021, CARsgen Therapeutics reported on their GPC3-CAR-T therapy in six patients with advanced liver cancer. Among them, one patient achieved partial remission, three had stable disease, resulting in a disease control rate of 50%, and a median progression-free survival of 4.2 months. Given the relatively weak antitumor efficacy of monoclonal antibody drugs and the inconvenience of CAR-T cell therapy, GPC3-targeted ADC drugs have unique developmental value and clinical demand.

### CONTENT OF THE PRESENT INVENTION

The technical problem to be solved by the present disclosure is to overcome the deficiency of the limited number of anti-GPC3 antibody-drug conjugates in the prior art, and to provide an anti-GPC3 antibody-drug conjugate, a preparation method therefor, and a use thereof. Compared with the prior art, the anti-GPC3 antibody-drug conjugate of the present disclosure has one or more of the following advantages: (1) better inhibition activity of tumor cell proliferation *in vitro;* (2) better targeting inhibition; (3) better plasma stability; (4) better *in vivo* tumor suppression effect; (5) better bystander killing effect (bystander effect); (6) better anti-transporter transport capacity; (7) better tumor targeting ability *in vivo;* and (8) better *in vivo* safety.

The present disclosure primarily addresses the above technical problem through the following technical means.

In one aspect, the present disclosure provides an anti-GPC3 antibody-drug conjugate, an isomer thereof, a pharmaceutically acceptable salt thereof, or a mixture thereof; the anti-GPC3 antibody-drug conjugate has a structure as shown in formula (I):

Ab-(L-M-D)ₚ (I)

wherein
L and M are linker units;
D is a cytotoxic drug;
p represents the average number of connections, and p is an integer or a decimal from 1 to 10, preferably an integer or a decimal from 3 to 8;
Ab is an anti-GPC3 antibody or an antigen-binding fragment thereof, which comprises a heavy chain variable region and a light chain variable region; the heavy chain variable region comprises the amino acid sequences of HCDR1, HCDR2, and HCDR3 as shown in SEQ ID NO: 1, SEQ ID NO: 2, and SEQ ID NO: 3, respectively; the light chain variable region comprises the amino acid sequences of LCDR1, LCDR2, and LCDR3 as shown in SEQ ID NO: 4, SEQ ID NO: 5, and SEQ ID NO: 6, respectively.

In one aspect, the present disclosure provides an anti-GPC3 antibody-drug conjugate, an isomer thereof, a pharmaceutically acceptable salt thereof, or a mixture thereof; the anti-GPC3 antibody-drug conjugate has a structure as shown in formula (I):

Ab-(L-M-D)ₚ (I)

wherein
L is a linker unit;
-M-D is a cytotoxic drug;
p represents the average number of connections, and p is an integer or a decimal from 1 to 10, preferably an integer or a decimal from 3 to 8;
Ab is an anti-GPC3 antibody or an antigen-binding fragment thereof, which comprises a heavy chain variable region and a light chain variable region; the heavy chain variable region comprises the amino acid sequences of HCDR1, HCDR2, and HCDR3 as shown in SEQ ID NO: 1, SEQ ID NO: 2, and SEQ ID NO: 3, respectively; the light chain variable region comprises the amino acid sequences of LCDR1, LCDR2, and LCDR3 as shown in SEQ ID NO: 4, SEQ ID NO: 5, and SEQ ID NO: 6, respectively.

In one aspect, the present disclosure provides an anti-GPC3 antibody-drug conjugate, an isomer thereof, a pharmaceutically acceptable salt thereof, or a mixture thereof, wherein the anti-GPC3 antibody-drug conjugate has a structure as shown in formula (I-1): 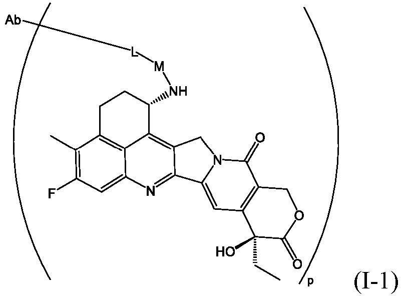 wherein
M is -L²-L³-X-L¹-;
L² is -O- or -S-;
L³ is -(C(R^{1a})(R^{1b}))ₘ- and m is 0, 1, 2, or 3, wherein when L³ comprises a methylene unit, 0 or 1 methylene unit of the L³ is replaced by -C(O)- or -C(S)-;
L¹ is -(C(R^{2a})(R^{2b}))ₙ- and n is selected from 1, 2, or 3, wherein when L¹ may comprise a methylene unit, 0 or 1 methylene unit of the L¹ is replaced by -C(O)- or -C(S)-;
X is 3- to 6-membered saturated carbocyclyl, 3- to 6-membered saturated heterocyclyl, or a single bond, and the 3- to 6-membered saturated carbocyclyl and the 3- to 6-membered saturated heterocyclyl are optionally substituted by one or more R^{3a};
wherein R^{1a}, R^{1b}, R^{2a}, R^{2b}, and R^{3a} may each independently be hydrogen, halogen, or a C₁₋₆ aliphatic group optionally substituted by one or more R;
wherein each R may independently be hydrogen or halogen;
L is a linker unit;
p represents the average number of connections, and p is an integer or a decimal from 1 to 10;
Ab is an anti-GPC3 antibody or an antigen-binding fragment thereof.

In some embodiments, the heteroatoms in the 3- to 6-membered saturated heterocyclyl as described in the present disclosure are selected from N, O, and S, with the number of the heteroatoms being 1 to 3.

In some embodiments, the anti-GPC3 antibody or the antigen-binding fragment thereof as described in the present disclosure comprises a heavy chain variable region and a light chain variable region; the heavy chain variable region comprises the amino acid sequences of HCDR1, HCDR2, and HCDR3 as shown in SEQ ID NO: 1, SEQ ID NO: 2, and SEQ ID NO: 3, respectively; the light chain variable region comprises the amino acid sequences of LCDR1, LCDR2, and LCDR3 as shown in SEQ ID NO: 4, SEQ ID NO: 5, and SEQ ID NO: 6, respectively.

In some embodiments, the anti-GPC3 antibody or the antigen-binding fragment thereof as described in the present disclosure comprises a heavy chain variable region having an amino acid sequence as shown in SEQ ID NO: 7 or a heavy chain variable region having at least 95%, 96%, 97%, 98%, or 99% identity thereto, and a light chain variable region having an amino acid sequence as shown in SEQ ID NO: 8 or a light chain variable region having at least 95%, 96%, 97%, 98%, or 99% identity thereto.

In some embodiments, the anti-GPC3 antibody or the antigen-binding fragment thereof as described in the present disclosure comprises a heavy chain variable region having an amino acid sequence as shown in SEQ ID NO: 7 and a light chain variable region having an amino acid sequence as shown in SEQ ID NO: 8.

In some embodiments, the anti-GPC3 antibody or the antigen-binding fragment thereof as described in the present disclosure is a murine antibody or a fragment thereof, a chimeric antibody or an antigen-binding fragment thereof, a humanized antibody or an antigen-binding fragment thereof, or a fully human antibody or an antigen-binding fragment thereof.

In some embodiments, the anti-GPC3 antibody or the antigen-binding fragment thereof as described in the present disclosure is a humanized antibody or a fragment thereof.

In some embodiments, the anti-GPC3 antibody or the antigen-binding fragment thereof as described in the present disclosure is Fab, Fab', Fab'-SH, Fv, scFv, F(ab')₂, sdAb, a bispecific antibody, or a linear antibody.

In some embodiments, the anti-GPC3 antibody as described in the present disclosure is a monoclonal antibody.

In some embodiments, the antibody as described in the present disclosure is in the form of an IgG1 antibody, IgG2 antibody, IgG3 antibody, or IgG4 antibody.

In some embodiments, the antibody as described in the present disclosure is in the form of an IgG1 antibody.

In some embodiments, the anti-GPC3 antibody or the antigen-binding fragment thereof as described in the present disclosure comprises a heavy chain having an amino acid sequence as shown in SEQ ID NO: 9 or a heavy chain having at least 95%, 96%, 97%, 98%, or 99% identity thereto, and a light chain having an amino acid sequence as shown in SEQ ID NO: 10 or a light chain having at least 95%, 96%, 97%, 98%, or 99% identity thereto.

In some embodiments, the anti-GPC3 antibody or the antigen-binding fragment thereof as described in the present disclosure comprises a heavy chain having an amino acid sequence as shown in SEQ ID NO: 9 and a light chain having an amino acid sequence as shown in SEQ ID NO: 10.

In some embodiments, the present disclosure describes the anti-GPC3 antibody-drug conjugate, the isomer thereof, the pharmaceutically acceptable salt thereof, or the mixture thereof, wherein the L² terminal of M is connected to the linker unit L, and the L¹ terminal is connected to D.

In some embodiments, the L² of the present disclosure is -O-.

In some embodiments, the L² of the present disclosure is -S-.

In some embodiments, the present disclosure describes the anti-GPC3 antibody-drug conjugate, the isomer thereof, the pharmaceutically acceptable salt thereof, or the mixture thereof, wherein L³ is -(C(R^{1a})(R^{1b}))ₘ-, wherein m is 0, 1, 2, or 3; wherein R^{1a} and R^{1b} may each independently be hydrogen, halogen, or a C₁₋₆ aliphatic group optionally substituted by 1, 2, or 3 R; wherein each R may independently be hydrogen or halogen.

In some embodiments, the present disclosure describes the anti-GPC3 antibody-drug conjugate, the isomer thereof, the pharmaceutically acceptable salt thereof, or the mixture thereof, wherein L³ is -(C(R^{1a})(R^{1b}))ₘ-, wherein m is 0, 1, 2, or 3; wherein R^{1a} and R^{1b} are each independently hydrogen, methyl, ethyl, or propyl.

In some embodiments, the present disclosure describes the anti-GPC3 antibody-drug conjugate, the isomer thereof, the pharmaceutically acceptable salt thereof, or the mixture thereof, wherein L³ is -(C(R^{1a})(R^{1b}))ₘ-, wherein m is 0, 1, 2, or 3; wherein R^{1a} and R^{1b} may each independently be hydrogen, halogen, CH₃, or CH₂CH₃.

In some embodiments, the present disclosure describes the anti-GPC3 antibody-drug conjugate, the isomer thereof, the pharmaceutically acceptable salt thereof, or the mixture thereof, wherein L³ is a single bond, -CH₂-, -CH(CH₃)-, -C(CH₃)₂-, -CH₂CH₂-, -CH(CH₃)CH₂-, or -C(CH₃)₂CH₂-. Herein, the left side of the above-mentioned group is preferably connected to L².

In some embodiments, the present disclosure describes the anti-GPC3 antibody-drug conjugate, the isomer thereof, the pharmaceutically acceptable salt thereof, or the mixture thereof, wherein L³ is 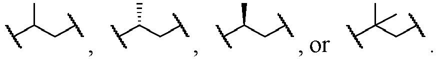 Herein, the left side of the structural moiety is preferably connected to L².

In some embodiments, the present disclosure describes the anti-GPC3 antibody-drug conjugate, the isomer thereof, the pharmaceutically acceptable salt thereof, or the mixture thereof, wherein L³ is a single bond.

In some embodiments, the present disclosure describes the anti-GPC3 antibody-drug conjugate, the isomer thereof, the pharmaceutically acceptable salt thereof, or the mixture thereof, wherein X is 3- to 6-membered saturated carbocyclyl optionally substituted by 1, 2, or 3 R^{3a} or a single bond; wherein each R^{3a} may independently be hydrogen, halogen, or a C₁₋₆ aliphatic group optionally substituted by 1, 2, or 3 R; wherein each R may independently be hydrogen or halogen.

In some embodiments, the present disclosure describes the anti-GPC3 antibody-drug conjugate, the isomer thereof, the pharmaceutically acceptable salt thereof, or the mixture thereof, wherein X is 3- to 6-membered saturated carbocyclyl optionally substituted by 1, 2, or 3 R^{3a} or a single bond; wherein each R^{3a} may independently be hydrogen, halogen, CH₃, or CH₂CH₃.

In some embodiments, the present disclosure describes the anti-GPC3 antibody-drug conjugate, the isomer thereof, the pharmaceutically acceptable salt thereof, or the mixture thereof, wherein X is cyclopropyl, cyclobutyl, cyclopentyl, cyclohexyl, or a single bond.

In some embodiments, the present disclosure describes the anti-GPC3 antibody-drug conjugate, the isomer thereof, the pharmaceutically acceptable salt thereof, or the mixture thereof, wherein X is 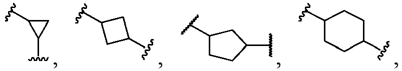 or a single bond. Herein, the right side of the above-mentioned group is preferably connected to L¹.

In some embodiments, the present disclosure describes the anti-GPC3 antibody-drug conjugate, the isomer thereof, the pharmaceutically acceptable salt thereof, or the mixture thereof, wherein X is a single bond.

In some embodiments, the present disclosure describes the anti-GPC3 antibody-drug conjugate, the isomer thereof, the pharmaceutically acceptable salt thereof, or the mixture thereof, wherein X is 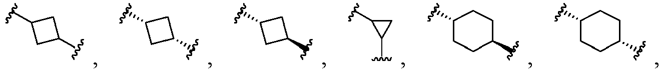 or 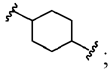 preferably, X is 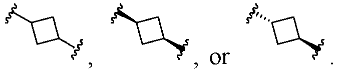 Herein, the right side of the above-mentioned group is preferably connected to L¹.

In some embodiments, the present disclosure describes the anti-GPC3 antibody-drug conjugate, the isomer thereof, the pharmaceutically acceptable salt thereof, or the mixture thereof, wherein the -L³-X- is -(C(R^{1a})(R^{1b}))ₘ-, 3- to 6-membered saturated carbocyclyl, or 3-to 6-membered saturated heterocyclyl; wherein m is 1, 2, or 3, and the 3- to 6-membered saturated carbocyclyl and 3- to 6-membered saturated heterocyclyl are optionally substituted by 1, 2, or 3 R^{3a}; wherein R^{1a}, R^{1b}, and R^{3a} may each independently be hydrogen, halogen, or a C₁₋₆ aliphatic group optionally substituted by R; wherein each R may independently be hydrogen or halogen.

In some embodiments, the present disclosure describes the anti-GPC3 antibody-drug conjugate, the isomer thereof, the pharmaceutically acceptable salt thereof, or the mixture thereof, wherein
the -L³-X- is -(C(R^{1a})(R^{1b}))ₘ- or 3- to 6-membered saturated carbocyclyl optionally substituted by 1, 2, or 3 R^{3a}; for example, the -L³-X- is -(C(R^{1a})(R^{1b}))ₘ-, cyclopropyl, cyclobutyl, cyclopentyl, or cyclohexyl, and the cyclopropyl, cyclobutyl, cyclopentyl, and cyclohexyl are optionally substituted by 1, 2, or 3 R^{3a}; preferably, the -L³-X- is -(C(R^{1a})(R^{1b}))ₘ-, 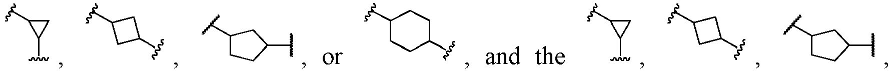 and 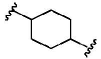 are optionally substituted by 1, 2, or 3 R^{3a};
the m is 1, 2, or 3;
each R^{1a} is independently halogen or a C₁₋₆ aliphatic group optionally substituted by 1, 2, or 3 R, such as methyl, ethyl, or propyl; R^{1b} and R^{3a} are each independently hydrogen, halogen, or a C₁₋₆ aliphatic group optionally substituted by 1, 2, or 3 R, such as hydrogen, methyl, ethyl, or propyl; each R is independently hydrogen or halogen.

In some embodiments, the present disclosure describes the anti-GPC3 antibody-drug conjugate, the isomer thereof, the pharmaceutically acceptable salt thereof, or the mixture thereof, wherein
the -L³-X- is selected from -(C(R^{1a})(R^{1b}))ₘ- or 3- to 6-membered saturated carbocyclyl optionally substituted by 1, 2, or 3 R^{3a}; preferably, the -L³-X- is -(C(R^{1a})(R^{1b}))ₘ-, 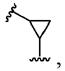 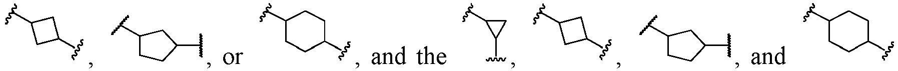 are optionally substituted by 1, 2, or 3 R^{3a}; wherein m is 1, 2, or 3; each R^{1a} is independently halogen or a C₁₋₆ aliphatic group optionally substituted by 1, 2, or 3 R; R^{1b} and R^{3a} are each independently hydrogen, halogen, or a C₁₋₆ aliphatic group optionally substituted by 1, 2, or 3 R; wherein each R may independently be hydrogen or halogen.

In some embodiments, the present disclosure describes the anti-GPC3 antibody-drug conjugate, the isomer thereof, the pharmaceutically acceptable salt thereof, or the mixture thereof, wherein the -L³-X- is

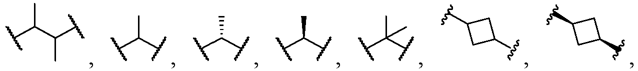

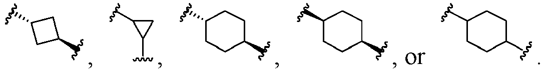

In some embodiments, the present disclosure describes the anti-GPC3 antibody-drug conjugate, the isomer thereof, the pharmaceutically acceptable salt thereof, or the mixture thereof, wherein the -L³-X- is

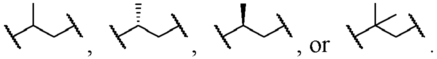

In some embodiments, the present disclosure describes the anti-GPC3 antibody-drug conjugate, the isomer thereof, the pharmaceutically acceptable salt thereof, or the mixture thereof, wherein L¹ is -C(R^{2a})(R^{2b})-, -C(R^{2a})(R^{2b})C(O)-, or -C(O)-; wherein R^{2a} and R^{2b} may each independently be hydrogen, halogen, or a C₁₋₆ aliphatic group optionally substituted by 1, 2, or 3 R; wherein each R may independently be hydrogen or halogen.

**In** some embodiments, the present disclosure describes the anti-GPC3 antibody-drug conjugate, the isomer thereof, the pharmaceutically acceptable salt thereof, or the mixture thereof, wherein L¹ is -C(R^{2a})(R^{2b})-, -C(R^{2a})(R^{2b})C(O)-, or -C(O)-; wherein R^{2a} and R^{2b} may each independently be hydrogen, methyl, ethyl, or propyl.

In some embodiments, the present disclosure describes the anti-GPC3 antibody-drug conjugate, the isomer thereof, the pharmaceutically acceptable salt thereof, or the mixture thereof, wherein L¹ is -C(R^{2a})(R^{2b})-, -C(R^{2a})(R^{2b})C(O)-, or -C(O)-; wherein R^{2a} and R^{2b} may each independently be hydrogen, halogen, CH₃, or CH₂CH₃.

In some embodiments, the present disclosure describes the anti-GPC3 antibody-drug conjugate, the isomer thereof, the pharmaceutically acceptable salt thereof, or the mixture thereof, wherein L¹ is -CH₂C(O)-, -CH(CH₃)C(O)-, or -C(O)-.

In some embodiments, the present disclosure describes the anti-GPC3 antibody-drug conjugate, the isomer thereof, the pharmaceutically acceptable salt thereof, or the mixture thereof, wherein L¹ is -C(O)-.

In some embodiments, the present disclosure describes the anti-GPC3 antibody-drug conjugate, the isomer thereof, the pharmaceutically acceptable salt thereof, or the mixture thereof, wherein L¹ is -CH₂C(O)-.

In some embodiments, the present disclosure describes the anti-GPC3 antibody-drug conjugate, the isomer thereof, the pharmaceutically acceptable salt thereof, or the mixture thereof, wherein
the M is -L²-L³-X-L¹-;
L² is -O-;
-L³-X- is 3- to 6-membered saturated carbocyclyl optionally substituted by 1, 2, or 3 R^{3a};
L¹ is -C(O)-;
wherein each R^{3a} may independently be hydrogen, halogen, or a C₁₋₆ aliphatic group optionally substituted by 1, 2, or 3 R;
wherein each R may independently be hydrogen or halogen.

In some embodiments, the present disclosure describes the anti-GPC3 antibody-drug conjugate, the isomer thereof, the pharmaceutically acceptable salt thereof, or the mixture thereof, wherein
the M is -L²-L³-X-L¹-;
L² is -O-;
-L³-X- is cyclopropyl, cyclobutyl, cyclopentyl, or cyclohexyl, and the cyclopropyl, cyclobutyl, cyclopentyl, and cyclohexyl are optionally substituted by 1, 2, or 3 R^{3a}; preferably, the -L³-X- is 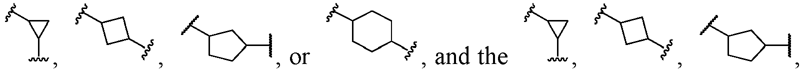 and 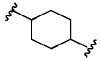 are optionally substituted by 1, 2, or 3 R^{3a};
L¹ is -C(O)-;
wherein each R^{3a} is independently hydrogen, halogen, or a C₁₋₆ aliphatic group optionally substituted by 1, 2, or 3 R;
wherein each R is independently hydrogen or halogen.

In some embodiments, the present disclosure describes the anti-GPC3 antibody-drug conjugate, the isomer thereof, the pharmaceutically acceptable salt thereof, or the mixture thereof, wherein
the M is -L²-L³-X-L¹-;
L² is -O-;
-L³-X- is 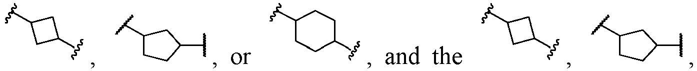 and 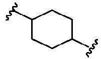 are optionally substituted by 1, 2, or 3 R^{3a}; each R^{3a} is independently hydrogen, halogen, or a C₁₋₆ aliphatic group;
L¹ is -C(O)-.

In some embodiments, the present disclosure describes the anti-GPC3 antibody-drug conjugate, the isomer thereof, the pharmaceutically acceptable salt thereof, or the mixture thereof, wherein
the M is -L²-L³-X-L¹-;
L² is -O- or -S-;
-L³-X- is -(C(R^{1a})(R^{1b}))ₘ-, wherein m is 1, 2, or 3;
L¹ is -C(O)-;
R^{1a}, R^{1b}, and R^{3a} are each independently hydrogen, halogen, or a C₁₋₆ aliphatic group optionally substituted by 1, 2, or 3 R;
each R may independently be hydrogen or halogen.

In some embodiments, the present disclosure describes the anti-GPC3 antibody-drug conjugate, the isomer thereof, the pharmaceutically acceptable salt thereof, or the mixture thereof, wherein
the M is -L²-L³-X-L¹-;
L² is -O- or -S-;
-L³-X- is -(C(R^{1a})(R^{1b}))ₘ-, wherein m is 1, 2, or 3;
L¹ is -CH₂C(O)-;
each R^{1a} is independently halogen, or a C₁₋₆ aliphatic group optionally substituted by 1, 2, or 3 R;
R^{1b} and R^{3a} are each independently hydrogen, halogen, or a C₁₋₆ aliphatic group optionally substituted by 1, 2, or 3 R;
each R may independently be hydrogen or halogen.

In some embodiments, the present disclosure describes the anti-GPC3 antibody-drug conjugate, the isomer thereof, the pharmaceutically acceptable salt thereof, or the mixture thereof, wherein
the M is -L²-L³-X-L¹-;
L² is -O- or -S-;
-L³-X- is -CH(CH₃)-, -C(CH₃)₂-, or -C(CH₃)CH(CH₃)-;
L¹ is -CH₂C(O)-.

In some embodiments, the present disclosure describes the anti-GPC3 antibody-drug conjugate, the isomer thereof, the pharmaceutically acceptable salt thereof, or the mixture thereof, wherein the L² terminal of M is connected to the linker unit L.

In some embodiments, the present disclosure describes the anti-GPC3 antibody-drug conjugate, the isomer thereof, the pharmaceutically acceptable salt thereof, or the mixture thereof, wherein the -M- is:

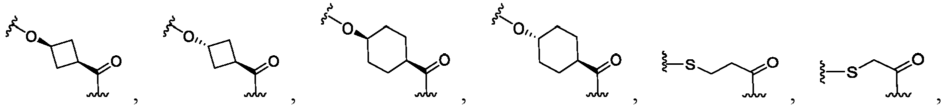

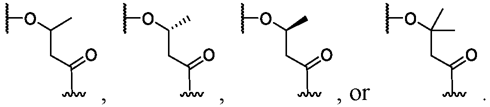

In some embodiments, the present disclosure describes the anti-GPC3 antibody-drug conjugate, the isomer thereof, the pharmaceutically acceptable salt thereof, or the mixture thereof, wherein the cytotoxic drug-M-D 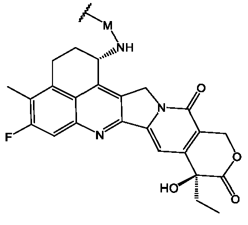 is selected from any one of the following structures:

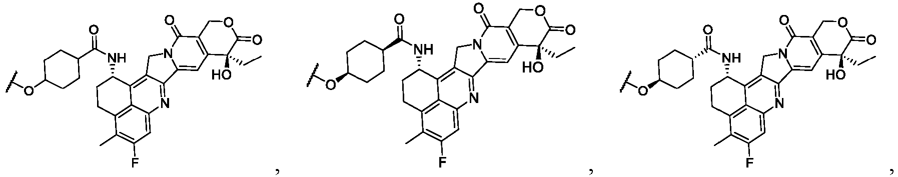

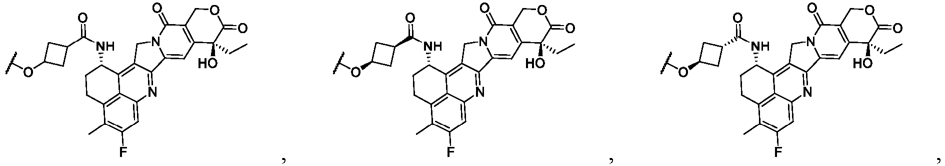

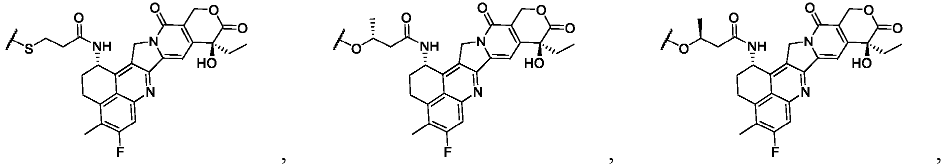

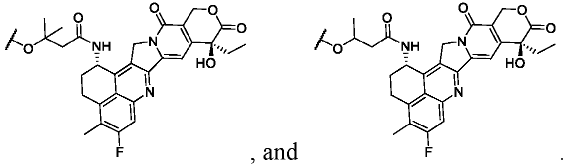

In some embodiments, the present disclosure describes the anti-GPC3 antibody-drug conjugate, the isomer thereof, the pharmaceutically acceptable salt thereof, or the mixture thereof, wherein the L is -Lₐ-L_{b}-L_{c}-,
the -Lₐ- is 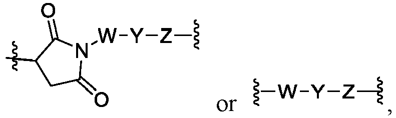
wherein W is -(C(R^{wa})(R^{wb}))_{wn}-, Y is -(OCH₂CH₂)_{yn}-O_{yp}-, and Z is -(C(R^{za})(R^{zb}))_{zn};
wherein wn is 1, 2, 3, or 6;
0 or 1 methylene unit of W is each independently replaced by -Cyr-, -N(R^{wx})C(O)-, - C(O)N(R^{wx})-, or -C(O)-;
wherein yn is 0, 4, or 8, and yp is 0 or 1;
wherein zn is 1, 2, or 3;
1 methylene unit of Z is each independently replaced by -Cyr-, -N(R^{zx})C(O)-, - C(O)N(R^{zx})-, or -C(O)-;
-Cyr- is 3- to 10-membered saturated carbocyclylene, wherein the -Cyr- is unsubstituted or independently substituted by 1 to 3 substituents R^{cx};
wherein R^{wa}, R^{wb}, R^{za}, R^{zb}, R^{wx}, R^{zx}, and R^{cx} are each independently hydrogen, halogen, -OR^{r}, or a C₁₋₆ aliphatic group optionally substituted by R^{r};
wherein each R^{r} is independently hydrogen, halogen, or a C₁₋₆ aliphatic group;
the -L_{b}- is a peptide residue consisting of 2 to 7 amino acids, and the peptide residue of -L_{b}- is formed from amino acids selected from the group consisting of phenylalanine, glycine, alanine, valine, citrulline, lysine, serine, glutamic acid, and aspartic acid; preferably, -L_{b}-represents a peptide residue consisting of 2 to 4 amino acids, and the peptide residue of -L_{b}- is formed from amino acids selected from the group consisting of phenylalanine, glycine, alanine, valine, citrulline, and lysine;
the -L_{c}- is 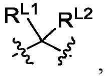
wherein R^{L1} and R^{L2} are each independently selected from the following group consisting of hydrogen, halogen, -OH, and a C₁₋₆ aliphatic group.

In some embodiments, the present disclosure describes the anti-GPC3 antibody-drug conjugate, the isomer thereof, the pharmaceutically acceptable salt thereof, or the mixture thereof, wherein the L is -Lₐ-L_{b}-L_{c}-,
the -Lₐ- is 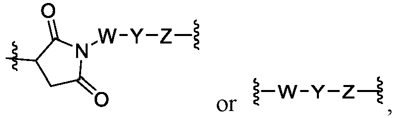
wherein W is -(C(R^{wa})(R^{wb}))_{wn}-, Y is -(OCH₂CH₂)_{yn}-O_{yp}-, and Z is -(C(R^{za})(R^{zb}))_{zn};
wherein wn is 1, 2, 3, or 6;
0 or 1 methylene unit of W is each independently replaced by -Cyr-, -N(R^{wx})C(O)-, - C(O)N(R^{wx})-, or -C(O)-;
wherein yn is 0, 4, or 8, and yp is 0 or 1;
wherein zn is 1, 2, or 3;
1 methylene unit of Z is each independently replaced by -Cyr-, -N(R^{zx})C(O)-, - C(O)N(R^{zx})-, or -C(O)-;
-Cyr- is 3- to 10-membered saturated carbocyclylene, wherein the -Cyr- is unsubstituted or independently substituted by 1 to 3 substituents R^{cx};
wherein R^{wa}, R^{wb}, R^{za}, R^{zb}, R^{wx}, R^{zx}, and R^{cx} are each independently hydrogen, halogen, -OR^{r}, or a C₁₋₆ aliphatic group optionally substituted by R^{r};
wherein each R^{r} is independently hydrogen, halogen, or a C₁₋₆ aliphatic group;
the -L_{b}- is selected from the following group consisting of: 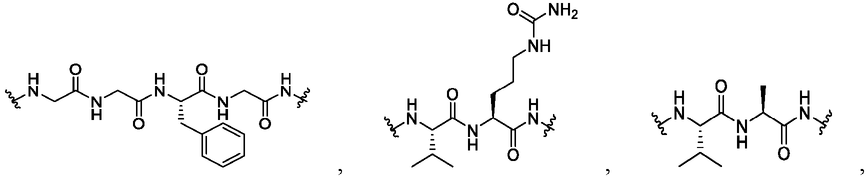 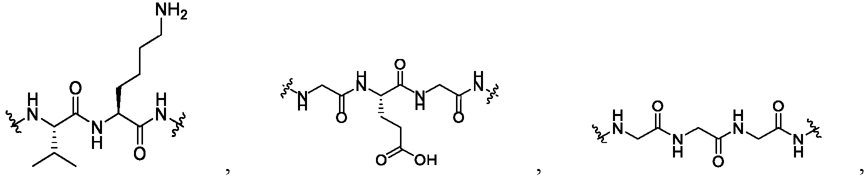 and 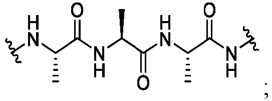
the -L_{c}- is 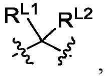
wherein R^{L1} and R^{L2} are each independently selected from the following group consisting of hydrogen, halogen, -OH, and a C₁₋₆ aliphatic group.

In some embodiments, the present disclosure describes the anti-GPC3 antibody-drug conjugate, the isomer thereof, the pharmaceutically acceptable salt thereof, or the mixture thereof, wherein the -Lₐ- is 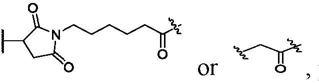 preferably

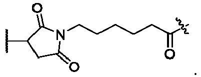

In some embodiments, the present disclosure describes the anti-GPC3 antibody-drug conjugate, the isomer thereof, the pharmaceutically acceptable salt thereof, or the mixture thereof, wherein the -L_{b}- is 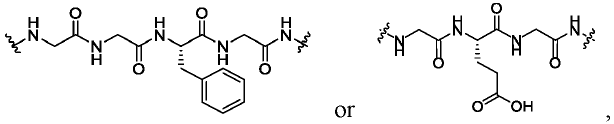 preferably

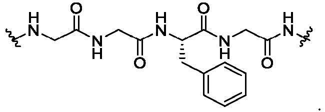

In some embodiments, the present disclosure describes the anti-GPC3 antibody-drug conjugate, the isomer thereof, the pharmaceutically acceptable salt thereof, or the mixture thereof, wherein the -L_{c}- is

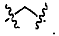

In some embodiments, the present disclosure describes the anti-GPC3 antibody-drug conjugate, the isomer thereof, the pharmaceutically acceptable salt thereof, or the mixture thereof, wherein the Lₐ terminal of linker unit L is connected to Ab, and the L_{c} terminal is connected to the linker unit M.

In some embodiments, the present disclosure describes the anti-GPC3 antibody-drug conjugate, the isomer thereof, the pharmaceutically acceptable salt thereof, or the mixture thereof, wherein the Lₐ terminal of linker unit L is connected to Ab, and the L_{c} terminal is connected to M.

In some embodiments, the present disclosure describes the anti-GPC3 antibody-drug conjugate, the isomer thereof, the pharmaceutically acceptable salt thereof, or the mixture thereof, wherein the L is

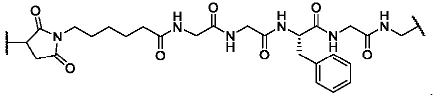

In some embodiments, the present disclosure describes the anti-GPC3 antibody-drug conjugate, the isomer thereof, the pharmaceutically acceptable salt thereof, or the mixture thereof, wherein the L is

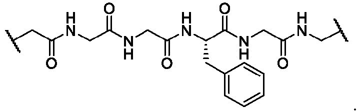

In some embodiments, the present disclosure describes the anti-GPC3 antibody-drug conjugate, the isomer thereof, the pharmaceutically acceptable salt thereof, or the mixture thereof, wherein -L-M-D 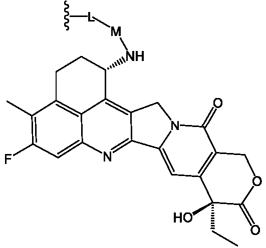 is selected from any one of the following structures: 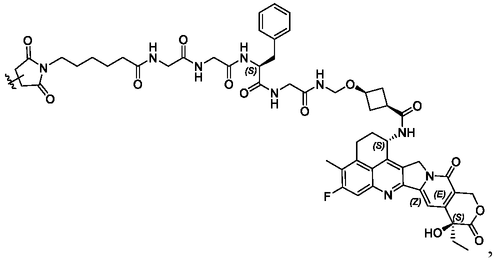 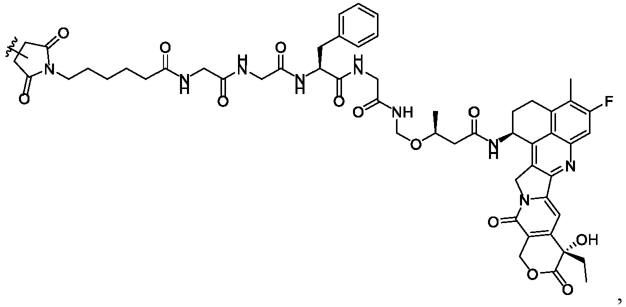 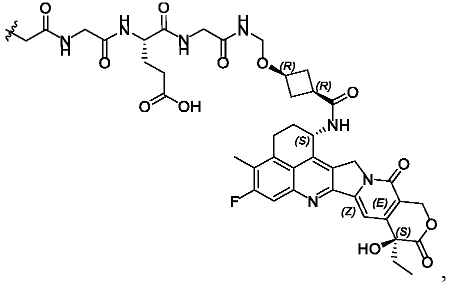 and

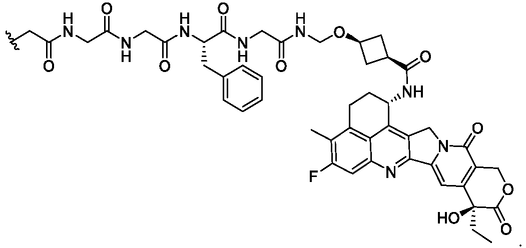

In some embodiments, the present disclosure describes the anti-GPC3 antibody-drug conjugate, the isomer thereof, the pharmaceutically acceptable salt thereof, or the mixture thereof, wherein the anti-GPC3 antibody-drug conjugate has a structure as shown in formula (II-1) or (II-2): 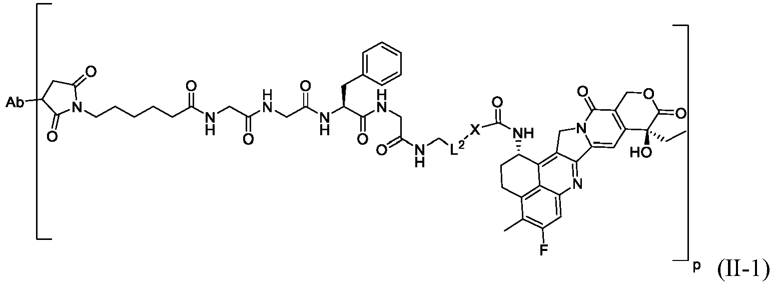 or 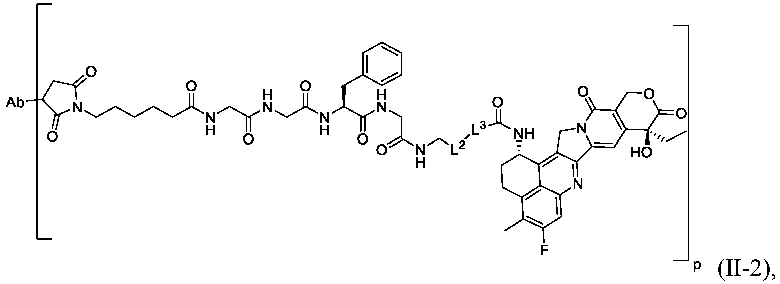 wherein
Ab, L², X, p, and L³ are each as defined in any one of the present disclosure.

In some embodiments, the present disclosure describes the anti-GPC3 antibody-drug conjugate, the isomer thereof, the pharmaceutically acceptable salt thereof, or the mixture thereof, wherein the anti-GPC3 antibody-drug conjugate has a structure as shown in formula (II-1) or (II-2): 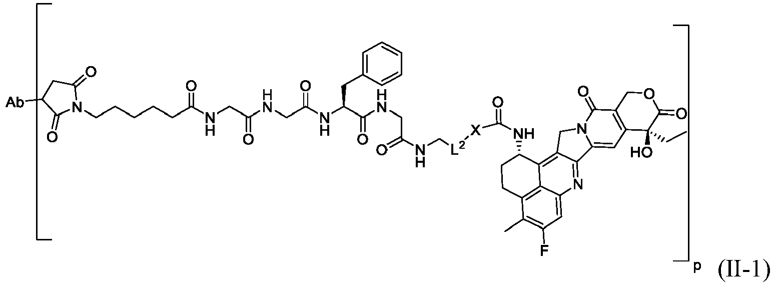 or 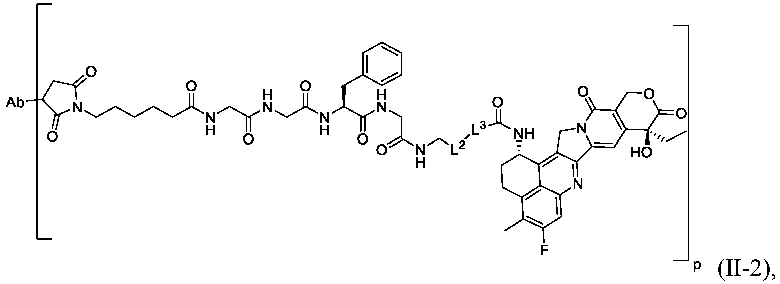 wherein
p represents the average number of connections, and p is an integer or a decimal from 1 to 10, preferably an integer or a decimal from 3 to 8;
Ab is the anti-GPC3 antibody or the antigen-binding fragment thereof as described in any embodiment of the present disclosure;
L² is -O- or -S-;
X is 3- to 6-membered saturated carbocyclyl optionally substituted by 1, 2, or 3 R^{3a}; preferably, X is 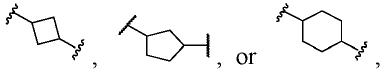 and the 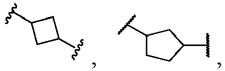 and 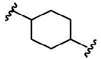 are optionally substituted by 1, 2, or 3 R^{3a};
L³ is -C(R^{1a})(R^{1b})-CH₂- or -C(R^{1a})(R^{1b})C(R^{1a})(R^{1b})-CH₂-;
each R^{1a} is independently halogen or a C₁₋₆ aliphatic group optionally substituted by 1, 2, or 3 R;
R^{1b} and R^{3a} are each independently hydrogen, halogen, or a C₁₋₆ aliphatic group optionally substituted by 1, 2, or 3 R;
wherein each R may independently be hydrogen or halogen.

In some embodiments, the present disclosure describes the anti-GPC3 antibody-drug conjugate, the isomer thereof, the pharmaceutically acceptable salt thereof, or the mixture thereof, wherein the anti-GPC3 antibody-drug conjugate has a structure as shown in formula (II-1) or (II-2): 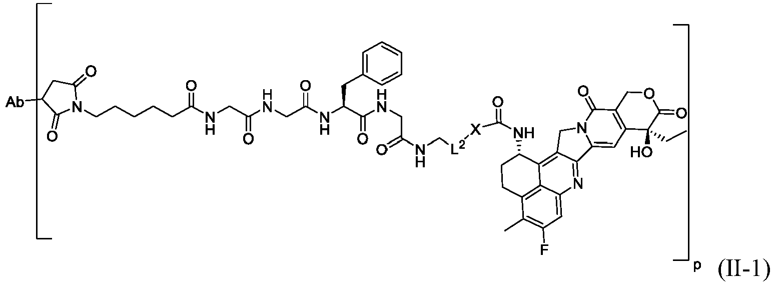 or 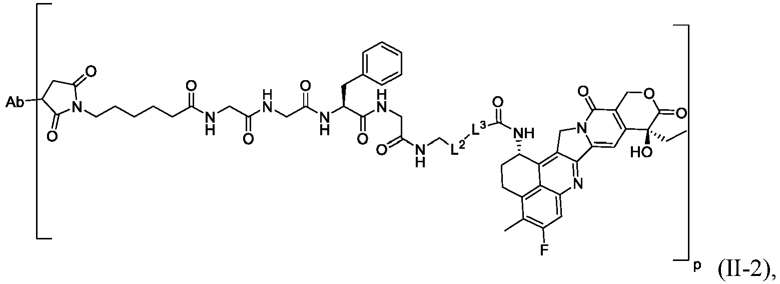 wherein
p represents the average number of connections, and p is an integer or a decimal from 1 to 10, preferably an integer or a decimal from 3 to 8;
Ab is the anti-GPC3 antibody or the antigen-binding fragment thereof as described in any embodiment of the present disclosure;
L² is -O- or -S-;
X is 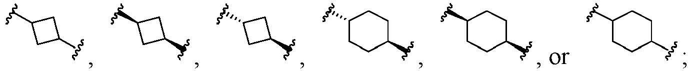
L³ is -CH(CH₃)CH₂- or -C(CH₃)₂CH₂-.

In some embodiments, the present disclosure describes the anti-GPC3 antibody-drug conjugate, the isomer thereof, the pharmaceutically acceptable salt thereof, or the mixture thereof, wherein the anti-GPC3 antibody-drug conjugate is selected from the following structural formulas: 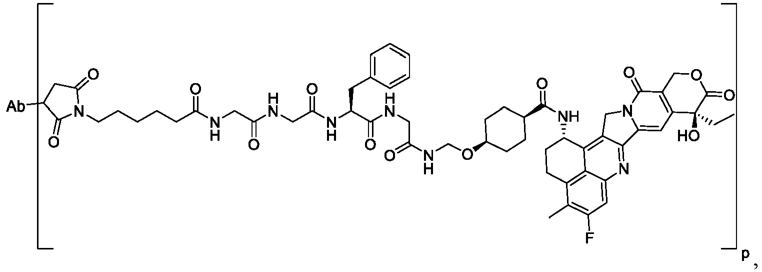 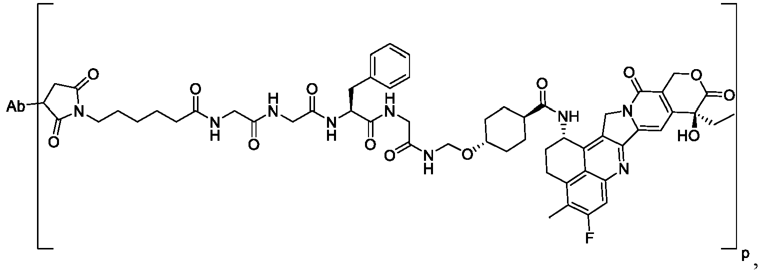 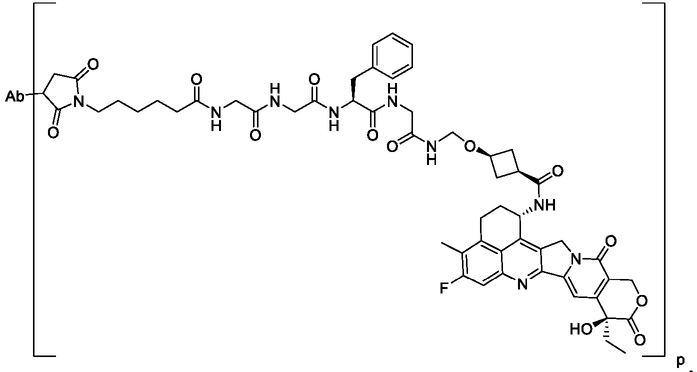 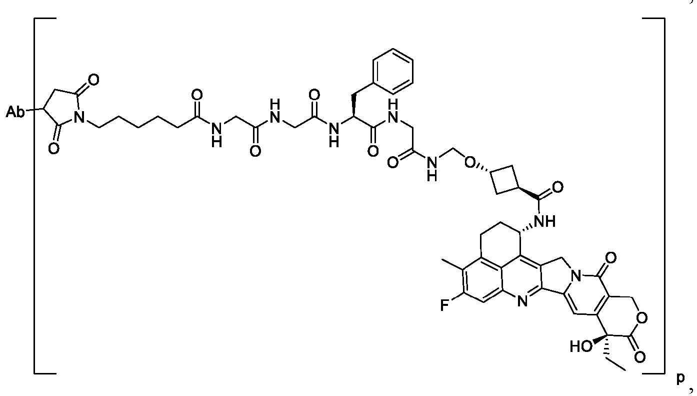 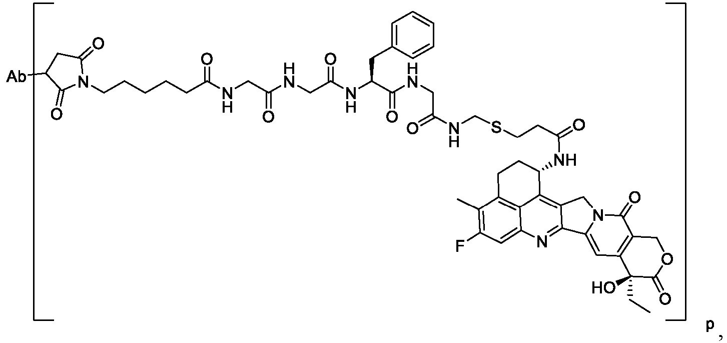 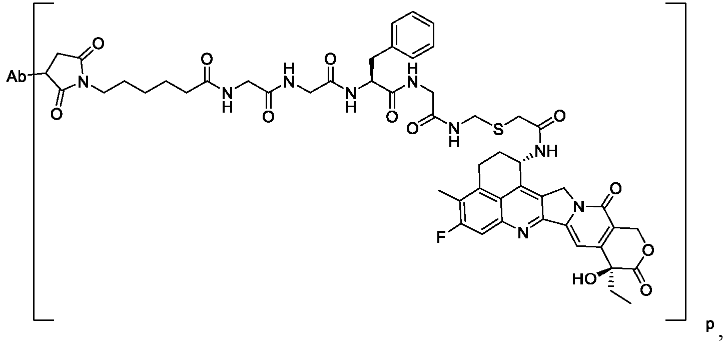 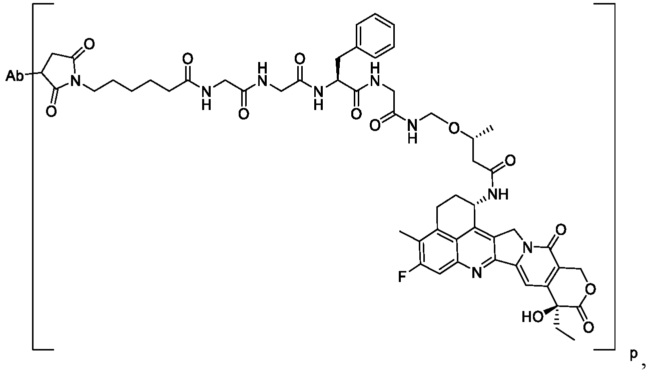 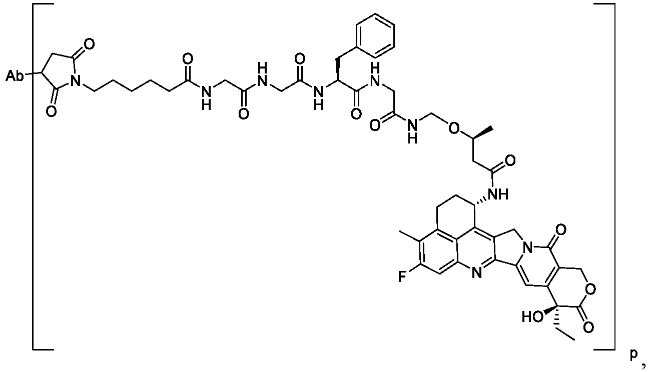 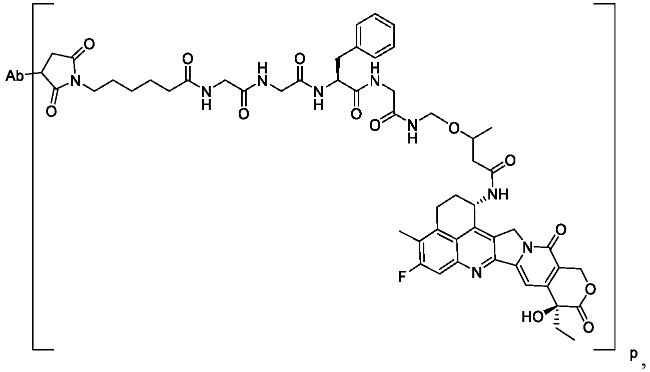 and 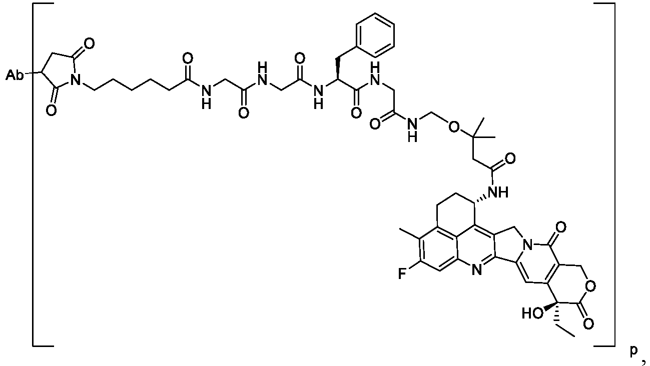 wherein
p represents the average number of connections, and p is an integer or a decimal from 1 to 10, preferably an integer or a decimal from 3 to 8;
Ab is the anti-GPC3 antibody or the antigen-binding fragment thereof as described in any embodiment of the present disclosure.

In yet another aspect, the present disclosure provides an anti-GPC3 antibody-drug conjugate, an isomer thereof, a pharmaceutically acceptable salt thereof, or a mixture thereof, wherein the anti-GPC3 antibody-drug conjugate is selected from: 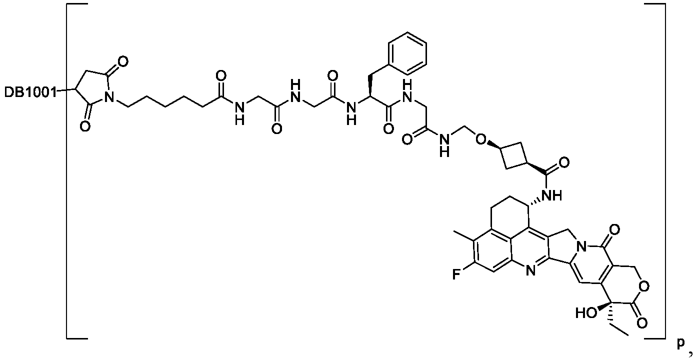 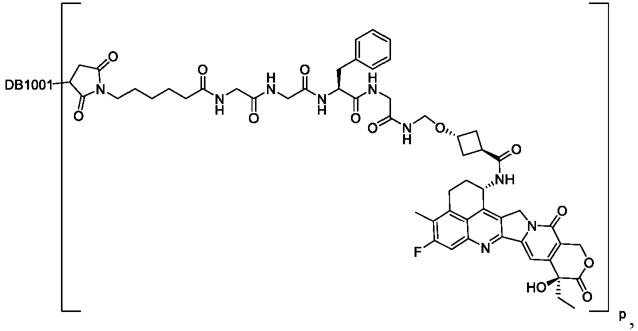 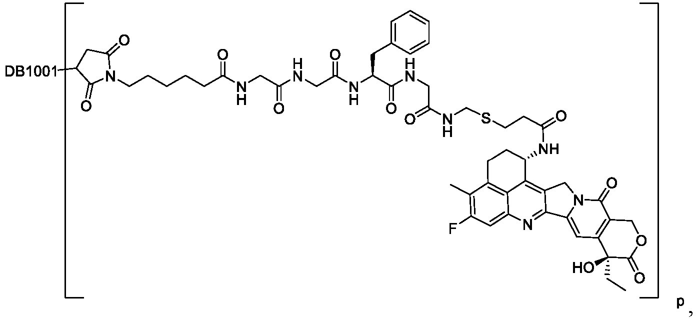 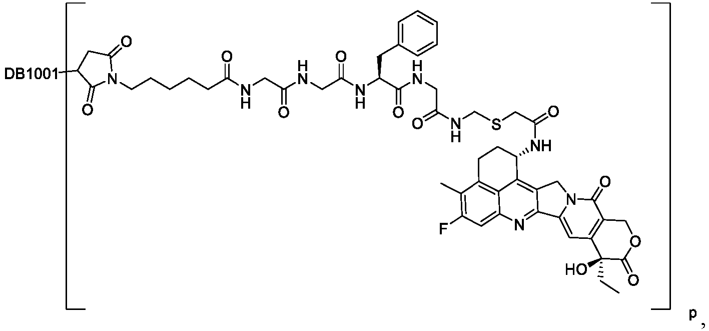 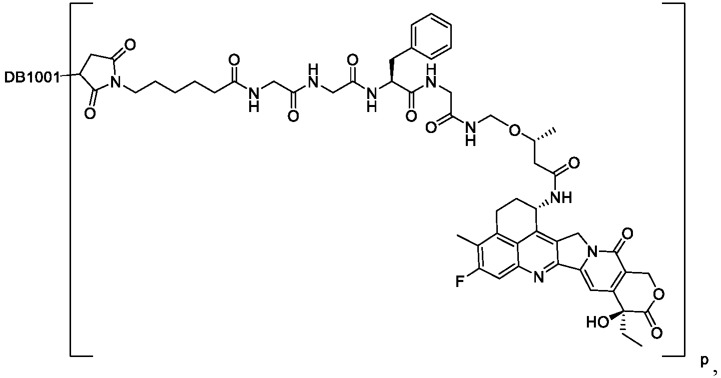 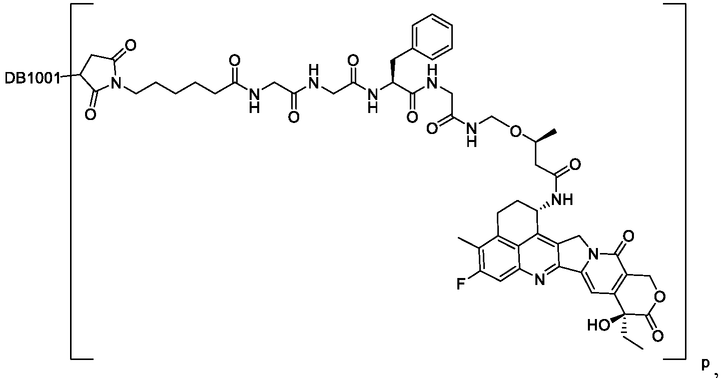 and 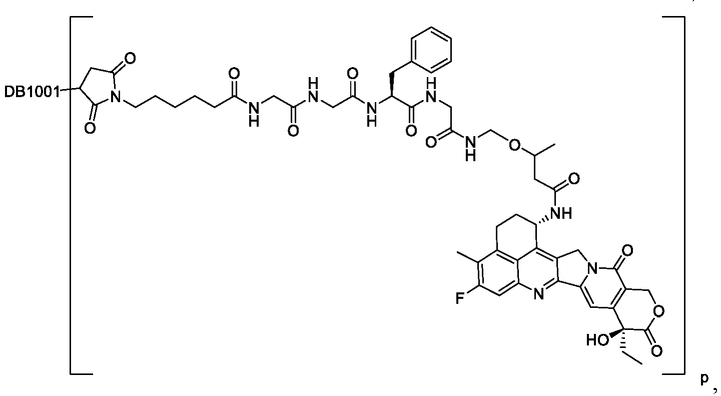 wherein
p represents the average number of connections, and p is an integer or a decimal from 1 to 10, preferably an integer or a decimal from 3 to 8.

In the present disclosure, the DB1001 is an anti-GPC3 antibody, wherein the anti-GPC3 antibody comprises a heavy chain amino acid sequence as shown in SEQ ID NO: 9 and a light chain amino acid sequence as shown in SEQ ID NO: 10.

In some embodiments, the average number of connections p as described in the present disclosure may be an integer or a decimal from 2 to 8. For example, the average number of connections p may be an integer or a decimal from 3 to 8. For example, the average number of connections p may be an integer or a decimal from 1 to 2, 2 to 3, 3 to 4, 4 to 5, 5 to 6, 6 to 7, 7 to 8, 8 to 9, or 9 to 10. For example, the average number of connections p is 7.51 or 7.72.

In yet another aspect, the present disclosure provides an anti-GPC3 antibody-drug conjugate, an isomer thereof, a pharmaceutically acceptable salt thereof, or a mixture thereof, wherein the anti-GPC3 antibody-drug conjugate is selected from any one of the following structures (p represents the average number of connections): 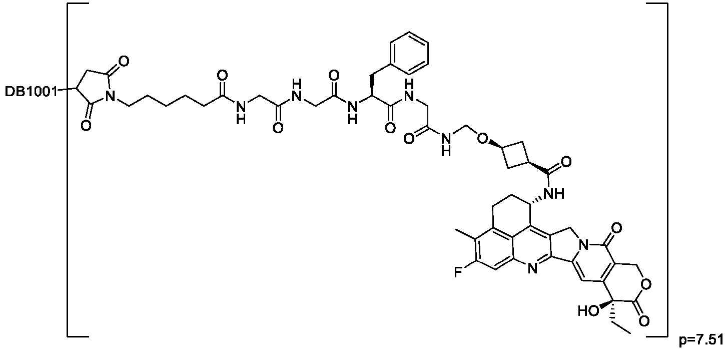 and 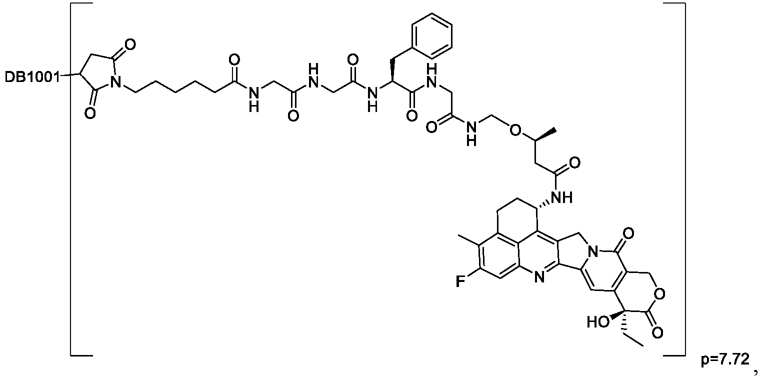 wherein the DB1001 is an anti-GPC3 antibody, wherein the anti-GPC3 antibody has a heavy chain amino acid sequence as shown in SEQ ID NO: 9 and a light chain amino acid sequence as shown in SEQ ID NO: 10.

In yet another aspect, the present disclosure provides a pharmaceutical composition comprising the anti-GPC3 antibody-drug conjugate, the isomer thereof, the pharmaceutically acceptable salt thereof, or the mixture thereof as described in any one of the present disclosure, and a pharmaceutically acceptable carrier or excipient.

In yet another aspect, the present disclosure provides a use of the anti-GPC3 antibody-drug conjugate, the isomer thereof, the pharmaceutically acceptable salt thereof, or the mixture thereof, or the pharmaceutical composition as described in any one of the present disclosure in the manufacture of a medicament for treating and/or preventing a GPC3-mediated disease or disorder; preferably, the disease or disorder is cancer, such as a cancer with positive expression of GPC3.

In yet another aspect, the present disclosure provides a method for treating and/or preventing a GPC3-mediated disease or disorder, comprising administering to a subject in need thereof the anti-GPC3 antibody-drug conjugate, the isomer thereof, the pharmaceutically acceptable salt thereof, or the mixture thereof as described in any one of the present disclosure, or the pharmaceutical composition as described in any one of the present disclosure; preferably, the disease or disorder is cancer, such as a cancer with positive expression of GPC3.

In yet another aspect, the present disclosure provides the anti-GPC3 antibody-drug conjugate, the isomer thereof, the pharmaceutically acceptable salt thereof, or the mixture as described in any one of the present disclosure, or the pharmaceutical composition as described in any one of the present disclosure for use in treating and/or preventing a GPC3-mediated disease or condition; preferably, the disease or disorder is cancer, such as a cancer with positive expression of GPC3.

In some embodiments, the cancer as described in the present disclosure is selected from liver cancer, breast cancer, ovarian cancer, prostate cancer, pancreatic cancer, renal cancer, lung cancer, gastric cancer, colon cancer, bladder cancer, esophageal cancer, cervical cancer, gallbladder cancer, glioblastoma, and melanoma.

In yet another aspect, the present disclosure provides a pharmaceutical combination, comprising the antibody-drug conjugate or the pharmaceutically acceptable salt thereof as described in any one of the present disclosure, or the pharmaceutical composition as described in any one of the present disclosure, and one or more additional therapeutic agents.

In yet another aspect, the present disclosure provides a kit, comprising the antibody-drug conjugate as described in any one of the present disclosure or the pharmaceutical composition as described in any one of the present disclosure.

### Definitions of Terms

Unless otherwise specified, the implementation of the present disclosure will employ conventional techniques of molecular biology (including recombinant technology), microbiology, cell biology, biochemistry, and immunology, all of which fall within the technical scope of the art.

For the purpose of facilitating a better understanding of the present disclosure, certain technical terms are specifically defined as follows. Unless otherwise clearly defined in other parts of this document, the technical terms used herein have the meanings commonly understood by those of ordinary skill in the art. For definitions and terms in the art, one skilled in the art may refer to "Current Protocols in Molecular Biology (Ausubel)." The abbreviations for amino acid residues employed herein correspond to the standard three-letter and/or one-letter codes universally recognized in the art to represent the 20 commonly used L-amino acids. As used herein (including the claims), the singular form shall be construed to include the plural form unless the context clearly dictates otherwise.

In the present disclosure, the term "about" generally refers to a variation within the range of 0.5% to 10% above or below a specified value, such as a variation within the range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below the specified value.

In the present disclosure, the term "antibody" refers to any form of antibody that possesses the desired biological activity. Accordingly, the term is used in its broadest sense, specifically including but not limited to monoclonal antibodies (including full-length monoclonal antibodies), polyclonal antibodies, multispecific antibodies (e.g., bispecific antibodies), humanized antibodies, fully human antibodies, chimeric antibodies, and camelid single-domain antibodies.

In the present disclosure, the term "monoclonal antibody" refers to an antibody obtained from a substantially homogeneous population of antibodies, where each antibody constituting the population is identical, except for naturally occurring mutations that may be present in minor quantities. Monoclonal antibodies are highly specific and are directed against a single antigenic epitope. In contrast, conventional (polyclonal) antibody preparations typically comprise a large number of antibodies directed against (or specific for) different epitopes. The modifier "monoclonal" indicates the characteristics of an antibody obtained from a substantially homogeneous antibody population and should not be construed as requiring the antibody to be produced by any particular method.

In the present disclosure, the term "full-length antibody" refers to an immunoglobulin molecule that, when naturally occurring, comprises four polypeptide chains: two heavy (H) chains (approximately 50-70 kDa in full-length) and two light (L) chains (approximately 25 kDa in full-length), linked to each other by disulfide bonds. Each heavy chain consists of a variable region of the heavy chain (abbreviated as VH herein) and a constant region of the heavy chain (abbreviated as CH herein). The constant region of the heavy chain consists of three domains: CH1, CH2, and CH3. Each light chain consists of a variable region of the light chain (abbreviated as VL herein) and a constant region of the light chain. The constant region of the light chain consists of one domain, CL. The VH and VL regions can be further subdivided into complementarity-determining regions (CDRs) with high variability and regions called framework regions (FRs) whose spacing is more conserved. Each VH or VL region consists of three CDRs and four FRs, arranged in the following order from the amino terminus to the carboxyl terminus: FR1, CDR1, FR2, CDR2, FR3, CDR3, and FR4. The variable regions of the heavy and light chains contain the binding domains that interact with the antigen. The constant regions of the antibody can mediate the binding of the immunoglobulin to host tissues or factors, including various cells of the immune system (e.g., effector cells) and the first component of the classical complement system (C1q).

In the present disclosure, the term "CDR" refers to the complementarity-determining regions within the variable sequences of an antibody. There are three CDRs present in each variable region of the heavy and light chains, designated as HCDR1, HCDR2, and HCDR3 for the heavy chain, and LCDR1, LCDR2, and LCDR3 for the light chain. The precise amino acid sequence boundaries of the CDRs in the variable regions of the antibodies described in the present disclosure can be determined using any of a number of well-known schemes, including: Chothia based on the three-dimensional structure of antibodies and the topology of CDR loops (Chothia et al., (1989) Nature 342:877-883; Al-Lazikani et al., "Standard conformations for the canonical structures of immunoglobulins", Journal of Molecular Biology, 273, 927-948 (1997)); Kabat based on antibody sequence variability (Kabat et al., Sequences of Proteins of Immunological Interest, 4th edition, U.S. Department of Health and Human Services, National Institutes of Health (1987)); AbM (University of Bath); Contact (University College London); the international ImMunoGeneTics database (IMGT) (1999 Nucleic Acids Research, 27, 209-212); and the North CDR definition based on affinity propagation clustering using a large set of crystal structures. The boundaries of the CDRs of the antibodies described in the present disclosure can be determined by those skilled in the art according to any scheme in the art (e.g., different assignment systems or combinations thereof).

It should be noted that the boundaries of CDRs in the variable region of the same antibody obtained based on different assignment systems may be different. In other words, the CDR sequences of the same antibody variable region defined under different assignment systems are different. Therefore, when referring to an antibody defined by a specific CDR sequence described in the present disclosure, the scope of the antibody also includes those antibodies whose variable region sequences contain the specific CDR sequences, but whose claimed CDR boundaries are different from those of the specific CDR boundaries as defined by the present disclosure due to the application of different schemes (e.g., different assignment systems or combinations thereof).

In the present disclosure, the term "antigen-binding fragment" of an antibody ("parent antibody") includes fragments or derivatives of the antibody, typically comprising at least one fragment of the antigen-binding region or variable region (e.g., one or more CDRs) of the parent antibody, which retains at least some of the binding specificity of the parent antibody. Examples of antibody-binding fragments include, but are not limited to, Fab, Fab', F(ab')₂, and Fv fragments; bispecific antibodies; linear antibodies; single-chain antibody molecules such as scFv; nanobodies formed from antibody fragments; and multispecific antibodies. When antigen-binding activity is expressed on a molar concentration basis, the binding fragment or derivative typically retains at least 10% of the antigen-binding activity of the parent antibody. Preferably, the binding fragment or derivative retains at least 20%, 50%, 70%, 80%, 90%, 95%, or 100% or more of the antigen-binding affinity of the parent antibody. It is also anticipated that the antigen-binding fragments of the antibody may include conservative or non-conservative amino acid substitutions (referred to as "conservative variants" or "functionally conservative variants" of the antibody) that do not significantly alter its biological activity.

In the present disclosure, the term "chimeric antibody" refers to an antibody that comprises the variable domain of a first antibody and the constant domain of a second antibody, wherein the first and second antibodies are derived from different species. Typically, the variable domains are derived from antibodies such as rodents ("parental antibodies"), while the constant domain sequences are derived from human antibodies, making the resulting chimeric antibodies less likely to induce an adverse immune response in human subjects compared to parental rodent antibodies.

In the present disclosure, the term "humanized antibody" refers to an antibody form that contains sequences from both human and non-human (e.g., mouse, rat) antibodies. Generally, a humanized antibody includes substantially all of at least one, typically two, variable domains, wherein all or substantially all of the hypervariable loops correspond to those of the non-human immunoglobulin, while all or substantially all of the framework regions (FRs) are framework regions of human immunoglobulin sequence. A humanized antibody may optionally include at least a portion of a human immunoglobulin constant region (Fc).

In the present disclosure, the term "fully human antibody" refers to an antibody that contains only human immunoglobulin protein sequences. If produced in mice, mouse cells, or hybridomas derived from mouse cells, a fully human antibody may contain murine glycan chains. Likewise, the term "mouse antibody" refers to an antibody that contains only mouse immunoglobulin sequences. Alternatively, if produced in rats, rat cells, or hybridomas derived from rat cells, a fully human antibody may contain rat glycan chains. Likewise, the term "rat antibody" refers to an antibody that contains only rat immunoglobulin sequences.

In the present disclosure, the term "isotype" antibody refers to the class of antibody provided by the heavy chain constant region genes (e.g., IgM, IgE, IgG such as IgG1, IgG2, or IgG4). Isotypes also include modified forms of one of these classes, where modifications have been made to alter Fc function, for example, to enhance or weaken effector function or binding to Fc receptors.

In the present disclosure, the term "Fc region" is used to define the C-terminal region of an immunoglobulin heavy chain that contains at least a portion of the constant region. This term includes both native sequence Fc regions and variant Fc regions. In some embodiments, the human IgG heavy chain Fc region extends from Cys226 or Pro230 to the carboxyl terminus of the heavy chain. However, the C-terminal lysine (Lys447) of the Fc region may be present or absent (the numbering in this paragraph is based on the EU numbering system, also known as the EU index, as described by Rabat et al., Sequences of Proteins of Immunological Interest, 5th Ed., Public Health Service, National Institutes of Health, Bethesda, MD, 1991).

In the present disclosure, the term "cross reaction" refers to the binding of antigen fragments of the same target molecule from human, monkey, and/or murine (mouse or rat). Therefore, "cross reaction" should be understood as an interspecies reaction with the same molecule X expressed in different species. The specificity of the cross reaction of a monoclonal antibody that recognizes human GPC3R, monkey GPC3R, and/or murine GPC3R (mouse or rat) can be determined by FACS analysis.

In the present disclosure, the term "affinity" or "binding affinity" refers to the inherent binding affinity that reflects the interaction between members of a binding pair. The affinity of molecule X for its partner Y can typically be represented by the equilibrium dissociation constant (KD), which is the ratio of the dissociation rate constant to the binding rate constant (kdis and kon, respectively). Affinity can be measured by common methods known in the art. One specific method for measuring affinity is the ForteBio kinetic binding assay mentioned herein.

In the present disclosure, the term "non-binding" protein or cell refers to not binding to the protein or cell, or not binding to the protein or cell with high affinity, i.e., binding to the protein or cell with a K_{D} of 1.0 × 10⁻⁶ M or higher, more preferably 1.0 × 10⁻⁵ M or higher, further more preferably 1.0 × 10⁻⁴ M or higher, 1.0 × 10⁻³ M or higher, still more preferably 1.0 × 10⁻² M or higher.

In the present disclosure, the term "high affinity" for an IgG antibody refers to a K_{D} for the antigen of 1.0 × 10⁻⁶ M or lower, preferably 5.0 × 10⁻⁸ M or lower, more preferably 1.0 × 10⁻⁸ M or lower, 5.0 × 10⁻⁹ M or lower, and further more preferably 1.0 × 10⁻⁹ M or lower. For other antibody isotypes, "high affinity" binding may vary. For example, "high affinity" binding for the IgM isotype refers to a K_{D} of 10⁻⁶ M or lower, preferably 10⁻⁷ M or lower, and more preferably 10⁻⁸ M or lower.

In the present disclosure, the term "cytotoxic drug" generally refers to a toxic drug that comprises chemical molecules capable of significantly disrupting the normal growth of tumor cells. Cytotoxic drugs can kill tumor cells at sufficiently high concentrations. The term "cytotoxic drug" may include payloads, such as small molecule payloads or enzymatically active payloads derived from bacterial, fungal, plant, or animal sources; radioactive isotopes (e.g., At²¹¹, I¹³¹, I¹²⁵, Y⁹⁰, Re¹⁸⁶, Re¹⁸⁸, Sm¹⁵³, Bi²¹², P³², or radioactive isotopes of Lu); toxic agents; chemotherapeutic drugs; antibiotics; and nucleolytic enzymes. For example, cytotoxic drugs may be toxic drugs, including but not limited to, camptothecin derivatives, such as exatecan (chemical name: (1S,9S)-1-amino-9-ethyl-5-fluoro-2,3-dihydro-9-hydroxy-4-methyl-1H,12H-benzo[de]pyrano[3',4' :6,7]indolizino[1,2-b]quinoline-10,13(9H,15H)-dione).

In the present disclosure, the term "linker unit" or "linker structure" generally refers to a chemical structural fragment or bond that connects a ligand at one end and a cytotoxic drug at the other end. It may also connect to other linkers before connecting to the cytotoxic drug. The direct or indirect connection to the ligand may mean that the group is directly connected to the ligand via a covalent bond or connected through a linker structure. For example, the linker structure may be the structure represented by -Lax-Lb-Lc- and/or -La-Lb-Lc- as described in the present disclosure. For example, chemical structure fragments or bonds containing acid-labile linker structures (e.g., hydrazones), protease-sensitive (e.g., peptidase-sensitive) linker structures, photo-labile linker structures, dimethyl linker structures, or disulfide-containing linker structures can be used as linker structures.

In the present disclosure, the term "optionally connected to other molecular moieties" typically refers to a structure that is either not connected to any other chemical structure or is connected to one or more other chemical structures distinct from the structure itself (e.g., the ligand described in the present disclosure), such as through a chemical bond or a linker structure.

In the present disclosure, the term "ligand-drug conjugate" generally refers to a ligand that is connected to a biologically active cytotoxic drug through a stable linker unit. In the present disclosure, a "ligand-drug conjugate" may be an antibody-drug conjugate (ADC), which refers to a monoclonal antibody or an antibody fragment connected to a biologically active cytotoxic drug through a stable linker unit.

In the present disclosure, the term "methylene" typically refers to a residue derived from the removal of two hydrogen atoms from a group of one carbon atom. Methylene may be substituted or unsubstituted, and it may be replaced or unreplaced. The term "alkylene" typically refers to a saturated, linear or branched aliphatic hydrocarbon group containing 2 residues derived from the removal of two hydrogen atoms from the same or different carbon atoms of the parent alkane. It can include linear or branched groups containing 1 to 20 carbon atoms, such as alkylene containing 1 to 12 carbon atoms, or such as alkylene containing 1 to 6 carbon atoms. Non-limiting examples of alkylene groups include, but are not limited to, methylene (-CH₂-), 1,1-ethylene (-CH(CH₃)-), 1,2-ethylene (-CH₂CH₂-), 1,1-propylene (-CH(CH₂CH₃)-), 1,2-propylene (-CH₂CH(CH₃)-), 1,3-propylene (-CH₂CH₂CH₂-), 1,4-butylene (-CH₂CH₂CH₂CH₂-), and 1,5-butylene (-CH₂CH₂CH₂CH₂CH₂-), etc. Alkylene groups may be substituted or unsubstituted, replaced or unreplaced. For example, when substituted, the substituent may be substituted at any available connection point. The substituents are preferably independently and optionally substituted by one or more substituents selected from alkyl, alkenyl, alkynyl, alkoxy, alkylthio, alkylamino, halogen, mercapto, hydroxyl, nitro, cyano, cycloalkyl, heterocyclyl, aryl, heteroaryl, cycloalkoxy, heterocycloalkoxy, cycloalkylthio, heterocycloalkylthio, and oxo groups. Examples of substituents may be hydrogen, deuterium, tritium, halogen, -NO₂, -CN, -OH, -SH, -NH₂, -C(O)H, -CO₂H, - C(O)C(O)H, -C(O)CH₂C(O)H, -S(O)H, -S(O)₂H, -C(O)NH₂, -SO₂NH₂, -OC(O)H, - N(H)SO₂H, or a C₁₋₆ aliphatic group.

In the present disclosure, the term "heterocyclylene" or "heterocyclyl" generally refers to a stable, non-aromatic 3- to 7-membered monocyclic structure, fused 7- to 10-membered bicyclic heterocyclic structure, or bridged 6- to 10-membered bicyclic heterocyclic structure. These cyclic structures can be saturated or partially saturated and contain one or more heteroatoms in addition to carbon atoms, where the heteroatoms may be selected from the group consisting of oxygen, sulfur, and nitrogen. For example, containing 1 to 4 heteroatoms as defined above. When referring to atoms on the heterocyclic ring structure, the term "nitrogen" may include nitrogen that has undergone substitution reactions. Heterocyclylene groups may be substituted or unsubstituted.

In the present disclosure, the term "carbocyclylene" typically refers to a residue derived from the removal of two hydrogen atoms from either the same or different carbon atoms of a carbon ring. The term "carbocyclic ring" generally refers to a saturated or partially unsaturated monocyclic or polycyclic cyclic hydrocarbon, containing 3 to 20 carbon atoms, may contain 3 to 12 carbon atoms, may contain 3 to 10 carbon atoms, may contain 3 to 8 carbon atoms, and for example, containing 3 to 6 carbon atoms. Non-limiting examples of monocyclic carbocyclic rings include cyclopropane, cyclobutane, cyclopentane, cyclopentene, cyclohexane, cyclohexene, l,3-cyclohexadiene, cycloheptane, cycloheptatriene, cyclooctane, etc. Polycyclic carbocyclic rings may include spiro, fused, and bridged carbocyclic rings. For example, "3- to 6-membered saturated carbocyclyl" includes cyclopropyl, cyclobutyl, cyclopentyl, cyclohexyl, cyclopropylene, cyclobutylene, cyclopentylene, or cyclohexylene. Carbocyclyl and carbocyclylene may be substituted or unsubstituted, e.g., may be substituted by one or more halogens, C₁₋₆ aliphatic groups, or halogen-substituted C₁₋₆ aliphatic groups.

In the present disclosure, the term "partially unsaturated" generally refers to a cyclic structure that contains at least one double bond or triple bond between ring atoms. The term "partially unsaturated" includes cyclic structures with multiple unsaturations, but is not intended to encompass aromatic rings or heteroaromatic rings as defined in the present disclosure. The term "unsaturated" means that the moiety has one or more degrees of unsaturation.

In the present disclosure, the term "halogen" generally refers to fluorine, chlorine, bromine, or iodine, for example, fluorine or chlorine.

In the present disclosure, the term "aliphatic group" generally refers to a linear, branched, or cyclic hydrocarbon structure containing 1 to 12 carbon atoms. These hydrocarbons can be fully saturated or contain one or more unsaturated units, provided that such unsaturated units are not aromatic. For example, suitable aliphatic groups can include substituted or unsubstituted linear, branched, or cyclic alkyl, alkenyl, alkynyl groups, or mixtures thereof; such as (cycloalkyl)alkyl, (cycloalkenyl)alkyl, or (cycloalkyl)alkenyl. For example, aliphatic groups have 1-12, 1-8, 1-6, 1-4, or 1-3 carbon atoms. For example, a "C₁₋₆ aliphatic group" refers to the aliphatic group as described above containing 1 to 6 carbon atoms, including but not limited to, linear, branched, or cyclic alkyl, alkenyl, or alkynyl groups containing 1 to 6 carbon atoms, such as methyl, ethyl, propyl, isopropyl, n-butyl, isobutyl, *tert-*butyl, pentyl, hexyl, etc.

In the present disclosure, the term "optional" or "optionally" generally means that the described event or condition may but does not necessarily occur, and that the description includes both cases where the event or condition does or does not occur. For example, "heterocyclyl optionally substituted by an alkyl group" means that the alkyl group may or may not be present, and the description includes both cases where the heterocyclyl is substituted by an alkyl group and where it is not.

In the present disclosure, the term "substituted" typically means that one or more hydrogen atoms (for example, up to 5, for another example, 1 to 3 hydrogen atoms) are independently substituted in a group by a corresponding number of substituents. Substituents are only located at their possible chemical positions, and a person skilled in the art is able to determine (by experiment or theory) possible or impossible substitutions without undue effort. For example, an amino or hydroxyl group with a free hydrogen may be unstable when bonded to a carbon atom with unsaturated (e.g., olefinic) bonds.

In the present disclosure, the term "0 or more" (e.g., 0 or at least 1, 0 or 1, 0) methylene units are "replaced" generally means that when the structure contains 1 or more methylene units, the one or more methylene units may not be replaced or replaced by one or more groups that are not methylene (e.g., -NHC(O)-, -C(O)NH-, -C(O)-, -OC(O)-, -C(O)O-, -NH-, -O-, -S-, -SO-, -SO₂-, -PH-, -P(=O)H-, -NHSO₂-, -SO₂NH-, -C(=S)-, -C(=NH)-, -N=N-, -C=N-, -N=C-, or -C(=N₂)-).

In the present disclosure, one or more hydrogen atoms (for example, up to 5, for another example, 1 to 3 hydrogen atoms) are independently substituted in a group by a corresponding number of substituents. Substituents are only located at their possible chemical positions, and a person skilled in the art is able to determine (by experiment or theory) possible or impossible substitutions without undue effort. For example, an amino or hydroxyl group with a free hydrogen may be unstable when bonded to a carbon atom with unsaturated (e.g., olefinic) bonds.

In the present disclosure, the term "pharmaceutically acceptable salt" or "medicinal salt" typically refers to salts of the drug conjugate of the present disclosure that are safe and/or effective when administered to a mammal and have the desired biological activity. The antibody-drug conjugate of the present disclosure may form salts with acids. Non-limiting examples of pharmaceutically acceptable salts include hydrochloride, hydrobromide, hydroiodide, sulfate, bisulfate, citrate, acetate, succinate, ascorbate, oxalate, nitrate, sorbate, biphosphate, dihydrogen phosphate, salicylate, hydrogen citrate, tartrate, maleate, fumarate, formate, benzoate, methanesulfonate, ethanesulfonate, benzenesulfonate, and p-toluenesulfonate.

In the present disclosure, the term "solvate" or "solvent compound" generally refers to pharmaceutically acceptable solvates formed by the drug conjugate of the present disclosure with one or more solvent molecules. Non-limiting examples of solvent molecules include water, ethanol, acetonitrile, isopropanol, DMSO, and ethyl acetate.

In the present disclosure, the term "drug loading" generally refers to the average number of cytotoxic drug molecules loaded onto each ligand, which can also be expressed as the ratio of the amount of cytotoxic drug to antibody. The range of cytotoxic drug loading can be 0-12 cytotoxic drug molecules per ligand (Ab), for example, 1-10 cytotoxic drug molecules. In an embodiment of the present disclosure, drug loading is represented as Na, with exemplary mean values of 1, 2, 3, 4, 5, 6, 7, 8, 9, or 10. The drug loading of each ADC molecule after the conjugation reaction can be characterized by conventional methods such as UV/visible spectroscopy, mass spectrometry, ELISA, and HPLC.

In the present disclosure, the term "pharmaceutically acceptable carrier" refers to ingredients of a pharmaceutical preparation or composition, other than the active ingredient, that are non-toxic to the subject. Pharmaceutically acceptable carriers include, but are not limited to, buffers, excipients, stabilizers, or preservatives.

In the present disclosure, the term "comprise", "contain", or "include" generally means including the explicitly stated features but does not exclude the presence of other elements. The terms "above" and "below" usually refer to the case where the number itself is included.

In the present disclosure, the term "subject" includes any human or non-human animal. The term "non-human animal" includes all vertebrates, such as mammals and non-mammals, such as non-human primates, sheep, dogs, cats, horses, cattle, chickens, amphibians, reptiles, etc. As used herein, the term "cyno" or "cynomolgus monkey" refers to the cynomolgus monkey.

In the present disclosure, the term "in combination with" the administration of one or more other therapeutic agents includes simultaneous (co-administration) and sequential administration in any order.

In the present disclosure, the terms "therapeutically effective amount", "therapeutically effective dose", and "effective amount" refer to the amount of the GPC3 antibody or its antigen-binding fragment of the present disclosure that, when administered alone or in combination with other therapeutic drugs to a cell, tissue, or subject, is effective in preventing or ameliorating one or more symptoms of a disease or condition, or in inhibiting the progression of the disease or condition. A therapeutically effective dose also refers to the amount of antibody or its antigen-binding fragment sufficient to cause an improvement in symptoms, such as treating, curing, preventing, or ameliorating a relevant medical condition, or enhancing the rate of treatment, cure, prevention, or amelioration of such condition. When the active ingredient is administered alone to a subject, the therapeutically effective dose refers solely to that component. When administered in combination, the therapeutically effective dose refers to the combined amount of active ingredients that produce a therapeutic effect, regardless of whether they are administered simultaneously, sequentially, or in combination. The effective amount of a therapeutic agent will result in at least a 10% improvement in diagnostic criteria or parameters, typically at least 20%, preferably at least about 30%, more preferably at least 40%, and most preferably at least 50%.

In the present disclosure, the term "cancer" is used herein to refer to a group of cells that exhibit abnormally high levels of proliferation and growth. Cancer may be benign (also referred to as benign tumors), premalignant, or malignant. Cancer cells can be either solid tumor cells or leukemic cancer cells. The term "tumor" as used herein refers to one or more cells that comprise cancer.

In the present disclosure, the term "tumor growth" is used herein to refer to the proliferation or growth of one or more cells containing cancer, which leads to a corresponding increase in the size or extent of the cancer.

### Anti-GPC3 Antibody

In the present disclosure, the term "GPC3" includes any variant or isotype of GPC3 that is naturally expressed by cells. GPC3 or any variant or isotype thereof can be isolated from cells or tissues that naturally express them or can be produced using recombinant techniques known in the art, as well as those described herein. Preferably, the anti-GPC3 antibody targets humanized GPC3 with a normal glycosylation pattern.

In the present disclosure, the term "anti-GPC3 antibody", "anti-GPC3", "GPC3 antibody", or "antibody that binds GPC3" refers to an antibody capable of binding to the GPC3 protein or its fragments with sufficient affinity such that the antibody can be used as a diagnostic agent or therapeutic agent for targeting GPC3.

The anti-GPC3 antibody or the antigen-binding fragment thereof described in the present disclosure references the anti-GPC3 antibody or the antigen-binding fragment thereof as described in WO2006006693.

In some embodiments, the CDR sequences of the antibody used in the drug conjugate, composition, use, or method of the present disclosure comprise the amino acid sequences of HCDR1, HCDR2, and HCDR3 as shown in SEQ ID NO: 1, SEQ ID NO: 2, and SEQ ID NO: 3, respectively, and the amino acid sequences of LCDR1, LCDR2, and LCDR3 as shown in SEQ ID NO: 4, SEQ ID NO: 5, and SEQ ID NO: 6, respectively.

In some embodiments, the variable region sequences of the antibody used in the drug conjugate, composition, or use of the present disclosure comprise a heavy chain variable region having an amino acid sequence as shown in SEQ ID NO: 7 and a light chain variable region having an amino acid sequence as shown in SEQ ID NO: 8.

In some embodiments, the antibody used in the drug conjugate, composition, use, or method of the present disclosure comprises a heavy chain variable region having an amino acid sequence as shown in SEQ ID NO: 9 and a light chain variable region having an amino acid sequence as shown in SEQ ID NO: 10. In some embodiments, the antibody used in the drug conjugate, composition, use, or method of the present disclosure is anti-GPC3 antibody DB1001.

The variable region amino acid sequences of the anti-GPC3 antibody DB1001 are as follows, with the CDR regions determined according to the IMGT numbering rules.
DB1001 heavy chain variable region: 
QVQLVQSGAEVKKPGASVKVSCKASGYTFTDYEMHWVRQAPGQGLEWMG
ALDPKTGDTAYSQKFKGRVTLTADKSTSTAYMELSSLTSEDTAVYYCTRFYSYTYWG
QGTLVTVSS (SEQ ID NO: 7)
HCDR1: DYEMH (SEQ ID NO: 1)
HCDR2: ALDPKTGDTAYSQKFKG (SEQ ID NO: 2)
HCDR3: FYSYTY (SEQ ID NO: 3)
DB1001 light chain variable region: 
DVVMTQSPLSLPVTPGEPASISCRSSQSLVHSNRNTYLHWYLQKPGQSPQLLI
YKVSNRFSGVPDRFSGSGSGTDFTLKISRVEAEDVGVYYCSQNTHVPPTFGQGTKLEI
K (SEQ ID NO: 8)
LCDR1: RSSQSLVHSNRNTYLH (SEQ ID NO: 4)
LCDR2: KVSNRFS (SEQ ID NO: 5)
LCDR3: SQNTHVPPT (SEQ ID NO: 6)
DB1001 heavy chain (IgG1) amino acid sequence 
QVQLVQSGAEVKKPGASVKVSCKASGYTFTDYEMHWVRQAPGQGLEWMG
ALDPKTGDTAYSQKFKGRVTLTADKSTSTAYMELSSLTSEDTAVYYCTRFYSYTYWG
QGTLVTVSSASTKGPSVFPLAPSSKSTSGGTAALGCLVKDYFPEPVTVSWNSGALTSG
VHTFPAVLQSSGLYSLSSVVTVPSSSLGTQTYICNVNHKPSNTKVDKKVEPKSCDKTH
TCPPCPAPELLGGPSVFLFPPKPKDTLMISRTPEVTCVVVDVSHEDPEVKFNWYVDGV
EVHNAKTKPREEQYNSTYRVVSVLTVLHQDWLNGKEYKCKVSNKALPAPIEKTISKA
KGQPREPQVYTLPPSRDELTKNQVSLTCLVKGFYPSDIAVEWESNGQPENNYKTTPPV
LDSDGSFFLYSKLTVDKSRWQQGNVFSCSVMHEALHNHYTQKSLSLSPGK (SEQ ID
NO: 9)
DB1001 light chain (Kappa) amino acid sequence

DVVMTQSPLSLPVTPGEPASISCRSSQSLVHSNRNTYLHWYLQKPGQSPQLLI
YKVSNRFSGVPDRFSGSGSGTDFTLKISRVEAEDVGVYYCSQNTHVPPTFGQGTKLEI
KRTVAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC (SEQ ID
NO: 10)

Any suitable method for producing antibodies can be employed to generate the antibodies of the present disclosure. Any suitable form of GPC3 can be used as an immunogen (antigen) for generating the antibody. By way of example and not limitation, any GPC3 variant or fragment thereof can be used as an immunogen. In some embodiments, hybridoma cells producing murine monoclonal anti-human GPC3 antibodies can be generated using methods well known in the art. Antibodies derived from rodents (such as mice) may induce undesirable antibody immunogenicity when used as therapeutic agents *in vivo,* leading to an immune response in humans against the therapeutic antibody upon repeated use. Such immune responses can result in at least a loss of therapeutic efficacy and, in severe cases, potentially lethal allergic reactions. One method to reduce the immunogenicity of rodent antibodies includes the production of chimeric antibodies, wherein a mouse variable region is fused with a human constant region (Liu et al., (1987) Proc. Natl. Acad. Sci. USA 84:3439-43). However, the retention of the intact rodent variable region in chimeric antibodies may still cause harmful immunogenicity in patients. Transplantation of complementarity-determining region (CDR) loops of the rodent variable domain to a human framework (i.e., humanization) has been used to further minimize rodent sequences (Jones et al., (1986) Nature 321:522; Verhoeyen et al., (1988) Science 239:1534).

In some embodiments, the chimeric or humanized antibodies described in the present disclosure can be prepared based on the sequences of murine monoclonal hybridoma antibodies. DNA encoding the heavy and light chain immunoglobulins can be obtained from the target murine hybridoma and engineered using standard molecular biology techniques to include non-murine (e.g., human) immunoglobulin sequences.

In some embodiments, the chimeric GPC3 antibodies of the present disclosure can be prepared by effectively linking the variable regions of the immunoglobulin heavy and light chains derived from the hybridoma to human IgG constant regions using methods known in the art (see, for example, U.S. Patent No. 4,816,567 belonging to Cabilly et al.), resulting in chimeric heavy and light chains. In some embodiments, the constant region contained in the chimeric antibodies of the present disclosure can be selected from any human IgG isotype, such as IgG1, IgG2, IgG3, or IgG4, preferably IgG4.

In some embodiments, the chimeric GPC3 antibody of the present disclosure can be obtained by "mixing and matching" chimeric light chain and chimeric heavy chain expression plasmids to transfect expression cells. The GPC3 binding of such "mixed and matched" antibodies can be tested using the binding assay described above and other conventional binding assays (e.g., ELISA).

The humanized antibodies described in the present disclosure can be generated by inserting murine CDR regions into human germline framework regions using methods known in the art. See Winter et al., U.S. Patent No. 5,225,539, and Queen et al., U.S. Patent Nos. 5,530,101; 5,585,089; 5,693,762, and 6,180,370.

In some embodiments, amino acid modifications include deletions, insertions, or substitutions of amino acids. In some embodiments, the anti-GPC3 antibody or antigen-binding fragment thereof of the present disclosure include antibodies that have undergone mutations via amino acid deletions, insertions, or substitutions, but still retain at least about 90%, 91%, 92%, 93%, 94%, 95%, 96%, 97%, 98%, 99%, or 100% identity to the amino acid sequence of the above-described antibodies, particularly in the CDR regions depicted in the above sequences. In some embodiments, the antibody of the present disclosure has no more than 1, 2, 3, 4, or 5 amino acid mutations in the CDR regions when compared to the CDR regions depicted in the specific sequences, where the mutations consist of amino acid deletions, insertions, or substitutions.

In some embodiments, one or more amino acid modifications may be introduced into the Fc region of the antibodies provided herein to generate Fc region variants. Fc region variants may include human Fc region sequences (e.g., human IgG1, IgG2, IgG3, or IgG4 Fc regions) containing amino acid modifications (e.g., substitutions) at one or more amino acid positions.

In some embodiments, it may be desirable to generate cysteine-engineered antibodies, such as "thio-MAbs," in which one or more residues of the antibody are replaced with cysteine residues.

In some embodiments, the antibodies provided herein may be further modified to include other non-protein moieties that are known and readily available in the art. Suitable moieties for antibody derivatization include, but are not limited to, water-soluble polymers. Non-limiting examples of water-soluble polymers include, but are not limited to, polyethylene glycol (PEG), ethylene glycol/propylene glycol copolymers, carboxymethylcellulose, dextran, polyvinyl alcohol, polyvinylpyrrolidone, poly-1,3-dialkane, poly-1,3,6-trialkane, ethylene/maleic anhydride copolymers, polyamino acids (homopolymers or random copolymers), and glucan or poly(N-vinylpyrrolidone), polyethylene glycol, polypropylene glycol homopolymers, polypropylene oxide/ethylene oxide copolymers, polyoxyethylated polyols (e.g., glycerol), polyvinyl alcohol, and mixtures thereof.

### Drug Conjugate

The present disclosure provides an anti-GPC3 antibody-drug conjugate, an isomer thereof, a pharmaceutically acceptable salt thereof, or a mixture thereof, wherein the anti-GPC3 antibody-drug conjugate has a structure as shown in formula (I-1): 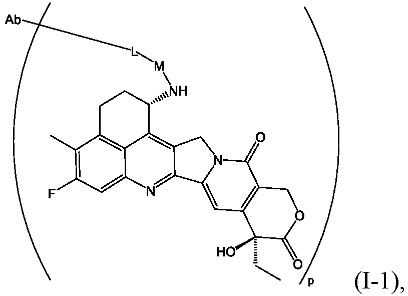
M is -L²-L³-X-L¹-;
L² is -O- or -S-;
L³ is -(C(R^{1a})(R^{1b}))ₘ- and m is 0, 1, 2, or 3, wherein when L³ comprises a methylene unit, 0 or 1 methylene unit of the L³ is replaced by -C(O)- or -C(S)-;
L¹ is -(C(R^{2a})(R^{2b}))ₙ- and n is 1, 2, or 3, wherein when L¹ may comprise a methylene unit, 0 or 1 methylene unit of the L¹ is replaced by -C(O)- or -C(S)-;
X is 3- to 6-membered saturated carbocyclyl, 3- to 6-membered saturated heterocyclyl, or a single bond, and the 3- to 6-membered saturated carbocyclyl and the 3- to 6-membered saturated heterocyclyl are optionally substituted by one or more R^{3a};
wherein R^{1a}, R^{1b}, R^{2a}, R^{2b}, and R^{3a} may each independently be hydrogen, halogen, or a C₁₋₆ aliphatic group optionally substituted by one or more R;
wherein each R may independently be hydrogen or halogen;
L is a linker unit;
p represents the average number of connections, and p is an integer or a decimal from 1 to 10, preferably an integer or a decimal from 3 to 8;
Ab is an anti-GPC3 antibody or an antigen-binding fragment thereof.

In some embodiments, the anti-GPC3 antibody or the antigen-binding fragment thereof described in the present disclosure comprises a heavy chain variable region and a light chain variable region; the heavy chain variable region comprises the amino acid sequences of HCDR1, HCDR2, and HCDR3 as shown in SEQ ID NO: 1, SEQ ID NO: 2, and SEQ ID NO: 3, respectively, and the light chain variable region comprises the amino acid sequences of LCDR1, LCDR2, and LCDR3 as shown in SEQ ID NO: 4, SEQ ID NO: 5, and SEQ ID NO: 6, respectively; preferably, the anti-GPC3 antibody or the antigen binding fragment as described in the present disclosure comprises: a heavy chain variable region having an amino acid sequence as shown in SEQ ID NO: 7 and a light chain variable region having an amino acid sequence as shown in SEQ ID NO: 8; more preferably, the anti-GPC3 antibody or the antigen binding fragment as described in the present disclosure comprises: a heavy chain having an amino acid sequence as shown in SEQ ID NO: 9 and a light chain having an amino acid sequence as shown in SEQ ID NO: 10.

In some embodiments, the present disclosure describes the anti-GPC3 antibody-drug conjugate, the isomer thereof, the pharmaceutically acceptable salt thereof, or the mixture thereof, wherein L³ is -(C(R^{1a})(R^{1b}))ₘ-, wherein m is 0, 1, 2, or 3; wherein R^{1a} and R^{1b} may each independently be hydrogen, halogen, or a C₁₋₆ aliphatic group optionally substituted by R, wherein each R may independently be hydrogen or halogen; preferably, L³ is -(C(R^{1a})(R^{1b}))ₘ-, wherein m is 0, 1, 2, or 3; wherein R^{1a} and R^{1b} may each independently be hydrogen, halogen, CH₃, or CH₂CH₃; preferably, L³ is a single bond, -CH₂-, -CH(CH₃), -C(CH₃)₂, -CH₂CH₂-, - CH(CH₃)CH₂-, or -C(CH₃)₂CH₂-.

In some embodiments, the present disclosure describes the anti-GPC3 antibody-drug conjugate, the isomer thereof, the pharmaceutically acceptable salt thereof, or the mixture thereof, wherein L¹ is -(C(R^{2a})(R^{2b}))ₙ-, and n is 1, 2, or 3, wherein when L¹ may comprise a methylene unit, 0 or 1 methylene unit of the L¹ is replaced by -C(O)- or -C(=S)-; wherein R^{2a} and R^{2b} may each independently be hydrogen, halogen, or a C₁₋₆ aliphatic group optionally substituted by R; wherein each R may independently be hydrogen or halogen; preferably, L¹ is -C(R^{2a})(R^{2b})-, -C(R^{2a})(R^{2b})C(O)-, -C(O)-; wherein R^{2a} and R^{2b} may each independently be hydrogen, halogen, CH₃, or CH₂CH₃; more preferably, L¹ is -CH₂-, -CH₂C(O)-, - CH(CH₃)C(O)-, or -C(O)-; further more preferably, L¹ is -CH₂C(O)-; still more preferably, L¹ is -C(O)-.

In some embodiments, the present disclosure describes the anti-GPC3 antibody-drug conjugate, the isomer thereof, the pharmaceutically acceptable salt thereof, or the mixture thereof, wherein X is 3- to 6-membered saturated carbocyclyl, 3- to 6-membered saturated heterocyclyl, or a single bond, and the 3- to 6-membered saturated carbocyclyl and 3- to 6-membered saturated heterocyclyl are optionally substituted by 1, 2, or 3 R^{3a}; wherein each R^{3a} may independently be hydrogen, halogen, or a C₁₋₆ aliphatic group optionally substituted by 1, 2, or 3 R, wherein each R may independently be hydrogen or halogen; preferably, X is 3- to 6-membered saturated carbocyclyl optionally substituted by 1, 2, or 3 R^{3a}, or a single bond; wherein each R^{3a} may independently be hydrogen, halogen, CH₃, or CH₂CH₃; more preferably, X is 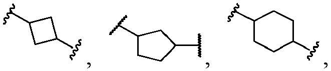 or a single bond.

In some embodiments, the present disclosure describes the anti-GPC3 antibody-drug conjugate, the isomer thereof, the pharmaceutically acceptable salt thereof, or the mixture thereof, wherein the -L³-X- is -(C(R^{1a})(R^{1b}))ₘ-, 3- to 6-membered saturated carbocyclyl, or 3-to 6-membered saturated heterocyclyl, wherein m is 1, 2, or 3, and the 3- to 6-membered saturated carbocyclyl and 3- to 6-membered saturated heterocyclyl are optionally substituted by 1, 2, or 3 R^{3a}; wherein R^{1a}, R^{1b}, and R^{3a} may each independently be hydrogen, halogen, or a C₁₋₆ aliphatic group optionally substituted by R, wherein each R may independently be hydrogen or halogen;
preferably, -L³-X- is -(C(R^{1a})(R^{1b}))ₘ- or 3- to 6-membered saturated carbocyclyl optionally substituted by 1, 2, or 3 R^{3a}; wherein m is 1, 2, or 3; wherein each R^{1a} is independently halogen or a C₁₋₆ aliphatic group optionally substituted by 1, 2, or 3 R; R^{1b} and R^{3a} are each independently hydrogen, halogen, or a C₁₋₆ aliphatic group optionally substituted by 1, 2, or 3 R; each R may independently be hydrogen or halogen;
more preferably, -L³-X- is

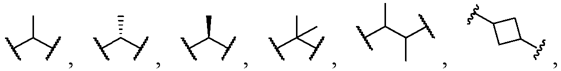

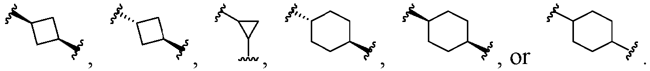

In some embodiments, the present disclosure describes the anti-GPC3 antibody-drug conjugate, the isomer thereof, the pharmaceutically acceptable salt thereof, or the mixture thereof, wherein
the M is -L²-L³-X-L¹-;
L² is -O-;
-L³-X- is 3- to 6-membered saturated carbocyclyl optionally substituted by 1, 2, or 3 R^{3a}; preferably, -L³-X- is 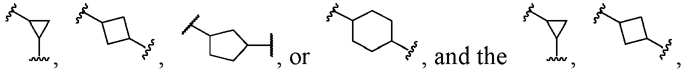 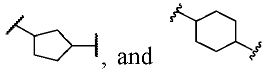 are optionally substituted by 1, 2, or 3 R^{3a}; preferably, -L³-X- is 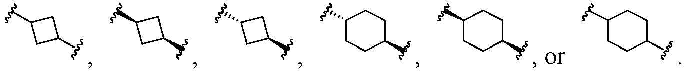
L¹ is -C(R^{2a})(R^{2b})-, -C(R^{2a})(R^{2b})C(O)-, or -C(O)-; preferably, L¹ is -C(O)-;
wherein R^{2a}, R^{2b}, and R^{3a} are each independently hydrogen, halogen, or a C₁₋₆ aliphatic group optionally substituted by R;
wherein each R may independently be hydrogen or halogen.

In some embodiments, the present disclosure describes the anti-GPC3 antibody-drug conjugate, the isomer thereof, the pharmaceutically acceptable salt thereof, or the mixture thereof, wherein
the M is -L²-L³-X-L¹-;
L² is -O- or -S-; preferably, L² is -O-; preferably, L² is -S-;
-L³-X- is -(C(R^{1a})(R^{1b}))ₘ-, wherein m is 1, 2, or 3; preferably, -L³-X- is 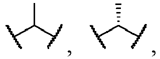 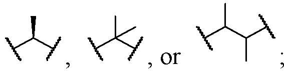 preferably, -L³-X- is 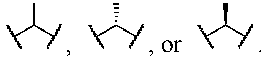
L¹ is -C(R^{2a})(R^{2b})-, -C(R^{2a})(R^{2b})C(O)-, or -C(O)-; preferably, L¹ is -CH₂C(O)-;
wherein each R^{1a} is independently halogen or a C₁₋₆ aliphatic group optionally substituted by 1, 2, or 3 R; R^{1b} and R^{3a} are each independently hydrogen, halogen, or a C₁₋₆ aliphatic group optionally substituted by 1, 2, or 3 R;
wherein each R may independently be hydrogen or halogen.

In some preferred examples, the present disclosure describes the anti-GPC3 antibody-drug conjugate, the isomer thereof, the pharmaceutically acceptable salt thereof, or the mixture thereof, wherein the -M- is:

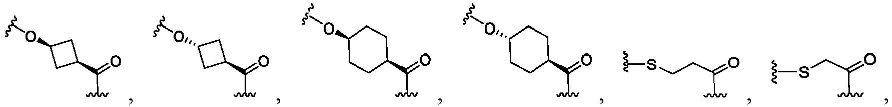

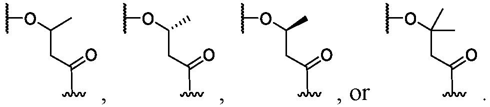

In some embodiments, the present disclosure describes the anti-GPC3 antibody-drug conjugate, the isomer thereof, the pharmaceutically acceptable salt thereof, or the mixture thereof, wherein the L is -Lₐ-L_{b}-L_{c}-,
the -Lₐ- is 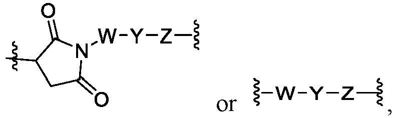
wherein W is -(C(R^{wa})(R^{wb}))_{wn}-, wherein wn is 1, 2, 3, or 6, and 0 or 1 methylene unit of W is each independently replaced by -Cyr-, -N(R^{wx})C(O)-, -C(O)N(R^{wx})-, or -C(O)-; preferably, W is -(C(R^{wa})(R^{wb}))₂- or -(C(R^{wa})(R^{wb}))₃-; preferably, W is -CH₂CH₂CH₂- or - CH₂CH₂-;
Y is -(OCH₂CH₂)_{yn}-O_{yp}-, wherein yn is 0, 4, or 8, yp is 0 or 1; preferably, Y is a single bond;
Z is -(C(R^{za})(R^{zb}))_{zn}, wherein zn is 1, 2, or 3, 1 methylene unit of Z is each independently replaced by -Cyr-, -N(R^{zx})C(O)-, -C(O)N(R^{zx})-, or -C(O)-, and -Cyr- is 3- to 10-membered saturated carbocyclylene, wherein the -Cyr- is unsubstituted or independently substituted by 1 to 3 substituents R^{cx}; preferably, Z is -(C(R^{wa})(R^{wb}))₂C(O)- or - (C(R^{wa})(R^{wb}))₃C(O)-; preferably, Z is -CH₂CH₂CH₂C(O)- or CH₂CH₂C(O)-;
wherein R^{wa}, R^{wb}, R^{za}, R^{zb}, R^{wx}, R^{zx}, and R^{cx} are each independently hydrogen, halogen, -OR^{r}, or a C₁₋₆ aliphatic group optionally substituted by R^{r};
wherein each R^{r} is independently hydrogen, halogen, or a C₁₋₆ aliphatic group;
preferably, the -Lₐ- is 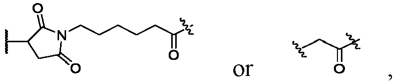 preferably 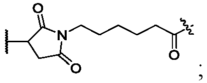
the -L_{b}- represents a peptide residue consisting of 2 to 4 amino acids, and the peptide residue of -L_{b}- is formed from amino acids selected from the group consisting of phenylalanine, glycine, alanine, valine, citrulline, and lysine;
preferably, the -L_{b}- is selected from the following group consisting of: 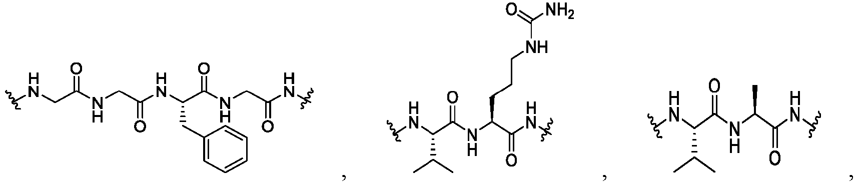 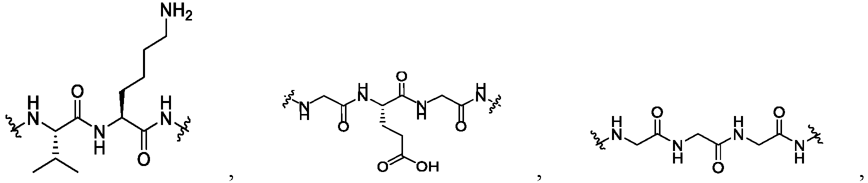 and 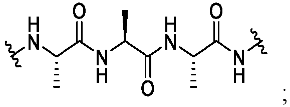 preferably, the -L_{b}- is 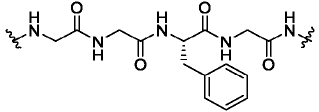 or 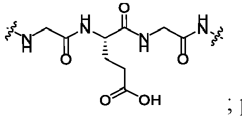 preferably, -L_{b}- is 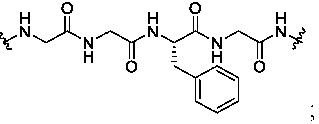
the -L_{c}- is 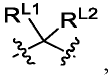 wherein R^{L1} and R^{L2} are each independently selected from the following group consisting of hydrogen, halogen, -OH, and a C₁₋₆ aliphatic group; preferably, the -L_{c}- is

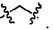

In some especially preferred examples, the present disclosure describes the anti-GPC3 antibody-drug conjugate, the isomer thereof, the pharmaceutically acceptable salt thereof, or the mixture thereof, wherein the L is:

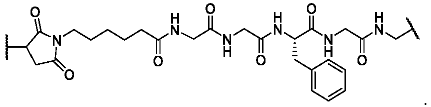

In some embodiments, the present disclosure describes the anti-GPC3 antibody-drug conjugate, the isomer thereof, the pharmaceutically acceptable salt thereof, or the mixture thereof, wherein the anti-GPC3 antibody-drug conjugate has a structure as shown in formula (II-1) or (II-2): 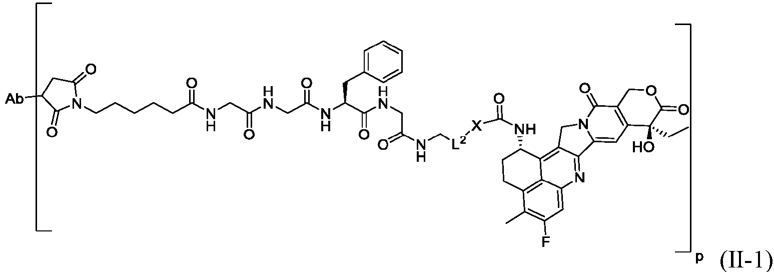 or 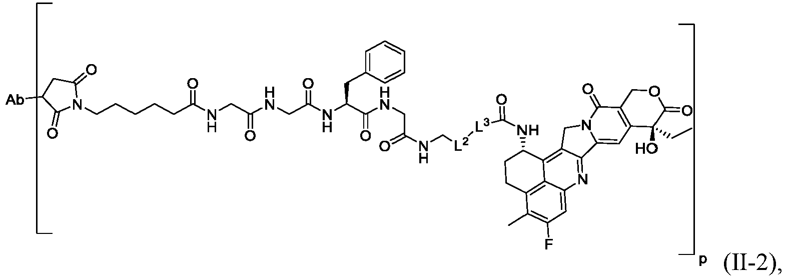 wherein
L² is -O- or -S-; preferably, L² is -O-; preferably, L² is -S-,
X is 3- to 6-membered saturated carbocyclyl optionally substituted by 1, 2, or 3 R^{3a}; preferably, X is 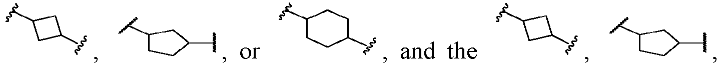 and 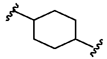 are optionally substituted by 1, 2, or 3 R^{3a}; preferably, X is 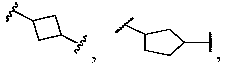 or 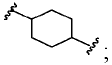
L³ is -C(R^{1a})(R^{1b})- or -C(R^{1a})(R^{1b})C(R^{1a})(R^{1b})-,
wherein each R^{1a} is independently halogen or a C₁₋₆ aliphatic group optionally substituted by 1, 2, or 3 R; R^{1b} and R^{3a} are each independently hydrogen, halogen, or a C₁₋₆ aliphatic group optionally substituted by 1, 2, or 3 R; each R may independently be hydrogen or halogen; preferably, L³ is -CH(CH₃)-, -C(CH₃)₂-, -CH(CH₃)CH₂-, or -C(CH₃)₂CH₂-;
p represents the average number of connections, and p is an integer or a decimal from 1 to 10, preferably an integer or a decimal from 3 to 8;
Ab is an anti-GPC3 antibody or an antigen-binding fragment thereof, which comprises a heavy chain variable region and a light chain variable region; the heavy chain variable region comprises the amino acid sequences of HCDR1, HCDR2, and HCDR3 as shown in SEQ ID NO: 1, SEQ ID NO: 2, and SEQ ID NO: 3, respectively, and the light chain variable region comprises the amino acid sequences of LCDR1, LCDR2, and LCDR3 as shown in SEQ ID NO: 4, SEQ ID NO: 5, and SEQ ID NO: 6, respectively; preferably, the anti-GPC3 antibody or the antigen binding fragment as described in the present disclosure comprises: a heavy chain variable region having an amino acid sequence as shown in SEQ ID NO: 7 and a light chain variable region having an amino acid sequence as shown in SEQ ID NO: 8; more preferably, the anti-GPC3 antibody or the antigen binding fragment as described in the present disclosure comprises: a heavy chain having an amino acid sequence as shown in SEQ ID NO: 9 and a light chain having an amino acid sequence as shown in SEQ ID NO: 10.

In some embodiments, the average number of connections p as described in the present disclosure may be an integer or a decimal from 2 to 8. For example, the average number of connections n may be an integer or a decimal from 3 to 8. For example, the average number of connections n may be an integer or a decimal from 1 to 2, 2 to 3, 3 to 4, 4 to 5, 5 to 6, 6 to 7, 7 to 8, 8 to 9, or 9 to 10.

### Pharmaceutical Composition and Pharmaceutical Preparation

In yet another aspect, the present disclosure provides a pharmaceutical composition comprising the anti-GPC3 antibody-drug conjugate, the isomer thereof, the pharmaceutically acceptable salt thereof, or the mixture thereof as described in any one of the present disclosure, and a pharmaceutically acceptable carrier or excipient.

It should be understood that the anti-GPC3 antibody-drug conjugate, the isomer thereof, the pharmaceutically acceptable salt thereof, or the mixture thereof, or the pharmaceutical composition thereof provided by the present disclosure can be integrated into preparations with suitable carriers, excipients, and other agents for combined administration, thereby offering improved transfer, delivery, tolerance, etc.

In the present disclosure, the term "pharmaceutical composition" refers to a preparation that allows the active ingredient contained therein to be present in a biologically active and effective form while excluding additional components that would have unacceptable toxicity to the subject to whom the preparation is administered.

Pharmaceutical preparations containing the anti-GPC3 antibody described herein can be prepared by mixing the anti-GPC3 antibody-drug conjugate of the present disclosure or a pharmaceutically acceptable salt thereof, with one or more optional pharmaceutical excipients (Remington's Pharmaceutical Sciences, 16th Edition, Osol, A. ed. (1980)), preferably in the form of aqueous solutions or lyophilized preparations.

The pharmaceutical composition or preparation of the present disclosure may also comprise one or more other active ingredients that are required for the treatment of a specific indication, preferably those active ingredients that do not adversely affect each other's complementary activity. In some embodiments, the other active ingredients are chemotherapeutic agents, immune checkpoint inhibitors, growth inhibitors, antibiotics, or various known antitumor or anticancer agents, which are appropriately present in combination in amounts effective for the intended use. In some embodiments, the pharmaceutical composition of the present disclosure further comprises a composition encoding a polynucleotide of an anti-GPC3 antibody.

In yet another aspect, the present disclosure provides a pharmaceutical combination, comprising the antibody-drug conjugate or the pharmaceutically acceptable salt thereof as described in any one of the present disclosure, or the pharmaceutical composition as described in any one of the present disclosure, and one or more additional therapeutic agents.

In yet another aspect, the present disclosure provides a kit, comprising the antibody-drug conjugate, the isomer thereof, the pharmaceutically acceptable salt thereof, or the mixture thereof as described in any one of the present disclosure, or the pharmaceutical composition as described herein, preferably further comprising a drug delivery device.

### Medical Use

In yet another aspect, the present disclosure provides a use of the antibody-drug conjugate, the isomer thereof, the pharmaceutically acceptable salt thereof, or the mixture thereof as described in any one of the present disclosure, or the pharmaceutical composition as described in any one of the present disclosure in the manufacture of a medicament for treating and/or preventing a GPC3-mediated disease or disorder; preferably, the disease or disorder is cancer.

In yet another aspect, the present disclosure provides the antibody-drug conjugate, the isomer thereof, the pharmaceutically acceptable salt thereof, or the mixture as described in any one of the present disclosure, or the pharmaceutical composition as described in any one of the present disclosure for use in treating and/or preventing a GPC3-mediated disease or condition; preferably, the disease or disorder is cancer.

In yet another aspect, the present disclosure provides a method for treating and/or preventing a GPC3-mediated disease or disorder, comprising administering to a subject in need thereof the antibody-drug conjugate, the isomer thereof, the pharmaceutically acceptable salt thereof, or the mixture thereof as described in any one of the present disclosure, or the pharmaceutical composition as described in any one of the present disclosure; preferably, the disease or disorder is cancer.

In some embodiments, the cancer is selected from breast cancer, ovarian cancer, prostate cancer, pancreatic cancer, renal cancer, lung cancer, liver cancer, gastric cancer, colon cancer, bladder cancer, esophageal cancer, cervical cancer, gallbladder cancer, glioblastoma, and melanoma.

In some embodiments, the routes of administration of the present disclosure include, but are not limited to, oral, intravenous, subcutaneous, intramuscular, intra-arterial, intra-articular (e.g., in arthritic joints), inhalation, aerosol delivery, or intratumoral administration.

In some embodiments, the present disclosure provides one or more therapies (e.g., treatment modalities and/or other therapeutic agents) co-administered in a therapeutically effective amount to a subject. In some embodiments, the therapy includes surgical treatment and/or radiation therapy.

In some embodiments, the method or use provided by the present disclosure further includes administering one or more therapies (e.g., treatment modalities and/or other therapeutic agents) to a subject. The antibody-drug conjugate of the present disclosure or a pharmaceutically acceptable salt thereof can be used alone or in combination with other therapeutic agents in therapy. For example, they may be co-administered with at least one additional therapeutic agent.

The drug conjugate described in the present disclosure may exhibit inhibitory activity against *in vitro* proliferation of tumor cells. The inhibitory activity may be demonstrated by a decrease in the proliferative capacity of tumor cells by 1% or more, 2% or more, 4% or more, 5% or more, 8% or more, 10% or more, 15% or more, 18% or more, 20% or more, 25% or more, 40% or more, 50% or more, 60% or more, 70% or more, 80% or more, 90% or more, or 95% or more when the drug conjugate of the present disclosure is added to the tumor cell culture medium compared to the addition of a negative control or a control drug. For example, the inhibitory activity may be represented by the IC₅₀ value (nM) for the tumor cells, which may be 10,000 or less, 5,000 or less, 4,000 or less, 3,000 or less, 2,000 or less, 1,000 or less, 500 or less, 400 or less, 300 or less, 200 or less, 150 or less, 120 or less, 110 or less, 100 or less, 99 or less, 98 or less, 97 or less, 95 or less, 90 or less, 80 or less, 75 or less, 70 or less, 65 or less, 62 or less, 60 or less, 50 or less, 40 or less, 30 or less, 25 or less, 23 or less, 22 or less, 20 or less, 19 or less, 18 or less, 18.5 or less, 17 or less, 15 or less, 12 or less, 10 or less, 9 or less, 8.5 or less, 7 or less, 6.7 or less, 6 or less, 5.9 or less, 5.5 or less, 5.0 or less, 4.8 or less, 4.5 or less, 4.4 or less, 4 or less, 3.5 or less, 3 or less, 2.5 or less, 2 or less, 1.5 or less, 1.0 or less, 0.5 or less, 0.3 or less, 0.29 or less, 0.25 or less, 0.21 or less, 0.20 or less, 0.18 or less, 0.17 or less, 0.15 or less, 0.12 or less, 0.10 or less, 0.09 or less, 0.08 or less, 0.07 or less, 0.06 or less, 0.05 or less, 0.04 or less, 0.03 or less, 0.02 or less, or 0.01 or less. For example, the tumor cells may include, but are not limited to, solid tumor cells, such as gastric cancer cells, breast cancer cells, or liver cancer cells.

The drug conjugate described in the present disclosure may exhibit targeted inhibitory properties. The targeted inhibitory property may be demonstrated by a decrease in the proliferative capacity of tumor cells with high expression of a specific target by 1% or more, 2% or more, 4% or more, 5% or more, 8% or more, 10% or more, 15% or more, 18% or more, 20% or more, 25% or more, 40% or more, 50% or more, 60% or more, 70% or more, 80% or more, 90% or more, or 95% or more when the drug conjugate of the present disclosure is added to the culture medium of these tumor cells with high expression of a specific target, compared to the addition of a negative control or a control drug. For example, the targeted inhibitory property may be represented by the IC₅₀ value (nM) for the tumor cells with high expression of a specific target, which may be 10,000 or less, 5,000 or less, 4,000 or less, 3,000 or less, 2,000 or less, 1,000 or less, 500 or less, 400 or less, 300 or less, 200 or less, 185 or less, 150 or less, 120 or less, 110 or less, 100 or less, 99 or less, 98 or less, 97 or less, 95 or less, 91 or less, 80 or less, 74 or less, 70 or less, 65 or less, 62 or less, 60 or less, 50 or less, 40 or less, 30 or less, 25 or less, 23 or less, 22 or less, 20 or less, 19 or less, 18 or less, 18.5 or less, 17 or less, 15 or less, 12 or less, 10 or less, 9 or less, 8.5 or less, 7 or less, 6.7 or less, 6 or less, 5.9 or less, 5.5 or less, 5.0 or less, 4.8 or less, 4.5 or less, 4.4 or less, 4 or less, 3.5 or less, 3 or less, 2.5 or less, 2 or less, 1.5 or less, 1.0 or less, 0.5 or less, 0.3 or less, 0.29 or less, 0.25 or less, 0.21 or less, 0.20 or less, 0.18 or less, 0.17 or less, 0.15 or less, 0.12 or less, 0.10 or less, 0.09 or less, 0.08 or less, 0.07 or less, 0.06 or less, 0.05 or less, 0.04 or less, 0.03 or less, 0.02 or less, or 0.01 or less. For example, the tumor cells with high expression of a specific target may include, but are not limited to, solid tumor cells, such as gastric cancer cells, breast cancer cells, or liver cancer cells.

The drug conjugate described in the present disclosure may exhibit plasma stability. The plasma stability can be demonstrated by the release rate of the cytotoxic drug from the drug conjugate when the drug conjugate of the present disclosure is added to plasma. The release rate is 50% or less, 40% or less, 30% or less, 20% or less, 10% or less, 7% or less, 5% or less, 4% or less, 3% or less, 2% or less, 1.9% or less, 1.8% or less, 1.7% or less, 1.6% or less, 1.5% or less, 1.4% or less, 1.3% or less, 1.2% or less, 1.1% or less, 1.0% or less, 0.9% or less, 0.8% or less, 0.7% or less, 0.6% or less, 0.5% or less, 0.4% or less, 0.3% or less, 0.2% or less, or 0.1% or less after 1 day, 3 days, 5 days, 7 days, 14 days, 20 days, or 30 days.

The drug conjugate of the present disclosure may exhibit *in vivo* tumor inhibition effects. The tumor inhibition effect may be demonstrated by a decrease in tumor volume in an animal by 1% or more, 2% or more, 4% or more, 5% or more, 8% or more, 10% or more, 15% or more, 18% or more, 20% or more, 25% or more, 40% or more, 50% or more, 55% or more, 60% or more, 70% or more, 73% or more, 75% or more, 80% or more, 90% or more, or 95% or more after 1 day, 3 days, 5 days, 7 days, 14 days, 20 days, 21 days, or 30 days when the drug conjugate of the present disclosure is administered to the animal, compared to the administration of a negative control or a control drug; alternatively, the tumor inhibition effect may be demonstrated by a decrease in tumor volume in an animal by 1.1-fold or more, 1.3-fold or more, 1.5-fold or more, 2-fold or more, 3-fold or more, 5-fold or more, 10-fold or more, 20-fold or more, 22-fold or more, 30-fold or more, 50-fold or more, 100-fold or more, 500-fold or more, 1,000-fold or more, or 1,500-fold or more after 1 day, 3 days, 5 days, 7 days, 14 days, 20 days, 21 days, or 30 days when the drug conjugate of the present disclosure is administered to the animal, compared to the administration of a negative control or a control drug. The animals may include, but are not limited to, mammals. For example, the animals may include, but are not limited to, cats, dogs, horses, pigs, cows, sheep, rabbits, mice, rats, monkeys, or humans. The administration may include, but are not limited to, oral administration, intravenous injection, intravenous infusion, intraperitoneal injection, or topical administration.

The drug conjugate of the present disclosure may exhibit a bystander killing effect (bystander effect). The bystander killing effect may be that the drug conjugate of the present disclosure has no obvious inhibitory effect on the cell proliferation of tumor cells with low expression of a specific target, but in the co-culture of tumor cells with low expression of a specific target and tumor cells with high expression of a specific target, the drug conjugate of the present disclosure can simultaneously inhibit the cell proliferation of tumor cells with low expression of a specific target and tumor cells with high expression of a specific target. For example, in the co-culture of tumor cells with low expression of a specific target and tumor cells with high expression of a specific target, the inhibitory activity may be that the IC₅₀ value (nM) for the tumor cells with low expression of the specific target may be 10,000 or less, 5,000 or less, 4,000 or less, 3,000 or less, 2,000 or less, 1,000 or less, 500 or less, 400 or less, 300 or less, 200 or less, 185 or less, 150 or less, 120 or less, 110 or less, 100 or less, 99 or less, 98 or less, 97 or less, 95 or less, 91 or less, 80 or less, 74 or less, 70 or less, 65 or less, 62 or less, 60 or less, 50 or less, 40 or less, 30 or less, 25 or less, 23 or less, 22 or less, 20 or less, 19 or less, 18 or less, 18.5 or less, 17 or less, 15 or less, 12 or less, 10 or less, 9 or less, 8.5 or less, 7 or less, 6.7 or less, 6 or less, 5.9 or less, 5.5 or less, 5.0 or less, 4.8 or less, 4.5 or less, 4.4 or less, 4 or less, 3.5 or less, 3 or less, 2.5 or less, 2 or less, 1.5 or less, 1.0 or less, 0.5 or less, 0.3 or less, 0.29 or less, 0.25 or less, 0.21 or less, 0.20 or less, 0.18 or less, 0.17 or less, 0.15 or less, 0.12 or less, 0.10 or less, 0.09 or less, 0.08 or less, 0.07 or less, 0.06 or less, 0.05 or less, 0.04 or less, 0.03 or less, 0.02 or less, or 0.01 or less. The expression of the specific target in tumor cells with low expression, compared to tumor cells with high expression, may be reduced by 1% or more, 2% or more, 4% or more, 5% or more, 8% or more, 10% or more, 15% or more, 18% or more, 20% or more, 25% or more, 40% or more, 50% or more, 60% or more, 70% or more, 80% or more, 90% or more, or 95% or more. For example, the tumor cells with high expression of a specific target may include, but are not limited to, solid tumor cells; for example, the tumor cells with high expression of a specific target include, but are not limited to, gastric cancer cells or breast cancer cells; for example, the tumor cells with high expression of a specific target may include, but are not limited to, HCC1569 cells or MDA-MB-453 cells. For example, the tumor cells with low expression of a specific target may include, but are not limited to, solid tumor cells.

The drug conjugate described in the present disclosure may exhibit anti-transporter transport capability. The anti-transporter transport capability may be demonstrated by a decrease in the efflux ratio of the drug conjugate of the present disclosure by 1% or more, 2% or more, 4% or more, 5% or more, 8% or more, 10% or more, 15% or more, 18% or more, 20% or more, 25% or more, 40% or more, 50% or more, 60% or more, 70% or more, 80% or more, 90% or more, or 95% or more compared to the standard of the transport substrate. For example, the efflux ratio can be tested using methods commonly employed by those skilled in the art or as described in the examples of the present disclosure.

The drug conjugate of the present disclosure may exhibit *in vivo* tumor-targeting capability. The *in vivo* tumor-targeting capability may refer to the administration of the drug conjugate labeled with a signaling substance to an animal, where the distribution of the labeled drug conjugate in the tumor tissue of the animal is increased by 1% or more, 2% or more, 4% or more, 5% or more, 8% or more, 10% or more, 15% or more, 18% or more, 20% or more, 25% or more, 40% or more, 50% or more, 60% or more, 70% or more, 80% or more, 90% or more, or 95% or more compared to other tissues and organs; or the distribution may increase by 1.1-fold or more, 1.3-fold or more, 1.5-fold or more, 2-fold or more, 3-fold or more, 5-fold or more, 10-fold or more, 20-fold or more, 22-fold or more, 30-fold or more, 50-fold or more, 100-fold or more, 500-fold or more, 1,000-fold or more, or 1,500-fold or more. The signaling substance may be a radioactive substance, for example, the signaling substance includes, but is not limited to, ¹²⁵I. The animals may include, but are not limited to, mammals. For example, the animals may include, but are not limited to, cats, dogs, horses, pigs, cows, sheep, rabbits, mice, rats, monkeys, or humans. The administration may include, but are not limited to, oral administration, intravenous injection, intravenous infusion, intraperitoneal injection, or topical administration. The tissues or organs may include, but are not limited to, heart, liver, spleen, lung, kidney, brain, or bone marrow.

The drug conjugate described in the present disclosure may exhibit good *in vivo* safety. The *in vivo* safety may be that after the drug conjugate of the present disclosure is administered to an animal, the release rate of free payloads in the body of the animal is 50% or less, 40% or less, 30% or less, 20% or less, 10% or less, 7% or less, 5% or less, 4% or less, 3% or less, 2% or less, 1.9% or less, 1.8% or less, 1.7% or less, 1.6% or less, 1.5% or less, 1.4% or less, 1.3% or less, 1.2% or less, 1.1% or less, 1.0% or less, 0.9% or less, 0.8% or less, 0.7% or less, 0.6% or less, 0.5% or less, 0.4% or less, 0.3% or less, 0.2% or less, or 0.1% or less. For example, the *in vivo* safety may be that the administration concentration of the drug conjugate of the present disclosure may be 0.5 mg/kg or more, 1 mg/kg or more, 2 mg/kg or more, 3 mg/kg or more, 4 mg/kg or more, 5 mg/kg or more, 10 mg/kg or more, 20 mg/kg or more, 30 mg/kg or more, 50 mg/kg or more, 70 mg/kg or more, 100 mg/kg or more, 200 mg/kg or more, 500 mg/kg or more, or 1,000 mg/kg or more, without causing toxic manifestations in animals. For example, the animals may include, but are not limited to, cats, dogs, horses, pigs, cows, sheep, rabbits, mice, rats, monkeys, or humans. The administration may include, but are not limited to, oral administration, intravenous injection, intravenous infusion, intraperitoneal injection, or topical administration.

The positive advancements of the present disclosure lies in:
In the anti-GPC3 antibody-drug conjugate of the present disclosure, the anti-GPC3 antibody or the antigen-binding fragment thereof is connected to a biologically active cytotoxic drug through a linker unit. After the antibody-drug conjugate is transferred to tumor cells, the linker is cleaved, releasing the cytotoxic drug H-M-D (such as the small molecule compounds prepared in preparation examples 1 to 9). The cytotoxic drugs of the present disclosure exhibit significantly enhanced proliferation inhibitory activity against NCI-N87 cells, JIMT-1 cells, Colo205 cells, and MDA-MB-231 cells, and can exert excellent antitumor effects.

The anti-GPC3 antibody-drug conjugate of the present disclosure shows significantly enhanced proliferation inhibitory activity against GPC3-positive expression cells Huh7 and HepG2. The anti-GPC3 antibody-drug conjugate of the present disclosure shows significantly enhanced antitumor activity in HepG2 tumor-bearing mice and Huh7 tumor-bearing mice.

Therefore, the present disclosure has promising application prospects in diseases (such as cancer) with positive expression of GPC3.

### BRIEF DESCRIPTION OF THE DRAWINGS

FIG. 1: *In vivo* antitumor efficacy of the antibody-drug conjugate against human liver cancer cells in HepG2 tumor-bearing mice.
FIG. 2: *In vivo* antitumor efficacy of the antibody-drug conjugate against human liver cancer cells in Huh7 tumor-bearing mice.

### DETAILED DESCRIPTION OF THE PREFERRED EMBODIMENT

The following specific examples illustrate the embodiments of the present disclosure. Those skilled in the art can readily understand other advantages and effects of the present disclosure from the disclosure provided in this specification.

### Sample detection

### 1. ADC DAR value analysis method - HIC-HPLC (hydrophobic interaction chromatography)

High performance liquid chromatograph: Waters e2965 high performance liquid chromatography system.

Chromatographic column: Thermo MAbPac^{™} HIC-Butyl (4.6 × 100 mm, 5µm).

Mobile phase A: 1.5 M (NH₄)₂SO₄ + 50 mM K₂HPO₄ (pH 7.0).

Mobile phase B: 50 mM K₂HPO₄ (pH 7.0)/isopropanol (75:25 V/V).

Elution was carried out according to the following elution procedure:

| Time | Mobile phase B |
|---|---|
| 0-2 min | 0%-10% |
| 2-22 min | 10%-65% |
| 22-24 min | 65%-100% |
| 24-26 min | 100%-0% |
| 26-30 min | 0% |

Detection conditions: The flow rate of the mobile phase was set to 1 mL/min, the detection wavelength was 280 nm, and the column temperature was 30°C.

### 2. SEC purity analysis - SEC-HPLC (size exclusion chromatography)

High performance liquid chromatograph: 1260 Agilent liquid chromatograph.

Chromatographic column: Waters Xbridge BEH200 SEC (7.8 × 300 mm, 3.5 µm)

Mobile phase: 50 mM NaH₂PO₄ + 200 mM arginine (pH 6.80) + 10% isopropanol

| Time | Mobile phase |
|---|---|
| 0-30 min | 100% |

Detection conditions: The flow rate of the mobile phase was set to 0.5 mL/min, the detection wavelength was 280 nm, and the column temperature was 30°C.

The present disclosure includes all combinations of the specific embodiments described herein. The full scope of further embodiments and their applicability will become apparent from the detailed description provided below. However, it should be understood that, while the detailed description and specific examples indicate preferred embodiments of the present disclosure, these descriptions and examples are provided for illustrative purposes only, as various changes and modifications within the spirit and scope of the present disclosure will become apparent to those skilled in the art upon reading the detailed description. For all purposes, including citations, all publications, patents, and patent applications referenced herein are hereby incorporated by reference in their entireties.

### Examples

The following examples are provided to demonstrate and further explain some of the preferred embodiments and aspects of the present disclosure and should not be construed as limiting its scope.

### Example 1: Anti-GPC3 antibody

The antibodies of the present disclosure were prepared with reference to WO2006006693, wherein the DB1001 variable region amino acid sequences of the anti-GPC3 antibody are as follows. The CDR regions are determined according to the IMGT numbering rules.
DB1001 heavy chain variable region: 
QVQLVQSGAEVKKPGASVKVSCKASGYTFTDYEMHWVRQAPGQGLEWM
GALDPKTGDTAYSQKFKGRVTLTADKSTSTAYMELSSLTSEDTAVYYCTRFYSYTY
WGQGTLVTVSS (SEQ ID NO: 7)
HCDR1: **DYEMH** (SEQ ID NO: 1)
HCDR2: **ALDPKTGDTAYSQKFKG** (SEQ ID NO: 2)
HCDR3: **FYSYTY** (SEQ ID NO: 3)
DB1001 light chain variable region: 
DVVMTQSPLSLPVTPGEPASISCRSSQSLVHSNRNTYLHWYLQKPGQSPQLL
IYKVSNRFSGVPDRFSGSGSGTDFTLKISRVEAEDVGVYYCSQNTHVPPTFGQGTKL
EIK (SEQ ID NO: 8)
LCDR1: **RSSQSLVHSNRNTYLH** (SEQ ID NO: 4)
LCDR2: **KVSNRFS** (SEQ ID NO: 5)
LCDR3: **SQNTHVPPT** (SEQ ID NO: 6)

Based on the above sequences, primers were designed for PCR to construct VH/VK gene fragments and obtain the variable regions. The antibody variable regions were then homologously recombined with the constant region gene fragments to construct the complete DB1001 antibody sequence. After transfection into CHO cells, antibody DB1001 was obtained using conventional expression and purification methods.
DB1001 heavy chain (IgG1) amino acid sequence 
QVQLVQSGAEVKKPGASVKVSCKASGYTFTDYEMHWVRQAPGQGLEWMG
ALDPKTGDTAYSQKFKGRVTLTADKSTSTAYMELSSLTSEDTAVYYCTRFYSYTYWG
QGTLVTVSSASTKGPSVFPLAPSSKSTSGGTAALGCLVKDYFPEPVTVSWNSGALTSG
VHTFPAVLQSSGLYSLSSVVTVPSSSLGTQTYICNVNHKPSNTKVDKKVEPKSCDKTH
TCPPCPAPELLGGPSVFLFPPKPKDTLMISRTPEVTCVVVDVSHEDPEVKFNWYVDGV
 
EVHNAKTKPREEQYNSTYRVVSVLTVLHQDWLNGKEYKCKVSNKALPAPIEKTISKA
KGQPREPQVYTLPPSRDELTKNQVSLTCLVKGFYPSDIAVEWESNGQPENNYKTTPPV
LDSDGSFFLYSKLTVDKSRWQQGNVFSCSVMHEALHNHYTQKSLSLSPGK (SEQ ID
NO: 9)
DB1001 light chain (Kappa) amino acid sequence

DVVMTQSPLSLPVTPGEPASISCRSSQSLVHSNRNTYLHWYLQKPGQSPQLLI
YKVSNRFSGVPDRFSGSGSGTDFTLKISRVEAEDVGVYYCSQNTHVPPTFGQGTKLEI
KRTVAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC (SEQ ID
NO: 10)

### Example 2: Preparation of anti-GPC3 antibody-drug conjugate (ADC)

### 2.1 Preparation of payload

### Preparation Example 1

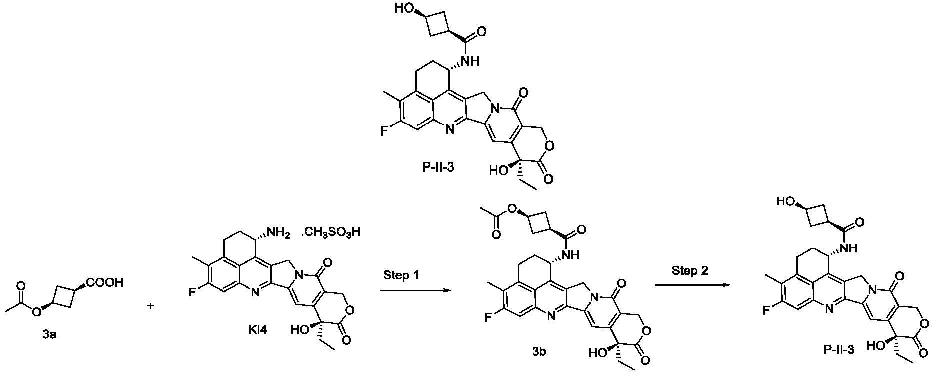

### Step 1:

Under nitrogen atmosphere at 0°C, DIEA(500 mg, 3.87 mmol) was added to a solution of KI4 (900 mg, 1.69 mmol), HATU (691 mg, 1.88 mmol), and 3a (320 mg, 2.00 mmol) in DMF (18 mL). The reaction mixture was stirred at 25°C for 3 hours. TLC (EA) indicated that the reaction was complete. The reaction mixture was added dropwise to 320 mL of deionized water, and filtered to obtain a gray solid (850 mg) with a yield of 87%.

### Step 2:

NaHCO₃ (42 mg, 0.50 mmol) solid was added to a solution of 3b (100 mg, 0.174 mmol) in MeOH/DCM (1/1, 3 mL), and the reaction mixture was stirred at 25°C for 3 hours. TLC (EA) indicated that the reaction was complete. The reaction mixture was filtered and subjected to rotary evaporation to dryness at low temperature. The residue was slurried with aq. HCl (0.5 M, 10 mL), filtered, purified using prep-HPLC (0.1% TFA), and then lyophilized to obtain 15 mg of gray solid with a yield of 16%.

MS m/z (ESI): 534 [M+1].

H-NMR (400 MHz, DMSO-D): 8.45(d, 1H), 7.81 (d, 1H), 7.32 (s, 1H), 6.52 (m, 1H), 5.58-5.56 (m, 1 H), 5.44 (s, 2H), 5.14 (dd, 2 H), 3.96 (m, 1H), 3.48 (m, 1H), 3.19 (m, 2H), 2.53-2.28 (m, 3H), 2.48 (s, 3H), 2.20-2.00(m, 4H), 1.95-1.80 (m, 2H), 0.89 (t, 3H).

### Preparation Example 2

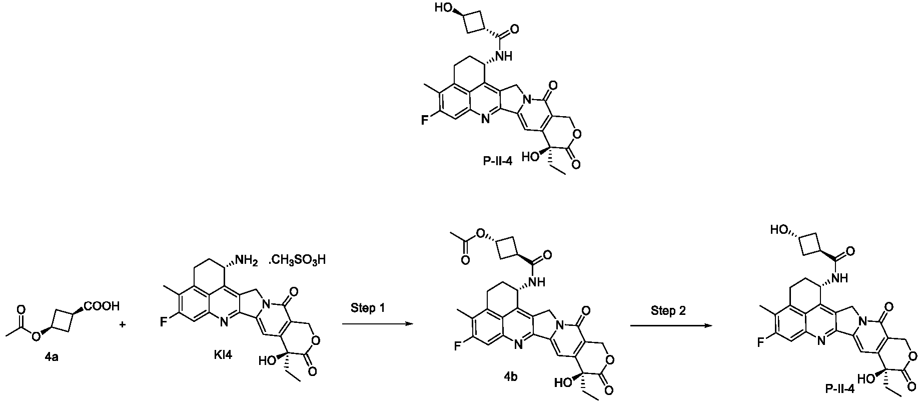

### Step 1:

Under nitrogen atmosphere at 0°C, DIEA(486 mg, 3.76 mmol) was added to a solution of KI4 (1.00 g, 1.88 mmol), HATU (691 mg, 1.88 mmol), and 4a (310 mg, 1.88 mmol) in DMF (18 mL). The reaction mixture was stirred at 25°C for 3 hours. TLC (EA) indicated that the reaction was complete. The reaction mixture was added dropwise to 320 mL of deionized water, and filtered to obtain a gray solid (910 mg) with a yield of 84%.

### Step 2:

NaHCO₃ (42 mg, 0.50 mmol) solid was added to a solution of 4b (100 mg, 1.58 mmol) in MeOH/DCM (1/1, 3 mL), and the reaction mixture was stirred at 25°C for 3 hours. TLC (EA) indicated that the reaction was complete. The reaction mixture was filtered and subjected to rotary evaporation to dryness at low temperatures. The residue was slurried with aq. HCl (0.5 M, 10 mL), filtered, purified using prep-HPLC (0.1% TFA), and then lyophilized to obtain 13 mg of yellow solid with a yield of 14%.

MS m/z (ESI): 534 [M+1].

H-NMR (400 MHz, DMSO-D): 8.39 (d, 1H), 7.77 (d, 1H), 7.29 (s, 1H), 6.52 (m, 1H), 5.58-5.54 (m, 1 H), 5.41 (s, 2H), 5.18-5.06 (m, 3H), 4.39-4.33 (m, 1H), 3.19-3.07 (m, 2H), 2.97-2.82 (m, 1H), 2.49-2.36 (m, 2H), 2.38 (s, 3H), 2.20-1.96 (m, 4H), 1.93-1.79 (m, 2H), 0.87 (t, 3H).

### Preparation Example 3

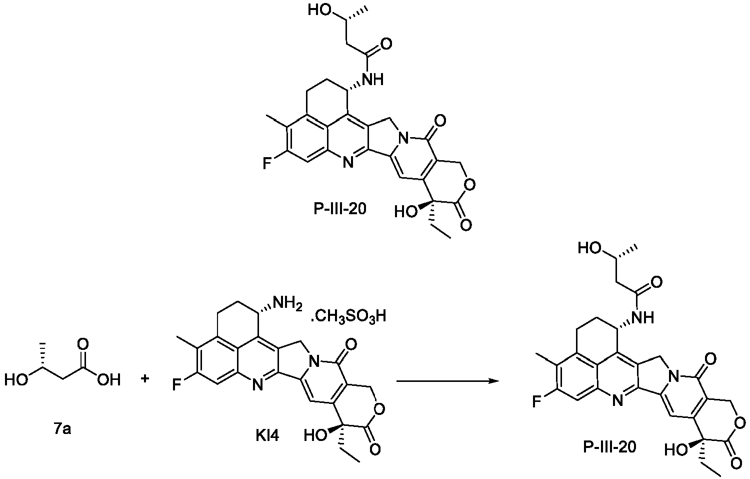

Under nitrogen atmosphere, DIEA (61 mg, 0.47 mmol) was added dropwise to a solution of KI4 (100 mg, 0.188 mmol), HATU (86 mg, 0.23 mmol), and 7a (22 mg, 0.21 mmol) in DMF (2 mL). After the addition was complete, the reaction was carried out at 25°C for 2.5 hours. LCMS indicated that the reaction was complete. The reaction mixture was then added to 20 mL of water to precipitate a solid, and filtered to obtain 13 mg with a yield of 11%.

MS m/z (ESI): 522 [M+1].

H-NMR (400 MHz, DMSO-D): 8.44 (d, 1H), 7.80 (d, 1H), 7.32(s, 1H), 6.54 (m, 1H), 5.60-5.50 (m, 1 H), 5.44 (s, 2H), 5.22 (dd, 2H), 4.10-4.00 (m, 1H), 3.30-3.17 (m, 2H), 2.41 (s, 3H), 2.38-2.10 (m, 4H), 1.96-1.80 (m, 2H), 1.11 (d, 3H), 0.89 (t, 3H).

### Preparation Example 4

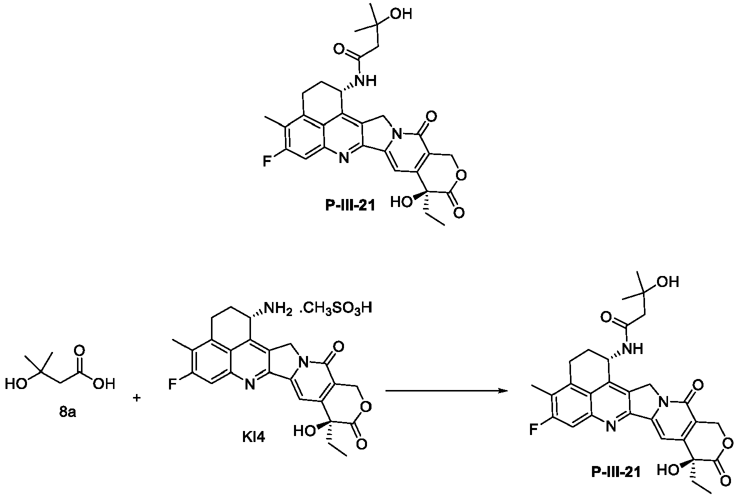

Under nitrogen atmosphere, a solution of DIEA (61 mg, 0.47 mmol) in DMF (1 mL) was added dropwise to a solution of KI4 (100 mg, 0.188 mmol), HATU (86 mg, 0.23 mmol), and 8a (24 mg, 0.21 mmol) in DMF (1 mL). After the addition was complete, the reaction was carried out at 25°C for 3 hours. TLC (EA) indicated that the reaction was complete. The reaction mixture was purified to obtain 17 mg of yellow solid with a yield of 17%.

MS m/z (ESI): 536 [M+1].

H-NMR (400 MHz, DMSO-D): 8.42 (d, 1H), 7.79 (d, 1H), 7.30(s, 1H), 6.53 (m, 1H), 5.59-5.55 (m, 1 H), 5.42 (s, 2H), 5.22 (dd, 2H), 4.69 (s, 1H), 3.20-3.11 (m, 2H), 2.39 (sc, 3H), 2.29 (s, 2H), 2.20-2.08 (m, 2H), 1.19 (d, 6H), 0.87 (t, 3H).

### Preparation Example 5

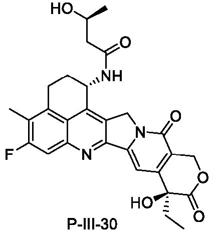

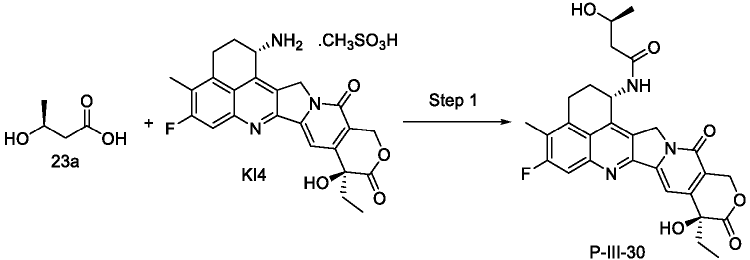

### Step 1

Under nitrogen atmosphere, DIEA (60.6 mg, 0.47 mmol) was added dropwise to a solution of KI4 (100 mg, 0.19 mmol), HATU (85.7 mg, 0.23 mmol), and 23a (21.5 mg, 0.21 mmol) in DMF (2 mL). After the addition was complete, the reaction was carried out at 0°C for 2 hours. LCMS indicated that the reaction was complete. The reaction mixture was then added dropwise to 20 mL of water with stirring. After the solid was precipitated, the mixture was filtered to obtain 60.2 mg of gray solid P-III-30 with a yield of 61%. MS-ESI: m/z 522.2 [M+H]+.

1H NMR (400 MHz, DMSO-d6) δ 8.42 (d, J = 8.7 Hz, 1H), 7.79 (d, J = 11.0 Hz, 1H), 7.30 (s, 1H), 6.53 (s, 1H), 5.62 - 5.53 (m, 1H), 5.42 (s, 2H), 5.30 - 5.16 (m, 2H), 4.63 (d, J = 4.6 Hz, 1H), 4.09 - 3.99 (m, 1H), 3.22 - 3.11 (m, 2H), 2.40 (s, 3H), 2.28 (dd, J = 13.7, 7.2 Hz, 1H), 2.22 - 2.08 (m, 3H), 1.94 - 1.78 (m, 2H), 1.08 (d, J = 6.1 Hz, 3H), 0.87 (t, J = 7.3 Hz, 3H).

### Preparation Example 6

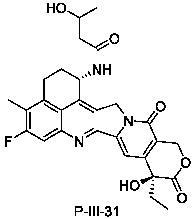

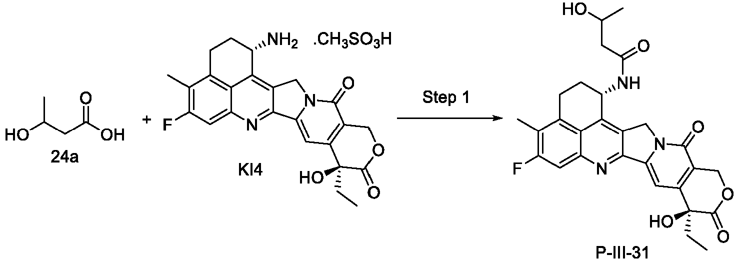

### Step 1

24a (19.6 mg, 0.188 mmol), KI4 (100 mg, 0.188 mmol), and DIEA (60.7 mg, 0.47 mmol) were added to a 100 mL three-neck flask, followed by the addition of DMF (2 mL) for dissolution. The flask was purged with N₂ three times, and then HATU (85.9 mg, 0.226 mmol) was added thereto with stirring at 0°C. The reaction was carried out at 0°C for 2 hours. TLC (DCM: MeOH = 10:1) indicated that the reaction was complete. The reaction mixture was added to water (20 mL), resulting in the precipitation of a gray solid. The solid was lyophilized and then purified using preparative TLC (EA: MeOH = 10:1) to obtain 54.5 mg of pale yellow solid P-III-31 with a yield of 56%. MS-ESI: m/z 522.5 [M+H]+.

1H NMR (400 MHz, DMSO-d6) δ 8.44 (dd, J = 8.7, 2.9 Hz, 1H), 7.78 (dd, J = 10.9, 2.6 Hz, 1H), 7.30 (s, 1H), 6.53 (s, 1H), 5.60 - 5.51 (m, 1H), 5.42 (s, 2H), 5.21 (dd, J = 10.9, 3.4 Hz, 2H), 4.66 (dd, J = 13.1, 4.7 Hz, 1H), 4.10 - 3.98 (m, 1H), 3.21 - 3.10 (m, 2H), 2.39 (s, 3H), 2.34 - 2.05 (m, 4H), 1.93 - 1.77 (m, 2H), 1.08 (dd, J = 6.2, 1.6 Hz, 3H), 0.87 (t, J = 7.3 Hz, 3H).

### Preparation Example 7

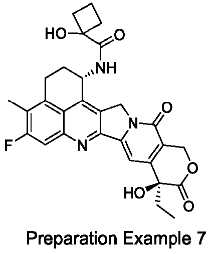

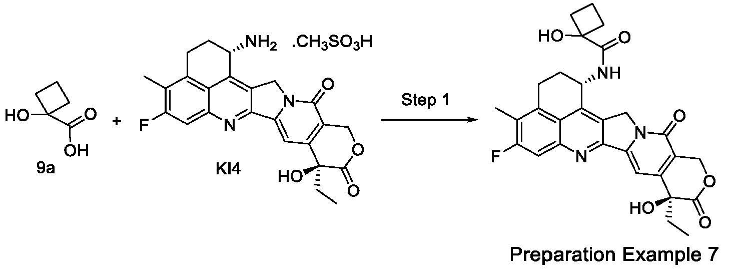

### Step 1

KI4 (50 mg, 0.094 mmol), 25a (11 mg, 0.094 mmol), and HATU (39 mg, 0.103 mmol) were added to DMF (3 mL). After purging with nitrogen, DIEA (30 mg, 0.235 mmol) was added thereto, and the reaction was carried out at 25°C for 1.5 hours. LCMS indicated that the reaction was complete. The reaction mixture was added dropwise to water (50 mL) with stirring. After the addition was complete, the mixture was allowed to stand for 5 minutes, and then filtered. The filter cake was lyophilized to obtain 20 mg of gray solid 25 with a yield of 40%.

MS-ESI: m/z 534.3 [M+H]+.

1H NMR (400 MHz, DMSO-d6) δ 8.26 (d, J = 9.1 Hz, 1H), 7.72 (d, J = 10.9 Hz, 1H), 7.28 (s, 1H), 6.51 (s, 1H), 6.12 (s, 1H), 5.59 - 5.50 (m, 1H), 5.40 (s, 2H), 5.15 (d, J = 18.8 Hz, 1H), 4.98 (d, J = 19.0 Hz, 1H), 3.28 - 3.16 (m, 1H), 3.15 - 3.02 (m, 1H), 2.74 - 2.52 (m, 2H), 2.36 (s, 3H), 2.24 - 2.04 (m, 4H), 1.92 - 1.79 (m, 4H), 0.86 (t, J = 7.3 Hz, 3H).

### Preparation Example 8

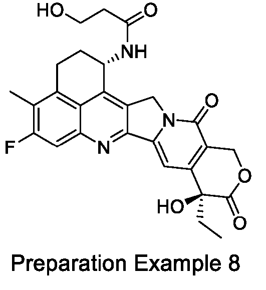

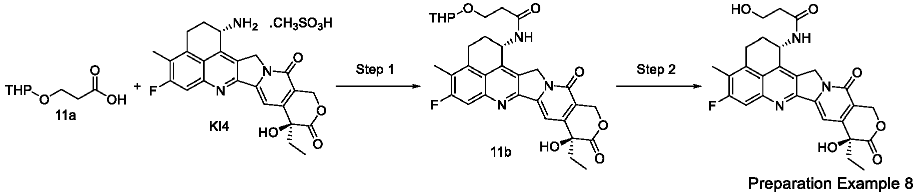

### Step 1

KI4 (100 mg, 0.188 mmol), 11a (33 mg, 0.188 mmol), and HATU (77 mg, 0.203 mmol) were added to DMF (3 mL). After purging with nitrogen, DIEA (73 mg, 0.564 mmol) was added thereto, and the reaction was carried out at 25°C for 1.5 hours. LCMS indicated that the reaction was complete. The reaction mixture was added dropwise to water (50 mL) with stirring. After the addition was complete, the mixture was allowed to stand for 5 minutes, and then filtered. The filter cake was lyophilized to obtain 80 mg of gray solid 11b with a yield of 72%. MS-ESI: m/z 592.4 [M+H]+.

### Step 2

27b (80 mg, 0.135 mmol) was dissolved in DCM (2 mL). The reaction system was purged with N₂ and cooled to 0°C. TFA (0.5 mL) was then added thereto, and the reaction was carried out at 0°C for 1.5 hours. LCMS indicated that the reaction was complete. The reaction mixture was subjected to rotary evaporation to dryness at low temperatures, and the residue was purified by PTLC. The purified product was added with acetonitrile and water and lyophilized to obtain 25 mg of white solid with a yield of 36%.

MS-ESI: m/z 508.3 [M+H]+.

1H NMR (400 MHz, DMSO-d6) δ 8.44 (d, J = 8.6 Hz, 1H), 7.79 (d, J = 11.0 Hz, 1H), 7.30 (s, 1H), 6.53 (s, 1H), 5.60 - 5.53 (m, 1H), 5.42 (s, 2H), 5.28 - 5.15 (m, 2H), 4.59 (t, J = 5.1 Hz, 1H), 3.72 - 3.63 (m, 2H), 3.21 - 3.12 (m, 2H), 2.40 (s, 3H), 2.32 (t, J = 6.4 Hz, 2H), 2.21 - 2.06 (m, 2H), 1.92 - 1.79 (m, 2H), 0.87 (t, J = 7.3 Hz, 3H).

### Preparation Example 9: DXd

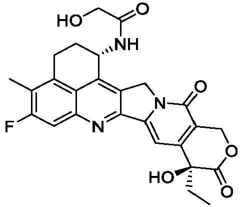

DXd was synthesized following the method provided in Example 75 on page 183 of the specification of patent "CN104755494A".

### Reference Example 1

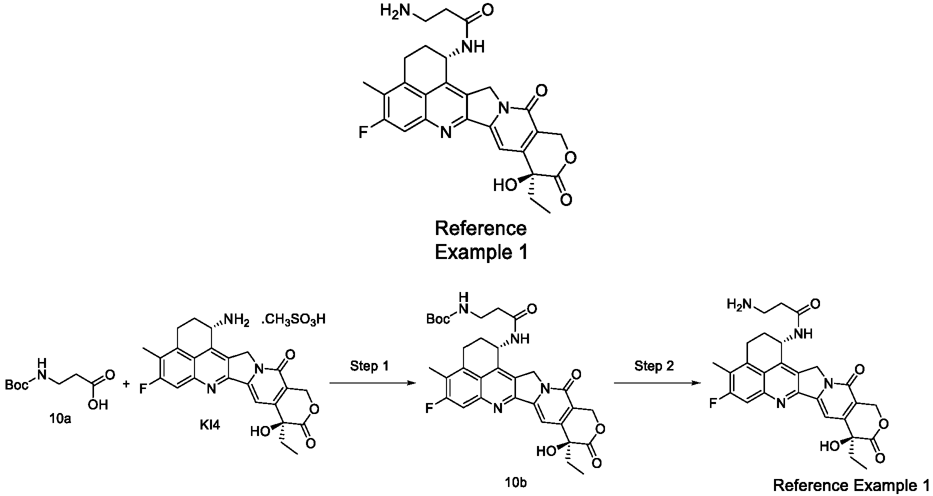

### Step 1

KI4 (50 mg, 0.094 mmol), 26a (18 mg, 0.094 mmol), and HATU (39 mg, 0.103 mmol) were added to DMF (3 mL). After purging with nitrogen, DIEA (48 mg, 0.376 mmol) was added thereto, and the reaction was carried out at 25°C for 1.5 hours. LCMS indicated that the reaction was complete. The reaction mixture was added dropwise to water (50 mL) with stirring. After the addition was complete, the mixture was allowed to stand for 5 minutes, and then filtered. The filter cake was lyophilized to obtain 30 mg of gray solid 26b with a yield of 52%. MS-ESI: m/z 607.4 [M+H]+.

### Step 2

26b (30 mg, 0.049 mmol) was dissolved in DCM (2 mL). The reaction system was purged with N₂ and cooled to 0°C. TFA (0.5 mL) was then added thereto, and the reaction was carried out at 0°C for 1.5 hours. LCMS indicated that the reaction was complete. The reaction mixture was subjected to rotary evaporation to dryness at low temperature, and the residue was added with DCM and subjected to rotary evaporation to dryness at low temperature. Then the residue was added with acetonitrile and water and lyophilized to obtain 20 mg of yellow solid with a yield of 80%.

MS-ESI: m/z 507.1 [M+H]+.

1H NMR (400 MHz, DMSO-d6) δ 8.67 (d, J = 8.6 Hz, 1H), 7.86 - 7.66 (m, 4H), 7.32 (s, 1H), 6.56 (brs, 1H), 5.63 - 5.54 (m, 1H), 5.43 (s, 2H), 5.29 (d, J = 18.9 Hz, 1H), 5.22 (d, J = 18.9 Hz, 1H), 3.23 - 3.15 (m, 2H), 3.13 - 3.03 (m, 2H), 2.57 - 2.51 (m, 2H), 2.43 - 2.38 (m, 3H), 2.27 - 2.08 (m, 2H), 1.94 - 1.78 (m, 2H), 0.87 (t, J = 7.3 Hz, 3H).

### 2.2. Preparation of linker-payload

### Linker-payload X1

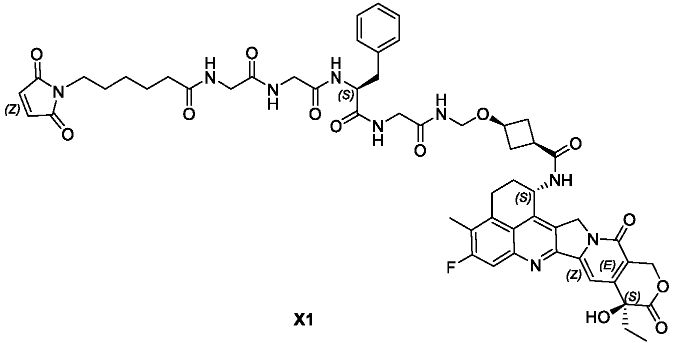

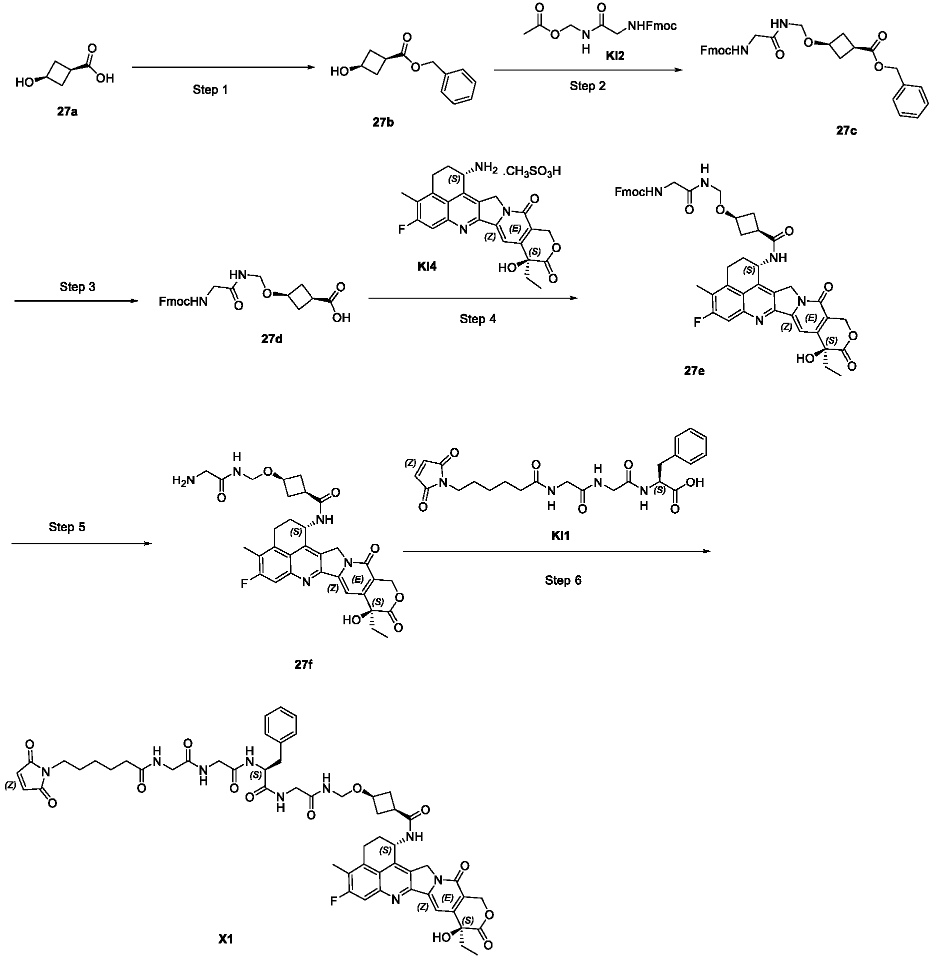

### Step 1

Under nitrogen atmosphere, benzyl bromide (11.0 g, 64.6 mmol) was added dropwise to a solution of 27a (5.00 g, 43.0 mmol) and NaHCO₃ (10.9 g, 129 mmol) in DMF (50 mL). The reaction was carried out at 25°C for 17 hours. TLC (PE/EA = 2/1) indicated that the reaction was complete. The reaction mixture was added to 500 mL of water and extracted twice with EA (250 mL). The phases were separated, and the organic phase was washed with saturated sodium chloride aqueous solution (500 mL), dried over anhydrous Na₂SO₄, and concentrated. The residue was purified by column chromatography (PE: EA = 3:2) to obtain 5.1 g of colorless liquid with a yield of 57.1%.

### Step 2

Under nitrogen atmosphere, a solution of 27b (4.50 g, 21.8 mmol) in THF (10 mL) was added dropwise to a solution of KI2 (4.00 g, 10.9 mmol) and TsOH (800 mg, 4.65 mmol) in THF (30 mL) at 0°C. The reaction was carried out at 25°C for 2 hours. TLC (PE/EA = 1/2) indicated that the reaction was complete. The reaction mixture was added to 200 mL of water and extracted twice with EA (200 mL). The phases were separated, and the organic phase was dried over anhydrous Na₂SO₄, and concentrated. The residue was purified by column chromatography (PE/EA = 3/2) to obtain 1.56 g of white solid with a yield of 26%.

### Step 3

Under hydrogen atmosphere at 0°C, Pd/C (80 mg) was added to a mixed solution of 27c (800 mg, 1.55 mmol) in EtOH (8 mL) and EA (8 mL). The reaction mixture was stirred at 0°C for 2.5 hours. LCMS indicated that the reaction was complete. The reaction mixture was filtered through diatomite, and the filter cake was washed with EA (200 mL). The mixture was concentrated, and the residue was dissolved in THF (20 mL) and then subjected to rotary evaporation to dryness to obtain 600 mg of white solid with a yield of 91%.

### Step 4

Under nitrogen atmosphere at 0°C, DIEA (152 mg, 1.18 mmol) was added to a solution of 27d (220 mg, 0.515 mmol), HY-13631A (250 mg, 0.47 mmol), and HATU (214 mg, 0.56 mmol) in DMF (6 mL). The reaction was carried out at 0°C for 2 hours. LCMS indicated that the reaction was complete. The reaction mixture was added to 150 mL of citric acid aqueous solution (pH = 4), and filtered. The filter cake was washed with 175 mL of water and filtered. After drying under vacuum using an oil pump, 260 mg of brown solid was obtained with a yield of 66%.

### Step 5

Under nitrogen atmosphere at 0°C, diethylamine (8 mL) was added dropwise to a solution of 27e (260 mg, 0.309 mmol) in DCM (30 mL). The reaction was carried out at 0°C for 3 hours. LCMS indicated that the reaction was complete. The reaction mixture was added to petroleum ether solution (600 mL) at 0°C, resulting in the precipitation of a solid. The mixture was allowed to stand until the solid settled at the bottom of the flask. The solution was decanted, and the residue was dried under vacuum using an oil pump to obtain 90 mg of brown solid with a yield of 47.1%.

### Step 6

Under nitrogen atmosphere at 0°C, HATU (74 mg, 0.19 mmol) was added to a solution of 27f (90 mg, 0.13 mmol), KI-1 (92 mg, 0.19 mmol), and DIEA (50 mg, 0.39 mmol) in DMF (2.5 mL). The reaction was carried out at 0°C for 2 hours. LCMS indicated that the reaction was nearly complete. At 0°C, the reaction mixture was added to 30 mL of citric acid aqueous solution (pH = 4), resulting in the precipitation of a flocculent solid. The mixture was filtered, and the filter cake was purified using preparative TLC (DCM/MeOH = 10/1) to obtain 9.2 mg of pale yellow solid X1 with a yield of 6%.

MS m/z (ESI): 1074 [M+1].

H-NMR (400 MHz, MeOD): 7.65 (d, 1H), 7.62 (s, 1H), 7.30-7.21(m, 5H), 6.79 (s, 2H), 5.69-5.65 (m, 1 H), 5.57 (d, 1H), 5.43-5.10 (m, 3H), 4.70 (d, 2H), 4.48-4.39 (m, 2H), 4.10-4.05 (m, 1H), 4.01-3.75 (m, 5H),3.46 (t, 2H), 3.22-3.15 (m, 2H), 3.07-3.00 (m, 1H), 2.75 (m, 1H), 2.62 (m, 1H), 2.45 (s, 3H), 2.37-2.20 (m, 6H), 2.10-2.02 (m, 2H), 2.00-1.92 (m, 2H) 1.68-1.57 (m, 6H), 1.01 (t, 3H).

### Linker-payload X2

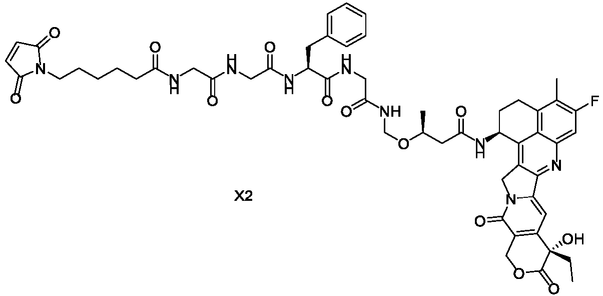

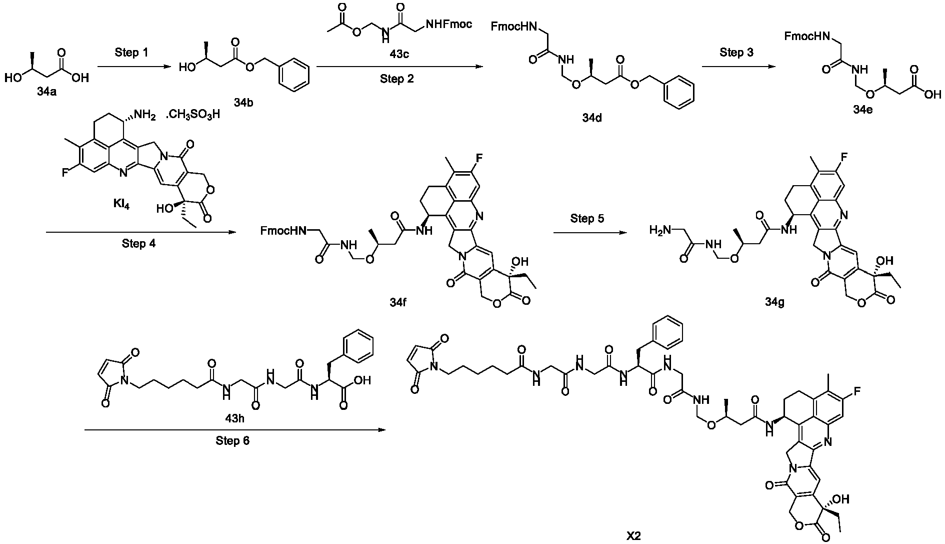

### Step 1

34a (5 g, 48.0 mmol) and K₂CO₃ (19.9 g, 144.0 mmol) were dissolved in DMF (20 mL). Benzyl bromide (12.3 g, 72.0 mmol) was added dropwise thereto, and the reaction was carried out at 25°C for 17 hours. TLC (PE/EA= 3/1) indicated that the reaction was complete. The reaction mixture was added to water (200 mL), and the mixture was extracted with EA (250 mL). The phases were separated. The organic phase was washed with saturated NaCl, dried over anhydrous Na₂SO₄, and concentrated. The residue was purified by column chromatography (PE: EA = 2/1) to obtain 8.7 g of colorless liquid 34b with a yield of 93%. MS-ESI: m/z 195.1 [M+H]+.

### Step 2

34c (7.3 g, 19.8 mmol) and TsOH (1.46 g, 8.5 mmol) were dissolved in THF (20 mL). The mixture was cooled to 0°C under nitrogen atmosphere, and a solution of 43b (7.7 g, 39.6 mmol) in THF (10 mL) was added dropwise thereto. After the addition was complete, the reaction was carried out at 0°C for 2 hours. TLC (PE/EA = 2/1) indicated that most of the starting material had reacted. The reaction mixture was poured into 100 mL of water, and extracted with DCM (100 mL). The phases were separated. The organic phase was washed with saturated NaCl, dried over anhydrous Na₂SO₄, and purified by column chromatography (PE/EA = 1/1) to obtain 3.9 g of colorless viscous liquid 34d with a yield of 39%. MS-ESI: m/z 503.3 [M+H]+.

### Step 3

Under hydrogen atmosphere at 0°C, Pd/C (1 g, 10 wt.%) was added to a mixed solution of 34d (1.9 g, 3.78 mmol) in EtOH (100 mL) and EA (100 mL). The reaction was carried out at 0°C for 3 hours. TLC (PE/EA = 2/1) indicated that the reaction was complete. The reaction mixture was filtered through diatomite, and the filter cake was washed with EA/EtOH (1:1, 100 mL × 3). The filtrate was concentrated, dissolved in THF (50 mL × 3), and subjected to rotary evaporation to dryness. This process was repeated three times to obtain 1 g of gray solid 34e with a yield of 64%. MS-ESI: m/z 435.2 [M+Na]+.

### Step 4

Under nitrogen atmosphere at 0°C, DIEA (303 mg, 2.35 mmol) was added dropwise to a solution of 34e (426 mg, 1.03 mmol), KI4 (500 mg, 0.94 mmol), and HATU (429 mg, 1.13 mmol) in DMF (20 mL). After the addition was complete, the reaction was carried out at 0°C for 2 hours. LCMS indicated that the reaction was complete. The reaction mixture was added dropwise to 300 mL of water, stirred, allowed to stand for 5 minutes, and filtered. The filter cake was dissolved in DCM/MeOH (10:1, 100 mL), dried, and subjected to rotary evaporation to dryness. The residue was blended with adsorbent for sample preparation, and purified by column chromatography (EA: MeOH = 30:1) to obtain 600 mg of yellow solid 34f with a yield of 77%. MS-ESI: m/z 830.3 [M+H]+.

### Step 5

Under nitrogen atmosphere at 0°C, diethylamine (5 mL) was added dropwise to a solution of 34f (150 mg, 0.18 mmol) in DCM (5 mL). The reaction was carried out at 0°C for 2 hours. LCMS indicated that the reaction was complete. The reaction mixture was added with petroleum ether solution (100 mL × 6), resulting in the precipitation of a solid. The mixture was allowed to stand until the solid settled, and the solution was decanted. The residue was then dried under vacuum using an oil pump to obtain 120 mg of white powder 34g. LCMS indicated the product content was 70% with a yield of 76%. MS-ESI: m/z 608.3 [M+H]+.

### Step 6

Under nitrogen atmosphere at 0°C, a solution of HATU (45 mg, 0.118 mmol) in DMF (1 mL) was added to a solution of 34g (60 mg, 0.099 mmol), 43h (51 mg, 0.108 mmol), and DIEA (32 mg, 0.25 mmol) in DMF (1 mL). The reaction was carried out at 0°C for 2 hours. LCMS indicated that the reaction was complete. The reaction mixture was directly purified by reverse-phase column chromatography using an eluent ((MeCN/MeOH = 1/1): H₂O = 60%: 40%) to obtain 14.8 mg of yellow solid X2 with a yield of 14%.

MS-ESI: m/z 1062.4 [M+H]+.

1H NMR (400 MHz, Methanol-d4) δ 7.69 - 7.61 (m, 2H), 7.22 - 7.16 (m, 2H), 7.16 - 7.09 (m, 3H), 6.76 (s, 2H), 5.70 - 5.64 (m, 1H), 5.60 (d, J = 16.4 Hz, 1H), 5.40 - 5.31 (m, 2H), 5.26 (d, J = 19.0 Hz, 1H), 4.65 - 4.50 (m, 7H), 4.25 - 4.16 (m, 1H), 3.87 (d, J = 16.7 Hz, 1H), 3.83 - 3.76 (m, 3H), 3.72 (d, J = 17.0 Hz, 2H), 3.44 (t, J = 7.1 Hz, 2H), 3.25 - 3.17 (m, 2H), 3.10 - 3.02 (m, 1H), 2.92 - 2.83 (m, 1H), 2.45 - 2.39 (m, 5H), 2.32 - 2.20 (m, 5H), 1.97 - 1.89 (m, 2H), 1.63 - 1.50 (m, 4H), 1.34 - 1.20 (m, 6H), 0.99 (t, J = 7.3 Hz, 3H).

### Linker-payload X3

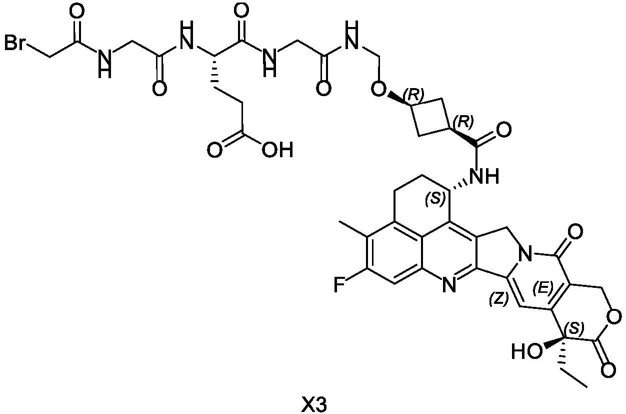

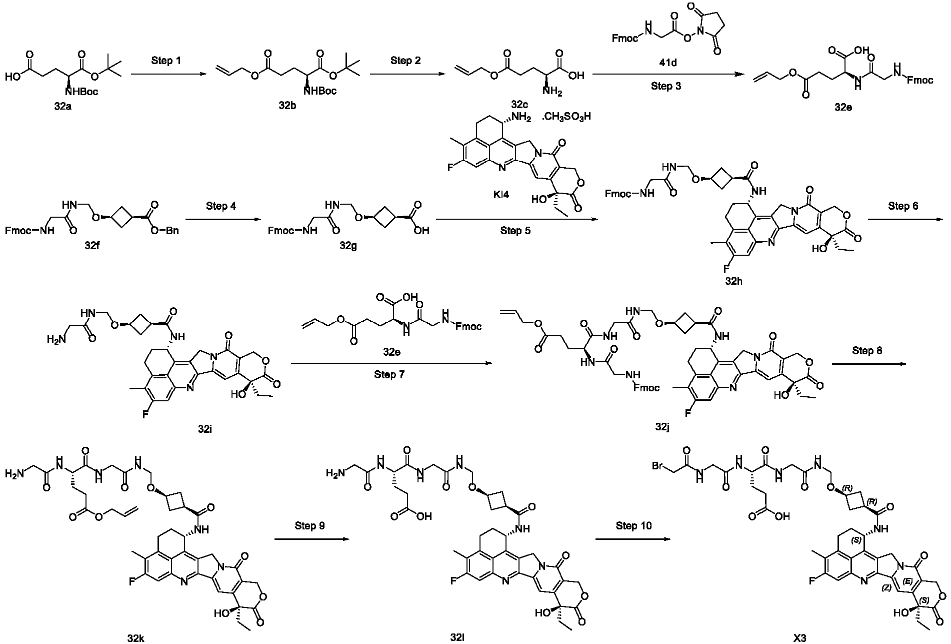

### Step 1

Bromopropene (960 mg, 7.92 mmol) was added to a solution of 32a (2.00 g, 6.6 mmol) and K₂CO₃ (1.82 g, 13.2 mmol) in MeCN (20 mL), and the reaction was carried out at 20°C for 5 hours. TLC (PE/EA = 1/2) indicated that the reaction was complete. The reaction mixture was poured into 100 mL of water, and the pH was adjusted to 5. The mixture was extracted three times with EA (100 mL), dried over anhydrous sodium sulfate, and subjected to rotary evaporation to dryness. The residue was purified by column chromatography (PE/EA = 2/1) to obtain 1.83 g of white solid 32b with a yield of 81%.

### Step 2

TFA (10 mL) was added to a solution of 32b (1.38 g, 4.02 mmol) in DCM (10 mL), and the reaction was carried out at 25°C for 17 hours. TLC (PE/EA = 1/3) indicated that the reaction was complete. The reaction mixture was subjected to rotary evaporation to dryness to obtain 0.91 g of yellow viscous material 32c (yield not calculated).

### Step 3

41d (1.92 g, 4.87 mmol) was added to a mixture of 32c (910 mg, 4.87 mmol) and NaHCO₃ (613 mg, 7.3 mmol) in DME/H₂O (20 mL/10 mL), and the reaction was carried out at 25°C for 3 hours. TLC (DCM/MeOH = 1/1) indicated that the reaction was complete. The reaction mixture was poured into 100 mL of water, and the pH was adjusted to 5 with aq. HCl (1 N). The mixture was extracted twice with EA (150 mL), dried over anhydrous sodium sulfate, and subjected to rotary evaporation to dryness. The residue was purified by column chromatography (DCM/MeOH = 20/1) to obtain 1.53 g of white solid 32e with a yield of 67%. MS-ESI: m/z 467.4 [M+H]+.

### Step 4

Pd/C (600 mg) was added to a solution of 32f (3 g, 5.83 mmol) in MeOH (50 mL), and the reaction mixture was stirred under a hydrogen balloon at 25°C for 5 hours. TLC (EA) indicated that the reaction was complete. The reaction mixture was filtered and subjected to rotary evaporation to dryness to obtain 1.9 g of white solid 32g with a yield of 77%.

### Step 5

HATU (707 mg, 1.86 mmol) was added to a solution of 32g (789 mg, 1.86 mmol), KI4 (900 mg, 1.69 mmol), and triethylamine (342 mg, 3.38 mmol) in DMF (10 mL). The reaction mixture was stirred at 0°C for 3.5 hours. TLC (EA) indicated that the reaction was complete. The reaction mixture was poured into H₂O (80 mL) and extracted twice with EA (100 mL). The organic phase was dried over anhydrous sodium sulfate, subjected to rotary evaporation to dryness, and purified by column chromatography (EA) to obtain 1.186 g of white solid 32h with a yield of 83%. MS-ESI: m/z 842.3 [M+H]+.

### Step 6

A solution of 32h (1.186 g, 1.41 mmol) in DCM/diethylamine (20 mL, 20/1) was stirred at 25°C for 17 hours. TLC (DCM/MeOH = 10/1) indicated that the reaction was complete. The reaction mixture was poured into petroleum ether (200 mL) and filtered to obtain 768 mg of white solid 32i with a yield of 88%. MS-ESI: m/z 620.3 [M+H]+.

### Step 7

HATU (414 mg, 1.09 mmol) was added to a solution of 32i (676 mg, 1.09 mmol), 32e (508 mg, 1.09 mmol), and DIEA (423 mg, 3.27 mmol) in DMF (10 mL). The reaction mixture was stirred at 20°C for 17 hours. TLC (PE/EA= 1/5) indicated that the reaction was complete. The reaction mixture was poured into water (30 mL) and filtered. The filter cake was purified by column chromatography (DCM/MeOH = 50/1) to obtain 511 mg of white solid 32j with a yield of 44%. MS-ESI: m/z 1068.3 [M+H]+.

### Step 8

A solution of 32j (482 mg, 0.451 mmol) in diethylamine/DCM (10 mL, 1/5) was stirred at 10°C for 17 hours. TLC (EA) indicated that the reaction was complete. The reaction mixture was poured into PE (300 mL), and filtered to obtain 301 mg of white solid 32k (yield not calculated).

### Step 9

Morpholine (93 mg, 1.07 mmol) was added to a solution of 32k (301 mg, 0.356 mmol) and Pd(PPh₃)₄ (82 mg, 0.071 mmol) in THF (5 mL), and the reaction mixture was stirred at 25°C for 5 hours. LCMS indicated that the reaction was complete. The reaction mixture was purified to obtain 108 mg of white solid 32l with a yield of 38%. MS-ESI: m/z 806.3 [M+H]+.

### Step 10

Bromoacetyl bromide (27 mg, 0.134 mmol) was added to a solution of 32l (108 mg, 0.134 mmol) and triethylamine (41 mg, 0.402 mmol) in THF (2 mL) and DMF (2 mL). The reaction mixture was stirred at 0°C for 1 hour. TLC (DCM/MeOH = 10/1) indicated that the reaction was complete. The reaction mixture was directly purified to obtain 15 mg of white solid X3 with a yield of 12%.

MS-ESI: m/z 926.3 [M+H]+.

1H NMR (400 MHz, DMSO-d6) δ 12.11 (s, 1H), 8.54 - 8.42 (m, 3H), 8.27 - 8.16 (m, 2H), 7.78 (d, J = 11.0 Hz, 1H), 7.30 (s, 1H), 6.53 (s, 1H), 5.61 - 5.51 (m, 1H), 5.42 (s, 2H), 5.20 - 5.05 (m, 2H), 4.56 - 4.42 (m, 2H), 4.32 - 4.22 (m, 1H), 3.96 - 3.87 (m, 3H), 3.79 (d, J = 5.6 Hz, 2H), 3.70 (d, J = 5.9 Hz, 2H), 3.25 - 3.08 (m, 2H), 2.61 - 2.53 (m, 2H), 2.45 - 2.36 (m, 4H), 2.36 - 2.22 (m, 3H), 2.20 - 2.03 (m, 4H), 1.99 - 1.68 (m, 4H), 0.87 (t, J = 7.3 Hz, 3H).

### Linker-payload X4

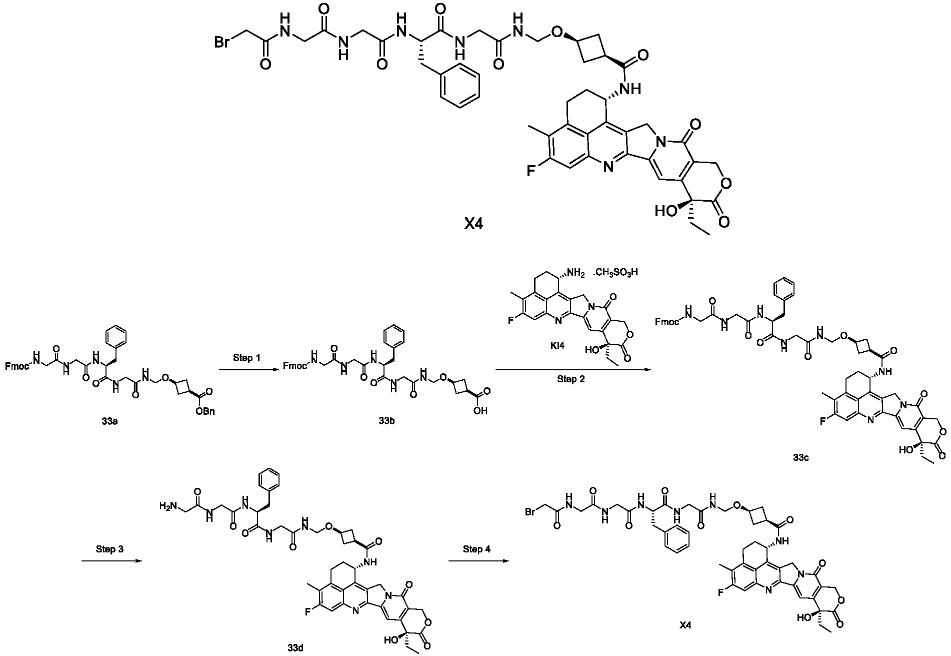

### Step 1

Pd/C (400 mg, 10 wt.%) was added to a solution of 33a (2.00 g, 2.58 mmol) in MeOH (20 mL), and the reaction mixture was stirred at 20°C for 5 hours. TLC (EA) indicated that the reaction was complete. The reaction mixture was filtered and subjected to rotary evaporation to dryness to obtain 1.3 g of white solid 33b with a yield of 74%.

### Step 2

HATU (305 mg, 0.802 mmol) was added to a solution of 33b (0.55 g, 0.802 mmol), KI4 (427 mg, 0.802 mmol), and DIPEA (310 mg, 2.40 mmol) in DMF (5 mL). The reaction mixture was stirred at 0°C for 2 hours. TLC (DCM/MeOH = 1/10) indicated that the reaction was complete. The reaction mixture was poured into water (40 mL) and filtered to obtain the crude product. The crude product was purified by column chromatography (DCM/MeOH = 20/1) to obtain 360 mg of yellow solid 33c with a yield of 41%.

### Step 3

Diethylamine (2 mL) was added to a solution of 33c (360 mg, 0.326 mmol) in DCM (10 mL). The reaction mixture was stirred at 25°C for 17 hours. TLC (DCM/MeOH = 5/1) indicated that the reaction was complete. The reaction mixture was poured into PE (100 mL) and filtered to obtain 205 mg of white solid 33d with a yield of 71%. MS-ESI: m/z 881.3 [M+H]+.

### Step 4

A solution of bromoacetyl bromide (94 mg, 0.446 mmol) in THF (2 mL) was added to a mixture of 33d (205 mg, 0.233 mmol) and triethylamine (118 mg, 1.17 mmol) in DMF (1 mL) and water (1 mL). The reaction mixture was stirred at 0°C for 1 hour and directly purified to obtain 15 mg of white solid X4 with a yield of 6%.

MS-ESI: m/z 1001.2 [M+H]+.

1H NMR (400 MHz, DMSO-d6) δ 8.57 - 8.50 (m, 1H), 8.50 - 8.43 (m, 2H), 8.35 - 8.29 (m, 1H), 8.19 - 8.12 (m, 2H), 7.80 (d, J = 10.8 Hz, 1H), 7.27 - 7.14 (m, 7H), 6.53 (s, 1H), 5.59 - 5.51 (m, 1H), 5.44 - 5.39 (m, 2H), 5.20 - 5.07 (m, 2H), 4.56 - 4.44 (m, 3H), 3.92 (s, 3H), 3.80 - 3.68 (m, 5H), 3.41 (s, 1H), 3.21 - 3.12 (m, 2H), 2.83 - 2.74 (m, 1H), 2.58 - 2.55 (m, 3H), 2.39 (s, 4H), 2.18 - 2.03 (m, 4H), 1.93 - 1.78 (m, 2H), 0.87 (t, J = 7.3 Hz, 3H).

### 2.3 Preparation of anti-GPC3 antibody-drug conjugates

### Preparation of antibody-drug conjugate ADC DB1001-X1

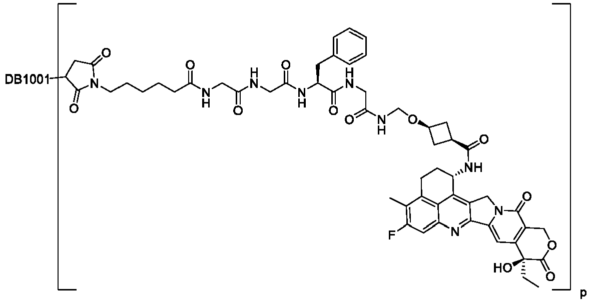

Ultra-pure water was used to prepare the reducing agent and protecting agent as follows: 2 mg/mL TCEP (Tris-2-carboxyethyl-phosphine, manufacturer: Thermo) aqueous solution and 100 mmol/L EDTA (disodium ethylenediaminetetraacetate, manufacturer: Sigma) aqueous solution.

Linker-payload X1 was dissolved in dry DMA (N,N-Dimethylacetamide, manufacturer: Sinopharm Group) to prepare a 10 mg/mL linker-payload DMA solution.

20 mg of DB1001 monoclonal antibody at a concentration of 6.28 mg/mL was placed in a 50 mL centrifuge tube, and diluted to a concentration of 3 mg/mL using 30 mM His-HAc, pH 5.5 buffer. EDTA aqueous solution (100 mM) was added according to 5% of the total volume of the reaction mixture, shaken and mixed well, and 2 mg/mL TCEP aqueous solution was added for antibody reduction. The molar ratio of TCEP to antibody was 18:1. After shaking and mixing well, the mixture was then placed on a refrigerated constant-temperature circulator to react at 37°C for 2 hours. The previously prepared DMA solution of linker-payload was added at a final molar ratio of 12:1 of drug to antibody, and DMA was supplemented according to 10% of the total volume of the reaction mixture. After shaking and mixing well, the mixture was then placed on a refrigerated constant-temperature circulator to react at 4°C for 1 hour. The sample preservation buffer was replaced using an ultrafiltration tube (MWCO 30KD, manufacturer: Millipore). The sample was first ultrafiltered three times with 30 mM His-HAc, pH 5.5 buffer containing 10% DMSO, and then ultrafiltered six times with 30 mM His-HAc, pH 5.5 buffer without DMSO. Finally, the sample was filtered for sterilization using a 0.2 µm PES membrane to obtain 12.7 mg of ADC DB1001-X1 at a concentration of 5.59 mg/mL with a yield of 63.5%.

HIC detection showed that the drug-to-antibody ratio (DAR) of the ADC DB1001-X1 was 7.51, and SEC analysis indicated a purity of 100%.

### Preparation of antibody-drug conjugate ADC DB1001-X2

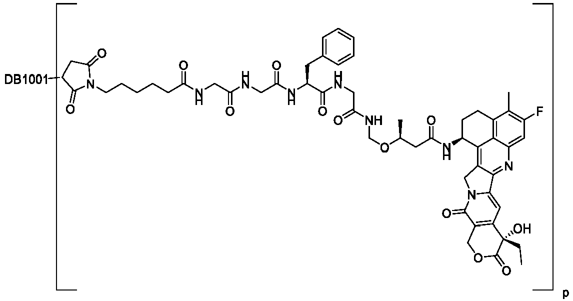

Ultra-pure water was used to prepare the reducing agent and protecting agent as follows: 2 mg/mL TCEP (Tris-2-carboxyethyl-phosphine, manufacturer: Thermo) aqueous solution and 100 mmol/L EDTA (disodium ethylenediaminetetraacetate, manufacturer: Sigma) aqueous solution.

Linker-payload X2 was dissolved in dry DMA (N,N-Dimethylacetamide, manufacturer: Sinopharm Group) to prepare a 10 mg/mL linker-payload DMA solution.

20 mg of DB1001 monoclonal antibody at a concentration of 6.28 mg/mL was placed in a 50 mL centrifuge tube, and diluted to a concentration of 3 mg/mL using 30 mM His-HAc, pH 5.5 buffer. EDTA aqueous solution (100 mM) was added according to 5% of the total volume of the reaction mixture, shaken and mixed well, and 2 mg/mL TCEP aqueous solution was added for antibody reduction. The molar ratio of TCEP to antibody was 18:1. After shaking and mixing well, the mixture was then placed on a refrigerated constant-temperature circulator to react at 37°C for 2 hours. The previously prepared DMA solution of linker-payload was added at a final molar ratio of 12:1 of drug to antibody, and DMA was supplemented according to 10% of the total volume of the reaction mixture. After shaking and mixing well, the mixture was then placed on a refrigerated constant-temperature circulator to react at 4°C for 1 hour. The sample preservation buffer was replaced using an ultrafiltration tube (MWCO 30KD, manufacturer: Millipore). The sample was first ultrafiltered three times with 30 mM His-HAc, pH 5.5 buffer containing 10% DMSO, and then ultrafiltered six times with 30 mM His-HAc, pH 5.5 buffer without DMSO. Finally, the sample was filtered for sterilization using a 0.2 µm PES membrane to obtain 14.3 mg of ADC DB1001-X2 at a concentration of 6.71 mg/mL with a yield of 71.5%.

HIC analysis showed that the drug-to-antibody ratio (DAR) of the ADC DB1001-X1 was 7.72, and SEC analysis indicated a purity of 99.3%.

### Preparation of antibody-drug conjugate ADC DB1001-Deruxtecan

The linker-payload used was Deruxtecan (CAS 1599440-13-7, purchased from Shanghai Haoyuan Chemexpress Co., Ltd.), and the antibody used was DB1001 of the present disclosure. DB1001-Deruxtecan was obtained with reference to the method described in Example 2.3 of the present disclosure.

### Example 3: Biological activity test

### 3.1 In vitro proliferation inhibition test of small molecule compounds (payload) on tumor cells

### Test purpose

To detect the *in vitro* proliferation inhibitory activity of the drug compounds on NCI-N87, JIMT-1, and MBA-MB-231 tumor cells. Cells were treated with different concentrations of the compounds *in vitro,* and after 6 days of culture, cell proliferation was detected using the CTG (CellTiter-Glo^{®} Luminescent Cell Viability Assay, Promega, Cat. No.: G7558) reagent. The IC₅₀ value was used to assess the *in vitro* activity of the compounds.
1. Cell culture: NCI-N87, JIMT-1, and MBA-MB-231 cells were cultured in RPMI-1640 medium containing 10% FBS.
2. Cell preparation: NCI-N87, JIMT-1, and MBA-MB-231 cells in the logarithmic growth phase were washed once with PBS, then digested with 2-3 mL of trypsin for 2-3 minutes. Once the cells were fully digested, 10-15 mL of cell culture medium was added to wash off the digested cells. The mixture was then centrifuged at 1000 rpm for 5 minutes, then the supernatant was discarded, and the cells were resuspended in 10-20 mL of cell culture medium to prepare a single-cell suspension.
3. Cell plating: The single-cell suspension of NCI-N87, JIMT-1, and MBA-MB-231 was mixed well, and the density of viable cells was adjusted to 6 × 10⁴ cells/mL using a cell culture medium. The density-adjusted cell suspension was mixed well and added to a 96-well cell culture plate at 50 µL per well. The culture plate was incubated in an incubator for 18 hours (37°C, 5% CO₂).
4. Compound preparation: The compounds were dissolved in DMSO to prepare stock solutions with an initial concentration of 10 mM.
   Eight concentrations of the small molecule compounds were prepared: 300, 100, 30, 10, 3, 1, 0.3, and 0.1 nM.
5. Sample addition operation: The prepared samples at different concentrations were added to a culture plate, with two replicate wells for each sample. The culture plate was incubated in an incubator for 6 days (37°C, 5% CO₂).
6. Color development operation: The 96-well cell culture plate was taken out, and 50 µL of CTG reagent was added to each well. The plate was incubated at room temperature for 10 minutes.
7. Plate reading operation: The 96-well cell culture plate was placed in a microplate reader, and the chemiluminescence was measured with the microplate reader.

Data analysis: The data were processed and analyzed using Microsoft Excel and GraphPad Prism 5.

**Table 1: IC₅₀ values of the small molecule compounds in the present disclosure for inhibiting tumor cell proliferation in vitro**

| Compound No. | NCI-N87 | JIMT-1 | MDA-MB-231 |
|---|---|---|---|
| | IC₅₀ (nM) | IC₅₀ (nM) | IC₅₀ (nM) |
| Preparation Example 7 | - | 23.1 | - |
| P-II-3 | 9.558 | 5.0 | 9.4 |
| P-II-4 | 8.032 | 6.6 | 7.6 |

| | | | |
|---|---|---|---|
| "-": not detected. Conclusion: Based on the results in Table 1, the payloads in the drug conjugates of the present disclosure exhibit significantly enhanced proliferation inhibitory activity against NCI-N87, JIMT-1, and MDA-MB-231 cells. | | | |

### 3.2 In vitro proliferation inhibition test of small molecule compounds (payloads) on tumor cells

### Test purpose

To detect the *in vitro* proliferation inhibitory activity of the drug compounds on NCI-N87, JIMT-1, and MBA-MB-231 tumor cells. Cells were treated with different concentrations of the compounds *in vitro,* and after 6 days of culture, cell proliferation was detected using the CTG (CellTiter-Glo^{®} Luminescent Cell Viability Assay, Promega, Cat. No.: G7558) reagent. The IC₅₀ value was used to assess the *in vitro* activity of the compounds.
1. Cell culture: NCI-N87, JIMT-1, and MBA-MB-231 cells were cultured in RPMI-1640 medium containing 10% FBS.
2. Cell preparation: NCI-N87, JIMT-1, and MBA-MB-231 cells in the logarithmic growth phase were washed once with PBS, then digested with 2-3 mL of trypsin for 2-3 minutes. Once the cells were fully digested, 10-15 mL of cell culture medium was added to wash off the digested cells. The mixture was then centrifuged at 1000 rpm for 5 minutes, then the supernatant was discarded, and the cells were resuspended in 10-20 mL of cell culture medium to prepare a single-cell suspension.
3. Cell plating: The single-cell suspension of NCI-N87, JIMT-1, and MBA-MB-231 was mixed well, and the density of viable cells was adjusted to 6 × 10⁴ cells/mL using a cell culture medium. The density-adjusted cell suspension was mixed well and added to a 96-well cell culture plate at 50 µL per well. The culture plate was incubated in an incubator for 18 hours (37°C, 5% CO₂).
4. Compound preparation: The compounds were dissolved in DMSO to prepare stock solutions with an initial concentration of 10 mM.
   Eight concentrations of the small molecule compounds were prepared: 300, 100, 30, 10, 3, 1, 0.3, and 0.1 nM.
5. Sample addition operation: The prepared samples at different concentrations were added to a culture plate, with two replicate wells for each sample. The culture plate was incubated in an incubator for 6 days (37°C, 5% CO₂).
6. Color development operation: The 96-well cell culture plate was taken out, and 50 µL of CTG reagent was added to each well. The plate was incubated at room temperature for 10 minutes.
7. Plate reading operation: The 96-well cell culture plate was placed in a microplate reader, and the chemiluminescence was measured with the microplate reader.
   Data analysis: The data were processed and analyzed using Microsoft Excel and GraphPad Prism 5.
8. Data analysis: The cell viability was calculated using the formula: V_{sample/}V_{vehicle control} x 100% where Vₛₐₘₚₗₑ represents the reading from the drug-treated group, and V_{vehicle control} represents the average value of the vehicle control group. GraphPad Prism software was used to plot the S-shaped dose-viability curve using a nonlinear regression model, and the IC₅₀ values were calculated.

**Table 2: IC₅₀ values of the small molecule compounds in the present disclosure for inhibiting proliferation of NCI-N87, JIMT-1, and MDA-MB-231 cells in vitro**

| Compound No. | NCI-N87 | JIMT-1 | MDA-MB-231 |
|---|---|---|---|
| | IC₅₀ (nM) | IC₅₀ (nM) | IC₅₀ (nM) |
| Reference Example 1 | - | 36.2 | - |
| P-III-20 | 5.888 | 4.351 | 6.65 |
| P-III-21 | 4.755 | 2.874 | 2.88 |

| | | | |
|---|---|---|---|
| "-": not detected Conclusion: Based on the results in Table 2, the payloads in the drug conjugates of the present disclosure exhibit significantly enhanced proliferation inhibitory activity against NCI-N87, JIMT-1, and MDA-MB-231 cells. | | | |

### 3.3 In vitro proliferation inhibition test of small molecule compounds (payloads) on tumor cells

### Test purpose

To detect the *in vitro* proliferation inhibitory activity of the drug compounds on NCI-N87 and Colo205 tumor cells. Cells were treated with different concentrations of the compounds *in vitro,* and after 6 days of culture, cell proliferation was detected using the CTG (CellTiter-Glo^{®} Luminescent Cell Viability Assay, Promega, Cat. No.: G7558) reagent. The IC₅₀ value was used to assess the *in vitro* activity of the compounds.
1. Cell culture: NCI-N87 and Colo205 cells were cultured in RPMI-1640 medium containing 10% FBS.
2. Cell preparation: NCI-N87 and Colo205 cells in the logarithmic growth phase were washed once with PBS, then digested with 2-3 mL of trypsin for 2-3 minutes. Once the cells were fully digested, 10-15 mL of cell culture medium was added to wash off the digested cells. The mixture was then centrifuged at 1000 rpm for 5 minutes, then the supernatant was discarded, and the cells were resuspended in 10-20 mL of cell culture medium to prepare a single-cell suspension.
3. Cell plating: The single-cell suspension of NCI-N87 and Colo205 was mixed well, and the density of viable cells was adjusted to 6 × 10⁴ cells/mL using a cell culture medium. The density-adjusted cell suspension was mixed well and added to a 96-well cell culture plate at 50 µL per well. The culture plate was incubated in an incubator for 18 hours (37°C, 5% CO₂).
4. Compound preparation: The compounds were dissolved in DMSO to prepare stock solutions with an initial concentration of 10 mM.
   Eight concentrations of the small molecule compounds were prepared: 300, 100, 30, 10, 3, 1, 0.3, and 0.1 nM.
5. Sample addition operation: The prepared samples at different concentrations were added to a culture plate, with two replicate wells for each sample. The culture plate was incubated in an incubator for 6 days (37°C, 5% CO₂).
6. Color development operation: The 96-well cell culture plate was taken out, and 50 µL of CTG reagent was added to each well. The plate was incubated at room temperature for 10 minutes.
7. Plate reading operation: The 96-well cell culture plate was placed in a microplate reader, and the chemiluminescence was measured with the microplate reader.

Data analysis: The data were processed and analyzed using Microsoft Excel and GraphPad Prism 5.

**Table 3: IC₅₀ values of the small molecule compounds in the present disclosure for inhibiting proliferation of NCI-N87 and Colo205 cells in vitro**

| Compound No. | Abs-IC₅₀ (nM) | | | |
|---|---|---|---|---|
| | P-III-30 | Reference Example 1 | Preparation Example 8 | Preparation Example 9 |
| Colo205 | 102.9 | >1000 | - | 294.3 |
| NCI-N87 | 6.5 | >300 | 15.1 | 13.9 |

| | | | | |
|---|---|---|---|---|
| Conclusion: Based on the results in Table 3, the payloads in the drug conjugates of the present disclosure exhibit significantly enhanced proliferation inhibitory activity against NCI-N87 and Colo205 cells. | | | | |

### Example 4: In vitro proliferation inhibition test of antibody-drug conjugates on tumor cells

### 4.1 In vitro proliferation inhibition test of DB1001 and DB1001-X1

The CellTiter-Glo^{®} luminescent cell viability assay (CTG method) was used to evaluate the inhibitory effect of anti-GPC3 naked antibody DB1001 and anti-GPC3 ADC DB1001-X1 on the proliferation of GPC3-positive expression human liver cancer cells HepG2 and Huh7 after 7 days of treatment.

Cells in the logarithmic growth phase were collected and plated at a density of 4000 cells/well (HepG2 cells) or 6000 cells/well (Huh7 cells). The cell plate was placed in a 37°C, 5% CO₂ incubator and cultured overnight. On the second day of the experiment, DB1001 and DB1001-X1 were diluted with a complete medium in a 3-fold serial dilution to obtain 9 concentration gradients, starting with a maximum concentration of 300 nM. The obtained solutions were added to the cell culture plate at 50 µL/well with a complete medium used as a blank control, and 3 replicate wells were set up. The plate was incubated for another 7 days in a 37°C, 5% CO₂ incubator. At the end of the incubation, the cell culture plate was taken out and equilibrated to room temperature. 50 µL of CTG detection reagent (Promega, Cat#: G7573) was added to each well, and the mixture was shaken and mixed well. After the mixture was allowed to stand for 10 minutes, the luminescence signal was detected and read using a microplate reader. GraphPad Prism software was used to plot the S-shaped dose-response curve using a nonlinear regression model, and the IC₅₀ values were calculated. The cell viability was calculated using the formula: cell viability = (Lum_{test compound} - Lum_{blank control})/(Lum_{vehicle control} - Lum_{blank control}) × 100% The experimental results are shown in the table below:

**Table 4: Proliferation inhibitory activity of antibodies and antibody-drug conjugates against human liver cancer cells**

| Cell | Drug | Cell killing IC₅₀ (nM) |
|---|---|---|
| Huh7 (GPC3+) | DB1001-X1 | 0.65 |
| | DB1001 | >300 |
| HepG2 (GPC3+) | DB1001-X1 | 2.63 |
| | DB1001 | >300 |

| | | |
|---|---|---|
| Conclusion: Based on the results in Table 4, the antibody-drug conjugate DB1001-X1 of the present disclosure exhibits significant proliferation inhibitory activity against GPC3-positive expression cells Huh7 and HepG2, and its efficacy is notably superior to that of the antibody DB1001. | | |

### 4.2 In vitro proliferation inhibition test of DB1001-X1 and DB1001-X2

The CTG method was used to evaluate the inhibitory effects of anti-GPC3 ADCs DB1001-X1 and DB1001-X2 on the proliferation of GPC3-positive expression human liver cancer cells HepG2 and Huh7 after 6 days of treatment.

Cells in the logarithmic growth phase were collected and plated at a density of 5000 cells/well (HepG2 cells) or 5000 cells/well (Huh7 cells). The cell plate was placed in a 37°C, 5% CO₂ incubator and cultured overnight. On the second day of the experiment, DB1001-X1 and DB1001-X2 were diluted with a complete medium in a 3-fold serial dilution to obtain 9 concentration gradients, starting with a maximum concentration of 1000 nM. The obtained solutions were added to the cell culture plate at 50 µL/well with a complete medium used as a blank control, and 3 replicate wells were set up. The plate was incubated for another 7 days in a 37°C, 5% CO₂ incubator. At the end of the incubation, the cell culture plate was taken out and equilibrated to room temperature. 50 µL of CTG detection reagent (Promega, Cat#: G7573) was added to each well, and the mixture was shaken and mixed well. After the mixture was allowed to stand for 10 minutes, the luminescence signal was detected and read using a microplate reader. GraphPad Prism software was used to plot the S-shaped dose-response curve using a nonlinear regression model, and the IC₅₀ values were calculated. The cell viability was calculated using the formula: cell viability = (Lum_{test compound} - Lum_{blank control})/(Lum_{vehicle control} - Lum_{blank control}) × 100% The experimental results are shown in the table below:

**Table 5: Proliferation inhibitory activity of antibodies and antibody-drug conjugates against human liver cancer cells**

| Cell | Drug | Cell killing IC₅₀ (nM) |
|---|---|---|
| Huh7 (GPC3+) | DB1001-X1 | 4.29 |
| | DB1001-X2 | 3.91 |
| HepG2 (GPC3+) | DB1001-X1 | 8.37 |
| | DB1001-X2 | 7.02 |

| | | |
|---|---|---|
| Conclusion: Based on the results in Table 5, the antibody-drug conjugates DB1001-X1 and DB1001-X2 of the present disclosure exhibit significantly enhanced proliferation inhibitory activity against GPC3-positive expression cells Huh7 and HepG2. | | |

### Example 5: In vivo tumor inhibition test of antibody-drug conjugates

To study the inhibitory effect of DB1001-X1 on tumor formation *in vivo,* the antitumor effect of DB1001-X1 was evaluated after xenograft tumors were formed in mice with GPC3-positive expression human liver cancer cells HepG2 or Huh7.

### 5.1 Efficacy evaluation of antibody-drug conjugates in HepG2 tumor-bearing mice

1. Test drugs and materials
   Blank control group (control group): normal saline
   DB1001-X1 (treatment group): 10 mg/kg
   DB1001 (treatment group): 10 mg/kg
2. Preparation method: All samples were diluted and prepared with normal saline.
3. Test animals: Female BALB/c mice, 8 weeks old, purchased from GemPharmatech Co., Ltd.
4. Test methods:

1 × 10⁷ HepG2 cells were subcutaneously inoculated into the right anterior scapula of 8-week-old female BALB/c nude mice. When the tumor grew to approximately 110 mm³, the tumor-bearing mice were randomly grouped using StudyDirector^{™} software, and on the same day (Day 0), DB1001 and DB1001-X1 were administered via intravenous injection (i.v.) once. The experiment was terminated after 21 days of administration. Tumor volume and body weight were measured twice weekly, and data were recorded.

Six mice were included in each group (vehicle control group or treatment group). Tumor growth inhibition rate (TGI%) was calculated by measuring tumor volume. TGI% = 100% - (Tumor volume of treatment group on measurement day - Tumor volume of treatment group on day 0) / (Tumor volume of control group on measurement day - Tumor volume of control group on day 0)

**Table 6: In vivo tumor inhibition effects of antibody-drug conjugates in HepG2 tumor-bearing mice**

| Treatment group | D13 TGI% | D16 TGI% | D20 TGI% |
|---|---|---|---|
| DB1001 | 6.65% | 0.13% | -11.97% |
| DB1001-X1 | 98.90% | 94.97% | 86.44% |

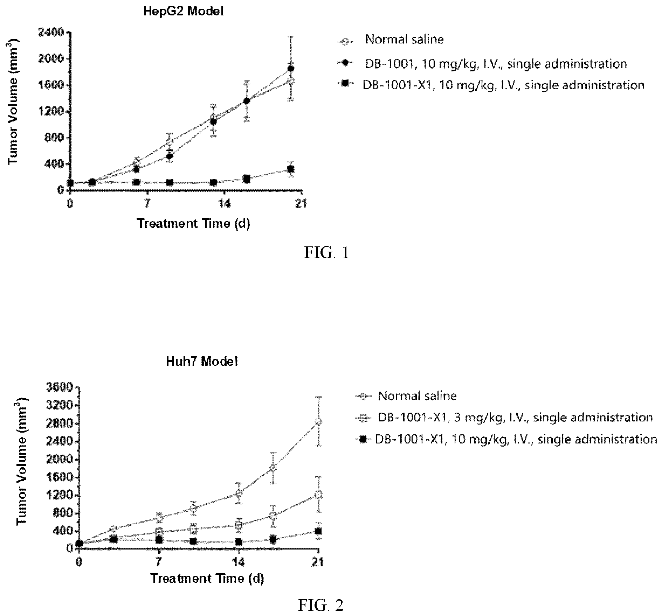

Experimental results are shown in FIG. 1 and Table 6. The anti-GPC3 naked antibody DB1001 of the present disclosure does not exhibit a significant tumor inhibition effect after a single administration. The antibody-drug conjugate DB1001-X1 exhibits significantly enhanced antitumor activity after a single administration.

### 5.2 Efficacy evaluation of antibody-drug conjugates in Huh7 tumor-bearing mice

1. Test drugs and materials
   Blank control group (control group): normal saline
   DB1001-X1 (treatment group): 3 mg/kg
   DB1001-X1 (treatment group): 10 mg/kg
2. Preparation method: All samples were diluted and prepared with normal saline.
3. Test animals: Female BALB/c mice, 8 weeks old, purchased from GemPharmatech Co., Ltd.
4. Test methods:

5 × 10⁶ Huh7 cells were subcutaneously inoculated into the right anterior scapula of 8-week-old female BALB/c nude mice. When the tumor grew to approximately 130 mm³, the tumor-bearing mice were randomly grouped using StudyDirector^{™} software, and on the same day (Day 0), DB1001-X1 (3 mg/kg) and DB1001-X1 (10 mg/kg) were administered via intravenous injection (i.v.) once. The experiment was terminated after 21 days of administration. Tumor volume and body weight were measured twice weekly, and data were recorded.

Six mice were included in each group (vehicle control group or treatment group). Tumor growth inhibition rate (TGI%) was calculated by measuring tumor volume. TGI% = 100% - (Tumor volume of treatment group on measurement day - Tumor volume of treatment group on day 0) / (Tumor volume of control group on measurement day - Tumor volume of control group on day 0)

**Table 7: In vivo tumor inhibition effects of antibody-drug conjugates in Huh7 tumor-bearing mice**

| Treatment group | D14 TGI% | D17 TGI% | D21 TGI% |
|---|---|---|---|
| DB1001-X1, 3 mpk | 63.83% | 63.59% | 59.76% |
| DB1001-X1, 10 mpk | 97.09% | 94.70% | 89.94% |

The experimental results are shown in FIG. 2 and Table 7. The antibody-drug conjugate DB1001-X1 exhibits significant dose-dependent tumor inhibitory activity after a single administration.

### 5.3 Efficacy evaluation of antibody-drug conjugates in HepG2 tumor-bearing mice

(1) Test drugs and materials
   Blank control group (control group): normal saline
   DB1001-Deruxtecan (treatment group): 1 mg/kg
   DB1001-X2 (treatment group): 1 mg/kg
   DB1001-Deruxtecan (treatment group): 3 mg/kg
   DB1001-X2 (treatment group): 3 mg/kg
(2) Preparation method: All samples were diluted and prepared with normal saline.
(3) Test animals: Female BALB/c mice, 8 weeks old, purchased from GemPharmatech Co., Ltd.
(4) Test methods:

1 × 10⁷ HepG2 cells were subcutaneously inoculated into the right anterior scapula of 8-week-old female BALB/c nude mice. When the tumor grew to approximately 110 mm³, the tumor-bearing mice were randomly grouped using StudyDirector^{™} software, and on the same day (Day 0), DB1001-X1 and DB1001-X2 were administered via intravenous injection (i.v.) once. The experiment was terminated after 21 days of administration. Tumor volume and body weight were measured twice weekly, and data were recorded.

Tumor growth inhibition rate (TGI%) was calculated by measuring tumor volume. TGI% = 100% - (Tumor volume of treatment group on measurement day - Tumor volume of treatment group on day 0)/(Tumor volume of control group on measurement day - Tumor volume of control group on day 0)

**Table 8: In vivo tumor inhibition effects of DB1001-Deruxtecan and DB1001-X2 in HepG2 tumor-bearing mice**

| Treatment group | TGI% |
|---|---|
| DB 1001-Deruxtecan, 1 mpk | + |
| DB 1001-Deruxtecan, 3 mpk | ++ |
| DB1001-X2, 1 mpk | ++ |
| DB1001-X2, 3 mpk | +++ |

| | |
|---|---|
| +: TGI < 50% ++: 60 ≤ TGI% < 75% +++: TGI% ≥ 80% The experimental results indicate that the antibody-drug conjugates in the present disclosure exhibit significantly enhanced antitumor activity after a single administration. | |

### 5.4 Efficacy evaluation of antibody-drug conjugates in Huh7 tumor-bearing mice

(1) Test drugs and materials
   Blank control group (control group): normal saline
   DB1001-Deruxtecan (treatment group): 1 mg/kg
   DB1001-X2 (treatment group): 1 mg/kg
   DB1001-Deruxtecan (treatment group): 3 mg/kg
   DB 1001-X2 (treatment group): 3 mg/kg
(2) Preparation method: All samples were diluted and prepared with normal saline.
(3) Test animals: Female BALB/c naked mice, 8 weeks old, purchased from GemPharmatech Co., Ltd.
(4) Test methods:

5 × 10⁶ Huh7 cells were subcutaneously inoculated into the right anterior scapula of 8-week-old female BALB/c nude mice. When the tumor grew to approximately 110 mm³, the tumor-bearing mice were randomly grouped using StudyDirector^{™} software, and on the same day (Day 0), DB1001-X1 and DB1001-X2 were administered via intravenous injection (i.v.) once. The experiment was terminated after 21 days of administration. Tumor volume and body weight were measured twice weekly, and data were recorded.

Tumor growth inhibition rate (TGI%) was calculated by measuring tumor volume. TGI% = 100% - (Tumor volume of treatment group on measurement day - Tumor volume of treatment group on day 0)/(Tumor volume of control group on measurement day - Tumor volume of control group on day 0)

**Table 9: In vivo tumor inhibition effects of DB1001-Deruxtecan and DB1001-X2 in Huh7 tumor-bearing mice**

| Treatment group | TGI% |
|---|---|
| DB 1001-Deruxtecan, 1 mpk | + |
| DB 1001-Deruxtecan, 3 mpk | ++ |
| DB1001-X2, 1 mpk | ++ |
| DB1001-X2, 3 mpk | +++ |

| | |
|---|---|
| +: TGI < 50% ++: 60 ≤ TGI% < 75% +++: TGI% ≥ 80% The experimental results indicate that the antibody-drug conjugates in the present disclosure exhibit significantly enhanced antitumor activity after a single administration. | |

Although the specific embodiments of the present disclosure have been described above, those skilled in the art will understand that these are merely illustrative. Various changes or modifications can be made to these embodiments without departing from the principles and spirit of the present disclosure. Therefore, the scope of protection of the present disclosure is defined by the appended claims.

## Claims

1. An anti-GPC3 antibody-drug conjugate, an isomer thereof, a pharmaceutically acceptable salt thereof, or a mixture thereof, wherein the anti-GPC antibody-drug conjugate has a structure as shown in formula (I-1): 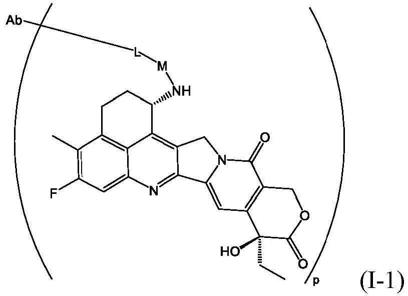 wherein
M is -L²-L³-X-L¹-;
L² is -O- or -S-;
L³ is -(C(R^{1a})(R^{1b}))ₘ- and m is 0, 1, 2, or 3, wherein when L³ comprises a methylene unit, 0 or 1 methylene unit of the L³ is replaced by -C(O)- or -C(S)-;
L¹ is -(C(R^{2a})(R^{2b}))ₙ- and n is 1, 2, or 3, wherein when L¹ comprises a methylene unit, 0 or 1 methylene unit of the L¹ is replaced by -C(O)- or -C(S)-;
X is 3- to 6-membered saturated carbocyclyl, 3- to 6-membered saturated heterocyclyl, or a single bond, and the 3- to 6-membered saturated carbocyclyl and the 3- to 6-membered saturated heterocyclyl are optionally substituted by one or more R^{3a};
wherein R^{1a}, R^{1b}, R^{2a}, R^{2b}, and R^{3a} are each independently hydrogen, halogen, or a C₁₋₆ aliphatic group optionally substituted by one or more R;
wherein each R is independently hydrogen or halogen;
L is a linker unit;
p represents the average number of connections, and p is an integer or a decimal from 1 to 10;
Ab is an anti-GPC3 antibody or an antigen-binding fragment thereof.

2. The anti-GPC3 antibody-drug conjugate, the isomer thereof, the pharmaceutically acceptable salt thereof, or the mixture thereof according to claim 1, wherein the anti-GPC3 antibody or the antigen-binding fragment thereof comprises a heavy chain variable region and a light chain variable region; the heavy chain variable region comprises the amino acid sequences of HCDR1, HCDR2, and HCDR3 as shown in SEQ ID NO: 1, SEQ ID NO: 2, and SEQ ID NO: 3, respectively, and the light chain variable region comprises the amino acid sequences of LCDR1, LCDR2, and LCDR3 as shown in SEQ ID NO: 4, SEQ ID NO: 5, and SEQ ID NO: 6, respectively.

3. The anti-GPC3 antibody-drug conjugate, the isomer thereof, the pharmaceutically acceptable salt thereof, or the mixture thereof according to claim 1 or 2, wherein the anti-GPC3 antibody or the antigen-binding fragment thereof comprises a heavy chain variable region having an amino acid sequence as shown in SEQ ID NO: 7 or a heavy chain variable region having at least 95%, 96%, 97%, 98%, or 99% identity thereto, and a light chain variable region having an amino acid sequence as shown in SEQ ID NO: 8 or a light chain variable region having at least 95%, 96%, 97%, 98%, or 99% identity thereto.

4. The anti-GPC3 antibody-drug conjugate, the isomer thereof, the pharmaceutically acceptable salt thereof, or the mixture thereof according to claim 1 or 2, wherein the anti-GPC3 antibody or the antigen-binding fragment thereof is a murine antibody or a fragment thereof, a chimeric antibody or a fragment thereof, or a humanized antibody or a fragment thereof.

5. The anti-GPC3 antibody-drug conjugate, the isomer thereof, the pharmaceutically acceptable salt thereof, or the mixture thereof according to claim 1 or 2, wherein the anti-GPC3 antibody or the antigen-binding fragment thereof is Fab, Fab', Fab'-SH, Fv, scFv, F(ab')₂, sdAb, a bispecific antibody, or a linear antibody.

6. The anti-GPC3 antibody-drug conjugate, the isomer thereof, the pharmaceutically acceptable salt thereof, or the mixture thereof according to claim 1 or 2, wherein the antibody is in the form of an IgG1 antibody, in the form of an IgG2 antibody, in the form of an IgG3 antibody, or in the form of an IgG4 antibody.

7. The anti-GPC3 antibody-drug conjugate, the isomer thereof, the pharmaceutically acceptable salt thereof, or the mixture thereof according to claim 1 or 2, wherein the anti-GPC3 antibody or the antigen-binding fragment thereof comprises a heavy chain having an amino acid sequence as shown in SEQ ID NO: 9 or a heavy chain having at least 95%, 96%, 97%, 98%, or 99% identity thereto, and a light chain having an amino acid sequence as shown in SEQ ID NO: 10 or a light chain having at least 95%, 96%, 97%, 98%, or 99% identity thereto.

8. The anti-GPC3 antibody-drug conjugate, the isomer thereof, the pharmaceutically acceptable salt thereof, or the mixture thereof according to any one of claims 1 to 7, wherein p is an integer or a decimal from 3 to 8.

9. The anti-GPC3 antibody-drug conjugate, the isomer thereof, the pharmaceutically acceptable salt thereof, or the mixture thereof according to any one of claims 1 to 7, wherein L³ is -(C(R^{1a})(R^{1b}))ₘ-, wherein m is 0, 1, 2, or 3;
wherein R^{1a} and R^{1b} are each independently hydrogen, halogen, or a C₁₋₆ aliphatic group optionally substituted by 1, 2, or 3 R, preferably hydrogen, methyl, ethyl, or propyl;
wherein each R is independently hydrogen or halogen.

10. The anti-GPC3 antibody-drug conjugate, the isomer thereof, the pharmaceutically acceptable salt thereof, or the mixture thereof according to claim 9, wherein L³ is a single bond, -CH₂-, -CH(CH₃)-, -C(CH₃)₂-, -CH₂CH₂-, -CH(CH₃)CH₂-, or -C(CH₃)₂CH₂-.

11. The anti-GPC3 antibody-drug conjugate, the isomer thereof, the pharmaceutically acceptable salt thereof, or the mixture thereof according to any one of claims 1 to 7, wherein X is 3- to 6-membered saturated carbocyclyl optionally substituted by 1, 2, or 3 R^{3a} or a single bond;
wherein each R^{3a} is independently hydrogen, halogen, or a C₁₋₆ aliphatic group optionally substituted by 1, 2, or 3 R;
wherein each R is independently hydrogen or halogen.

12. The anti-GPC3 antibody-drug conjugate, the isomer thereof, the pharmaceutically acceptable salt thereof, or the mixture thereof according to claim 11, wherein X is 3- to 6-membered saturated carbocyclyl or a single bond, preferably cyclopropyl, cyclobutyl, cyclcopentyl, cyclohexyl, or a single bond, more preferably 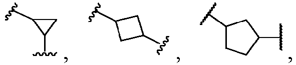 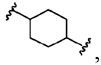 or a single bond.

13. The anti-GPC3 antibody-drug conjugate, the isomer thereof, the pharmaceutically acceptable salt thereof, or the mixture thereof according to any one of claims 1 to 7, wherein - L³-X- is -(C(R^{1a})(R^{1b}))ₘ-, 3- to 6-membered saturated carbocyclyl, or 3- to 6-membered saturated heterocyclyl;
wherein m is 1, 2, or 3, and the 3- to 6-membered saturated carbocyclyl and the 3- to 6-membered saturated heterocyclyl are optionally substituted by 1, 2, or 3 R^{3a};
wherein R^{1a}, R¹⁶, and R^{3a} are each independently hydrogen, halogen, or a C₁₋₆ aliphatic group optionally substituted by 1, 2, or 3 R;
wherein each R is independently hydrogen or halogen.

14. The anti-GPC3 antibody-drug conjugate, the isomer thereof, the pharmaceutically acceptable salt thereof, or the mixture thereof according to claim 13, wherein the -L³-X- is - (C(R^{1a})(R^{1b}))ₘ- or 3- to 6-membered saturated carbocyclyl optionally substituted by 1, 2, or 3 R^{3a}; for example, -L³-X- is -(C(R^{1a})(R^{1b}))ₘ-, cyclopropyl, cyclobutyl, cyclopentyl, or cyclohexyl, and the cyclopropyl, cyclobutyl, cyclopentyl, and cyclohexyl are optionally substituted by 1, 2, or 3 R^{3a}; preferably, -L³-X- is -(C(R^{1a})(R^{1b}))ₘ-, 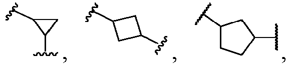 or 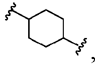 and the 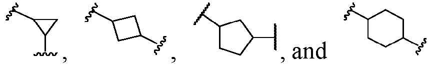 are optionally substituted by 1, 2, or 3 R^{3a};
m is 1, 2, or 3;
each R^{1a} is independently halogen or a C₁₋₆ aliphatic group optionally substituted by 1, 2, or 3 R, such as methyl, ethyl, or propyl; R^{1b} and R^{3a} are each independently hydrogen, halogen, or a C₁₋₆ aliphatic group optionally substituted by 1, 2, or 3 R, such as hydrogen, methyl, ethyl, or propyl; each R is independently hydrogen or halogen.

15. The anti-GPC3 antibody-drug conjugate, the isomer thereof, the pharmaceutically acceptable salt thereof, or the mixture thereof according to claim 14, wherein the -L³-X- is

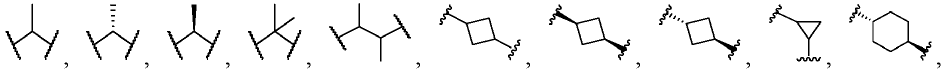

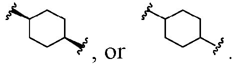

16. The anti-GPC3 antibody-drug conjugate, the isomer thereof, the pharmaceutically acceptable salt thereof, or the mixture thereof according to any one of claims 1 to 7, wherein L¹ is -C(R^{2a})(R^{2b})-, -C(R^{2a})(R^{2b})C(O)-, or -C(O)-;
wherein R^{2a} and R^{2b} are each independently hydrogen, halogen, or a C₁₋₆ aliphatic group optionally substituted by R, preferably hydrogen, methyl, ethyl, or propyl;
wherein each R is independently hydrogen or halogen.

17. The anti-GPC3 antibody-drug conjugate, the isomer thereof, the pharmaceutically acceptable salt thereof, or the mixture thereof according to claim 16, wherein L¹ is -CH₂C(O)-, -CH(CH₃)C(O)-, or -C(O)-.

18. The anti-GPC3 antibody-drug conjugate, the isomer thereof, the pharmaceutically acceptable salt thereof, or the mixture thereof according to any one of claims 1 to 17, wherein
M is -L²-L³-X-L¹-;
L² is -O-;
-L³-X- is 3- to 6-membered saturated carbocyclyl optionally substituted by 1, 2, or 3 R^{3a}; alternatively, -L³-X- is cyclopropyl, cyclobutyl, cyclopentyl, or cyclohexyl, and the cyclopropyl, cyclobutyl, cyclopentyl, and cyclohexyl are optionally substituted by 1, 2, or 3 R^{3a}; preferably, the -L³-X- is 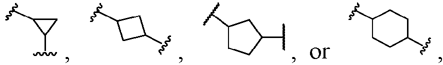 and the 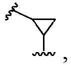 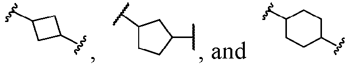 are optionally substituted by 1, 2, or 3 R^{3a};
L¹ is -C(O)-;
wherein each R^{3a} is independently hydrogen, halogen, or a C₁₋₆ aliphatic group optionally substituted by R;
wherein each R is independently hydrogen or halogen.

19. The anti-GPC3 antibody-drug conjugate, the isomer thereof, the pharmaceutically acceptable salt thereof, or the mixture thereof according to claim 18, wherein
M is -L²-L³-X-L¹-;
L² is -O-;
-L³-X is 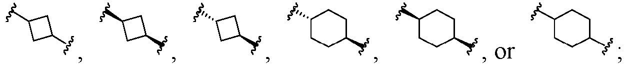
L¹ is -C(O)-.

20. The anti-GPC3 antibody-drug conjugate, the isomer thereof, the pharmaceutically acceptable salt thereof, or the mixture thereof according to any one of claims 1 to 17, wherein
M is -L²-L³-X-L¹-;
L² is -O-;
-L³-X- is -(C(R^{1a})(R^{1b}))ₘ-, wherein m is 1, 2, or 3;
L¹ is -CH₂C(O)-;
wherein each R^{1a} is independently halogen or a C₁₋₆ aliphatic group optionally substituted by 1, 2, or 3 R; R^{1b} and R^{3a} are each independently hydrogen, halogen, or a C₁₋₆ aliphatic group optionally substituted by 1, 2, or 3 R;
wherein each R is independently hydrogen or halogen.

21. The anti-GPC3 antibody-drug conjugate, the isomer thereof, the pharmaceutically acceptable salt thereof, or the mixture thereof according to claim 20, wherein
M is -L²-L³-X-L¹-;
L² is -O-;
-L³-X- is 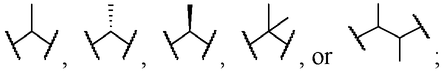
L¹ is -CH₂C(O)-.

22. The anti-GPC3 antibody-drug conjugate, the isomer thereof, the pharmaceutically acceptable salt thereof, or the mixture thereof according to claim 21, wherein
M is -L²-L³-X-L¹-;
L² is -O-;
-L³-X- is 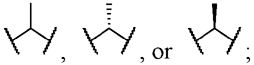
L¹ is -CH₂C(O)-.

23. The anti-GPC3 antibody-drug conjugate, the isomer thereof, the pharmaceutically acceptable salt thereof, or the mixture thereof according to any one of claims 1 to 17, wherein -M- is

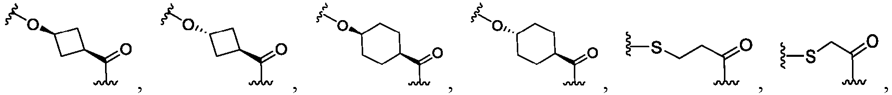

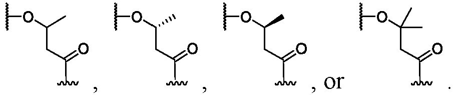

24. The anti-GPC3 antibody-drug conjugate, the isomer thereof, the pharmaceutically acceptable salt thereof, or the mixture thereof according to any one of claims 1 to 23, wherein L is -Lₐ-L_{b}-L_{c}-,
-Lₐ- is 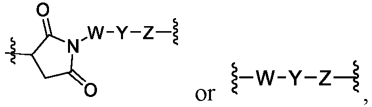
wherein W is -(C(R^{wa})(R^{wb}))_{wn}-, Y is -(OCH₂CH₂)_{yn}-O_{yp}-, and Z is -(C(R^{za})(R^{zb}))_{zn};
wherein wn is 1, 2, 3, or 6;
0 or 1 methylene unit of W is each independently substituted by -Cyr-, -N(R^{wx})C(O)-, - C(O)N(R^{wx})-, or -C(O)-;
wherein yn is 0, 4, or 8, and yp is 0 or 1;
wherein zn is 1, 2, or 3;
1 methylene unit of Z is each independently substituted by -Cyr-, -N(R^{zx})C(O)-, - C(O)N(R^{zx})-, or -C(O)-;
-Cyr- is 3- to 10-membered saturated carbocyclylene, wherein the -Cyr- is unsubstituted or independently substituted by 1 to 3 substituents R^{cx};
wherein R^{wa}, R^{wb}, R^{za}, R^{zb}, R^{wx}, R^{zx}, and R^{cx} are each independently hydrogen, halogen, - OR^{r}, or a C₁₋₆ aliphatic group optionally substituted by R^{r};
wherein each R^{f} is independently hydrogen, halogen, or a C₁₋₆ aliphatic group;
-L_{b}- represents a peptide residue consisting of 2 to 4 amino acids, and the peptide residue of -L_{b}- is formed from amino acids selected from the group consisting of phenylalanine, glycine, alanine, valine, citrulline, and lysine;
-L_{c}- is 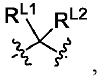
wherein R^{L1} and R^{L2} are each independently selected from the following group consisting of hydrogen, halogen, -OH, and a C₁₋₆ aliphatic group.

25. The anti-GPC3 antibody-drug conjugate, the isomer thereof, the pharmaceutically acceptable salt thereof, or the mixture thereof according to claim 24, wherein the -Lₐ- is

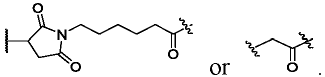

26. The anti-GPC3 antibody-drug conjugate, the isomer thereof, the pharmaceutically acceptable salt thereof, or the mixture thereof according to claim 24, wherein the -L_{b}- is selected from the following group consisting of: 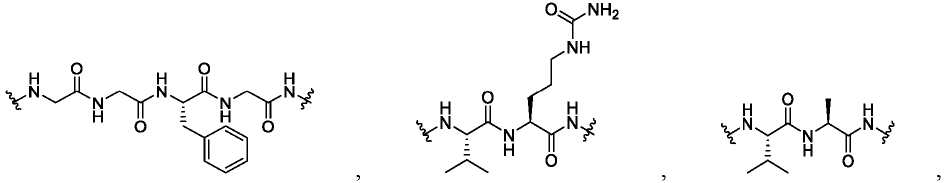 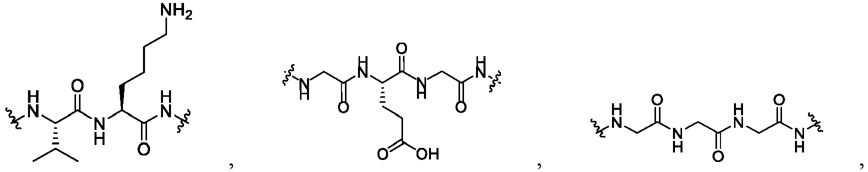
and 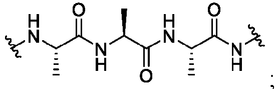
preferably, -L_{b}- is

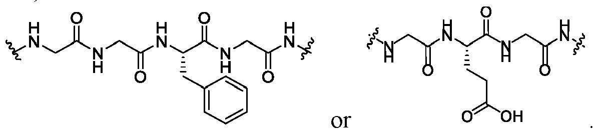

27. The anti-GPC3 antibody-drug conjugate, the isomer thereof, the pharmaceutically acceptable salt thereof, or the mixture thereof according to claim 24, wherein -L_{c}- is

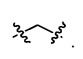

28. The anti-GPC3 antibody-drug conjugate, the isomer thereof, the pharmaceutically acceptable salt thereof, or the mixture thereof according to any one of claims 24 to 27, wherein L is

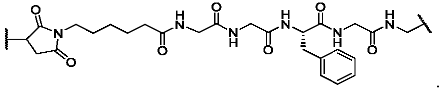

29. The anti-GPC3 antibody-drug conjugate, the isomer thereof, the pharmaceutically acceptable salt thereof, or the mixture thereof according to any one of claims 24 to 27, wherein L is

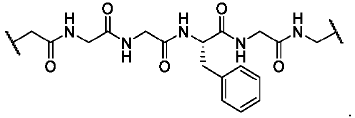

30. The anti-GPC3 antibody-drug conjugate, the isomer thereof, the pharmaceutically acceptable salt thereof, or the mixture thereof according to any one of claims 1 to 29, wherein the anti-GPC3 antibody-drug conjugate has a structure as shown in formula (II-1) or (II-2): 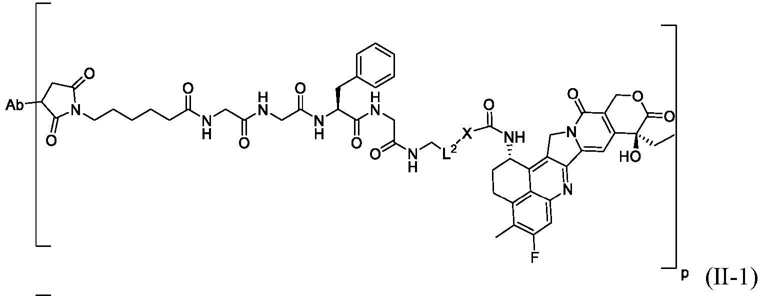 or 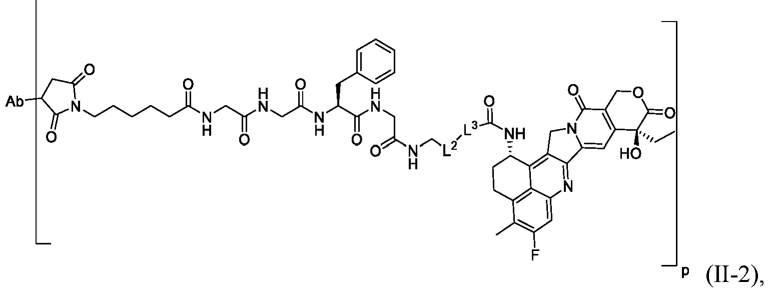 wherein
p is as defined in claim 1 or 8;
Ab is as defined in any one of claims 1 to 7;
L² is -O- or -S-, preferably -O-;
X is 3- to 6-membered saturated carbocyclyl optionally substituted by 1, 2, or 3 R^{3a}; preferably, X is 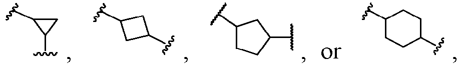 and the 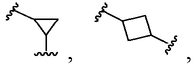 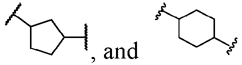 are optionally substituted by 1, 2, or 3 R^{3a};
L³ is -C(R^{1a})(R¹⁶)-CH₂- or -C(R^{1a})(R¹⁶)C(R^{1a})(R¹⁶)-CH₂-;
each R^{1a} is independently halogen or a C₁₋₆ aliphatic group optionally substituted by 1, 2, or 3 R; R^{1b} and R^{3a} are each independently hydrogen, halogen, or a C₁₋₆ aliphatic group optionally substituted by 1, 2, or 3 R; each R is independently hydrogen or halogen.

31. The anti-GPC3 antibody-drug conjugate, the isomer thereof, the pharmaceutically acceptable salt thereof, or the mixture thereof according to any one of claims 1 to 30, wherein the anti-GPC3 antibody-drug conjugate is selected from the following structures: 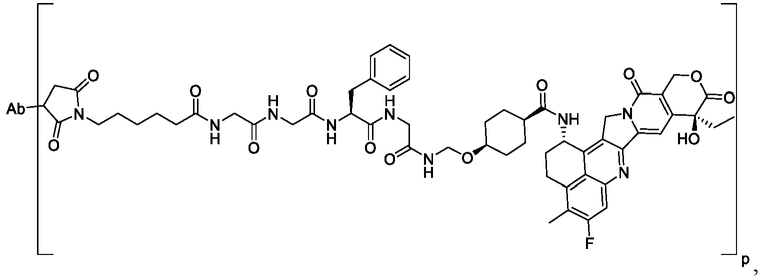 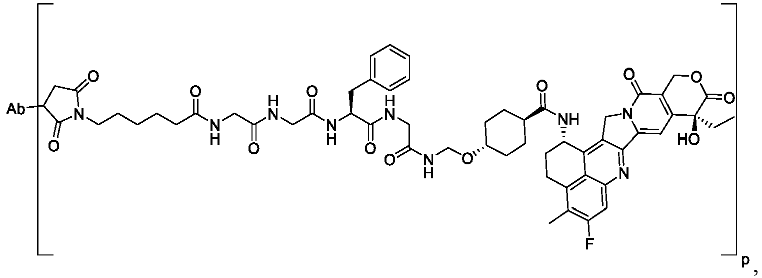 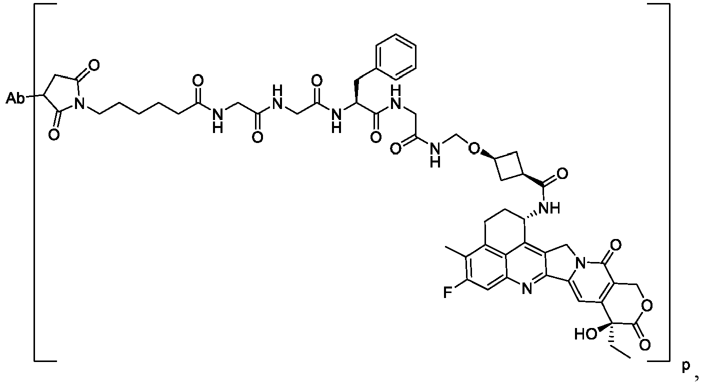 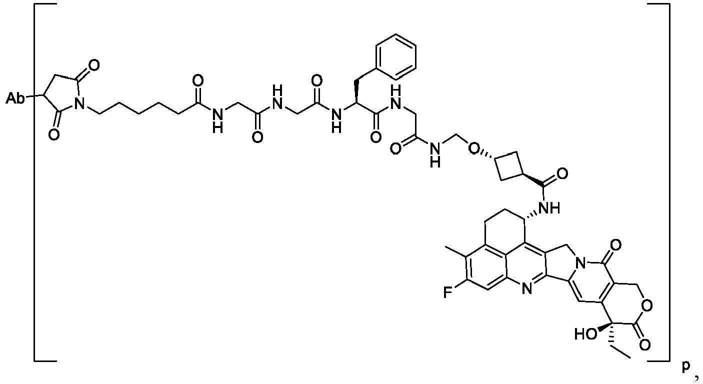 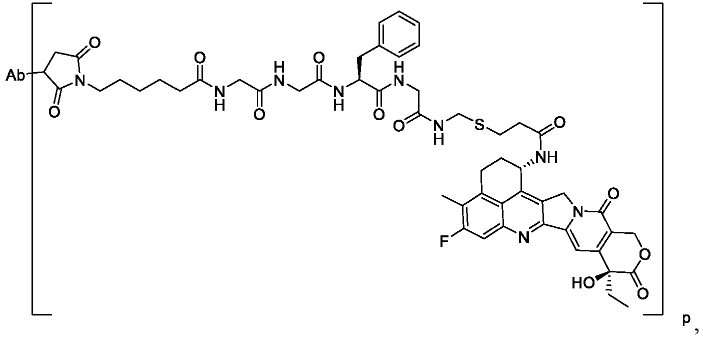 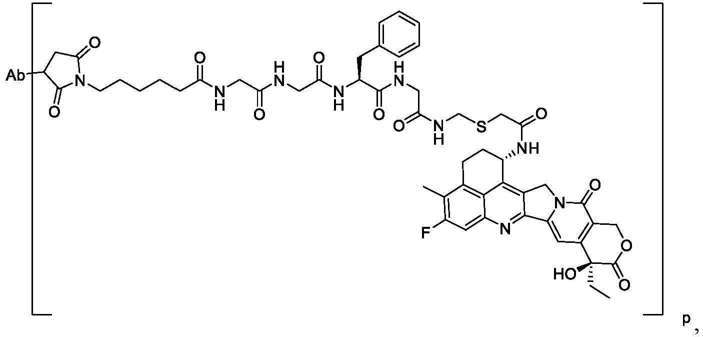 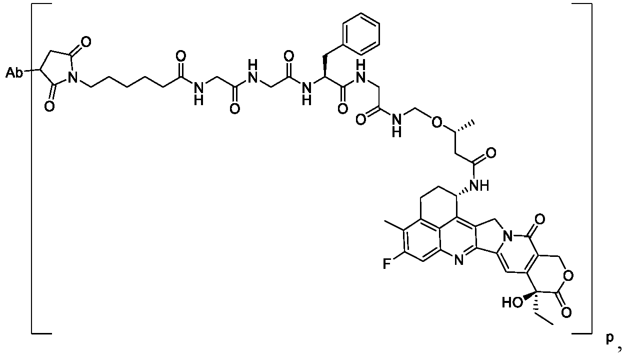 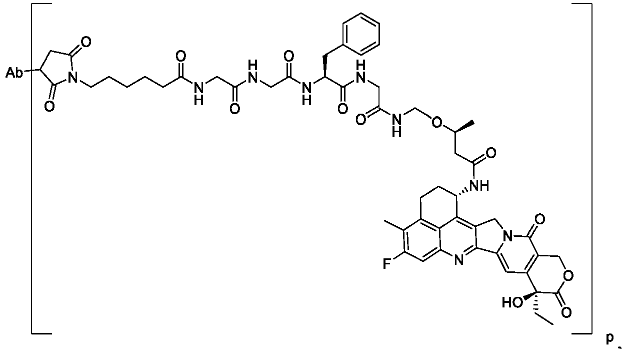 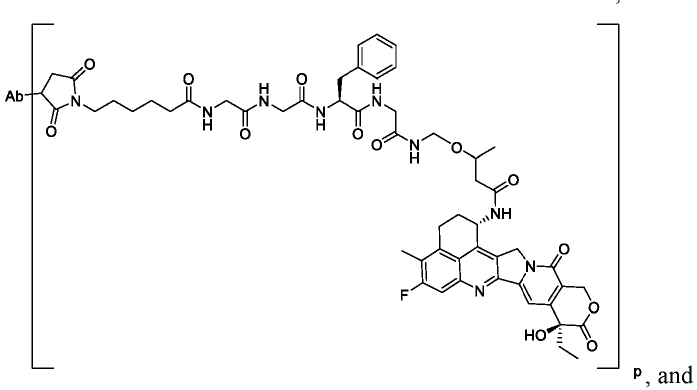 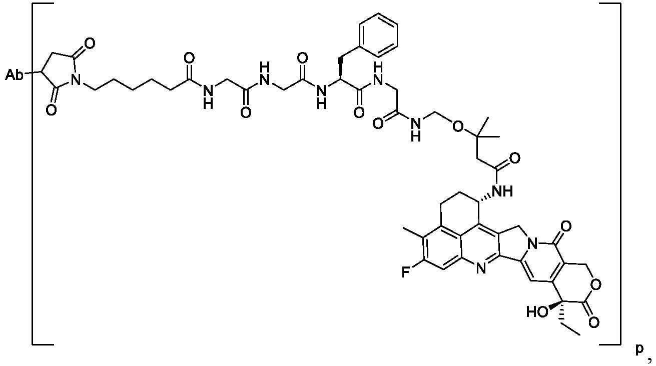 wherein
p is as defined in claim 1 or 8;
Ab is as defined in any one of claims 1 to 7.

32. An anti-GPC3 antibody-drug conjugate, an isomer thereof, a pharmaceutically acceptable salt thereof, or a mixture thereof, wherein the anti-GPC3 antibody-drug conjugate is selected from: 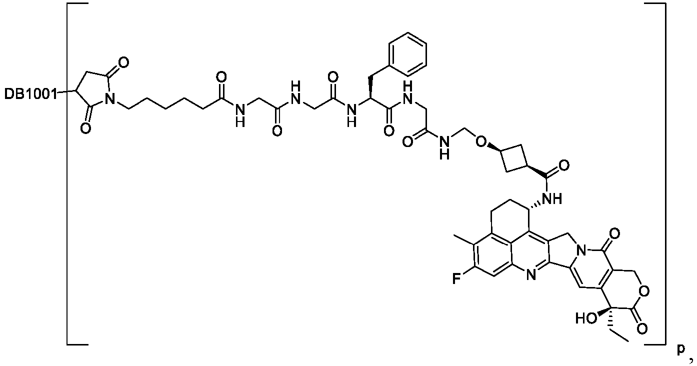 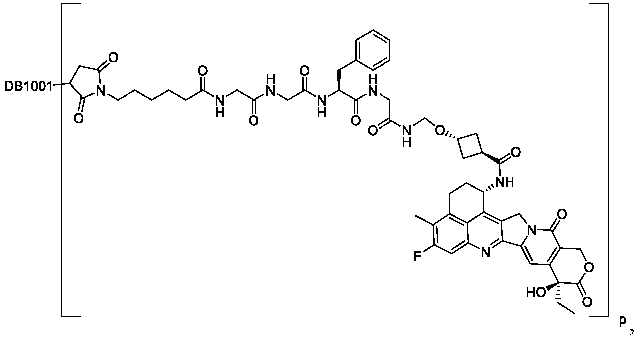 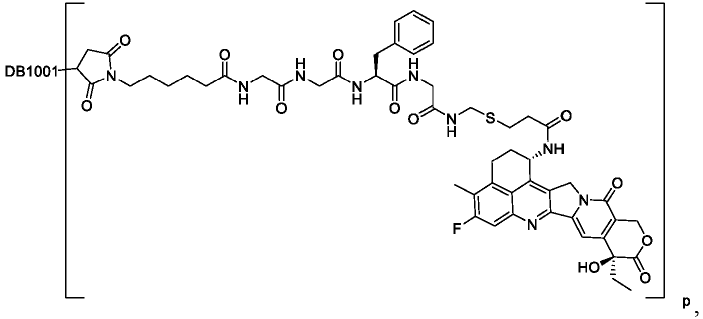 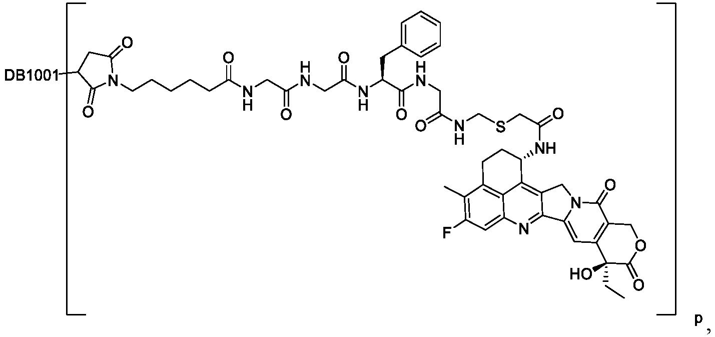 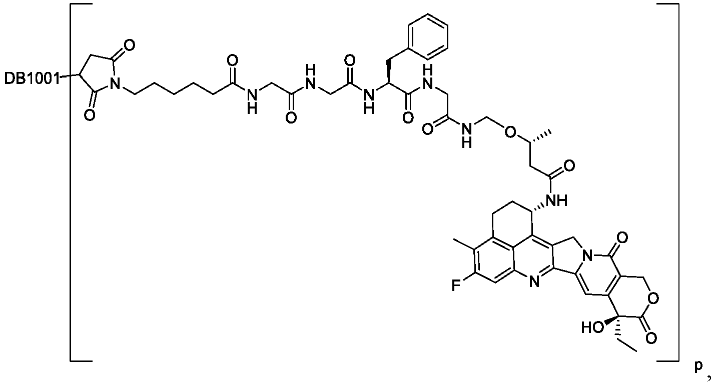 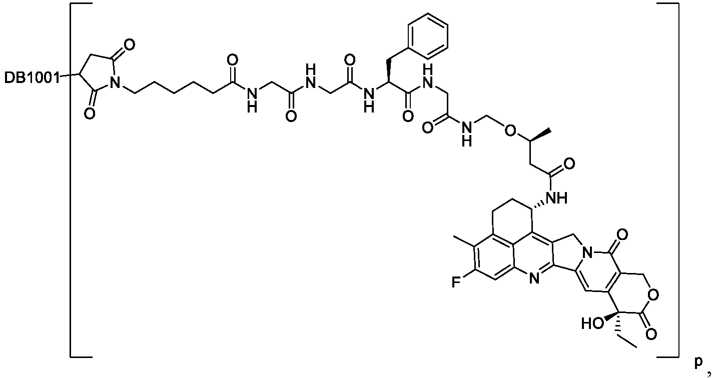 and 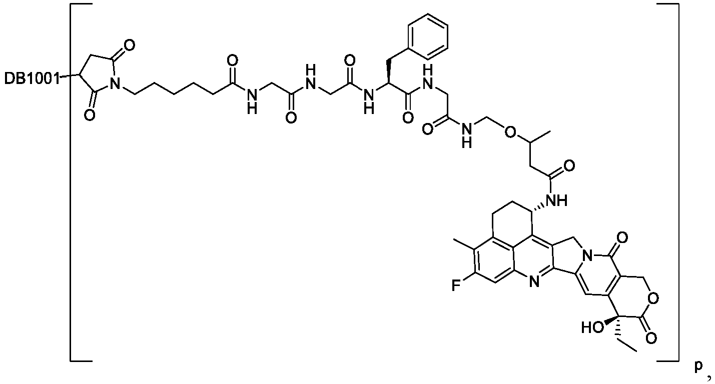 wherein
p represents the average number of connections, and p is an integer or a decimal from 1 to 10, preferably an integer or a decimal from 3 to 8.

33. A pharmaceutical composition, comprising the anti-GPC3 antibody-drug conjugate, the isomer thereof, the pharmaceutically acceptable salt thereof, or the mixture thereof according to any one of claims 1 to 32, and a pharmaceutically acceptable carrier or excipient.

34. A use of the anti-GPC3 antibody-drug conjugate, the isomer thereof, the pharmaceutically acceptable salt thereof, or the mixture thereof according to any one of claims 1 to 32, or the pharmaceutical composition according to claim 33 in the manufacture of a medicament for treating and/or preventing a GPC3-mediated disease or disorder; preferably, the disease or disorder is cancer; more preferably, the cancer is selected from breast cancer, ovarian cancer, prostate cancer, pancreatic cancer, renal cancer, lung cancer, liver cancer, gastric cancer, colon cancer, bladder cancer, esophageal cancer, cervical cancer, gallbladder cancer, glioblastoma, and melanoma.

35. A method for treating and/or preventing a GPC3-mediated disease or disorder, comprising administering to a subject in need thereof the anti-GPC3 antibody-drug conjugate, the isomer thereof, the pharmaceutically acceptable salt thereof, or the mixture thereof according to any one of claims 1 to 32, or the pharmaceutical composition according to claim 33; preferably, the disease or disorder is cancer; more preferably, the cancer is selected from breast cancer, ovarian cancer, prostate cancer, pancreatic cancer, renal cancer, lung cancer, liver cancer, gastric cancer, colon cancer, bladder cancer, esophageal cancer, cervical cancer, gallbladder cancer, glioblastoma, and melanoma.
